# EUROPEAN PATENT SPECIFICATION

(11) **EP 1 601 653 B1**
(45) Date of publication and mention of the grant of the patent: **04.02.2009**
(21) Application number: 04717574.0
(22) Date of filing: 05.03.2004
(51) Int. Cl.: C07D 213/79, C07D 213/80, C07D 405/12, C07D 409/12, C07D 401/12, C07D 413/12, C07D 417/12, C07D 401/06, C07D 401/08, C07D 417/06, C07D 417/08, C07D 413/06, C07D 413/08

(54) **PROCESS FOR THE PRODUCTION OF SUBSTITUTED NICOTINIC ACID ESTERS**
VERFAHREN ZUR HERSTELLUNG SUBSTITUIERTER NIKOTINSÄUREESTER
PROCEDE DE PRODUCTION D'ESTERS D'ACIDES NICOTINIQUES SUBSTITUES

(30) Priority: 07.03.2003 CH 373032003
(43) Date of publication of application: 07.12.2005
(73) Proprietor: Syngenta Participations AG, 4058 Basel (CH)
(72) Inventor: JACKSON, David Anthony, Syngenta Huddersfield, West Yorkshire, HD2 1FF (GB); BOWDEN, Martin Charles, Huddersfield, West Yorkshire, HD2 1FF (GB)
(74) Representative: Hölscher, Ingo
(86) International application number: PCT/EP2004/002291
(87) International publication number: WO 2004/078729

(56) References cited:
- EP-A- 1 340 747
- WO-A-00/39094
- WO-A-01/94339
- PATENT ABSTRACTS OF JAPAN vol. 2000, no. 23, 10 February 2001 (2001-02-10) & JP 2001 158774 A (ISHIHARA SANGYO KAISHA LTD), 12 June 2001 (2001-06-12)
- BOTTORFF, E M; JONES R G, KORNFELD E C; MANN M J: "Pyridine Syntheses. I. Some Reactions of "Ene Amines" with 1,3-dicarbonyl derivatives" JOURNAL OF THE AMERICAN CHEMICAL SOCIETY, vol. 73, 1951, pages 4380-4383, XP002286147

## Description

The present invention relates to a novel process for the preparation of 6-haloalkyl-3-nicotinic acid esters and also to novel enamine intermediates for use in that process.

6-Haloalkyl-3-nicotinic acid esters are valuable intermediates for the preparation of herbicides such as those described, for example, in WO 01/94339.

From Heterocycles, Vol. 48, No. 4, 1998, pages 779-785 it is known to prepare 6-trifluoro-3-nicotinic acid ethyl esters substituted by aryl in the 4-position, corresponding to formula A, by means of dehydrogenation and subsequent oxidation of the compound of formula B in accordance with the following scheme

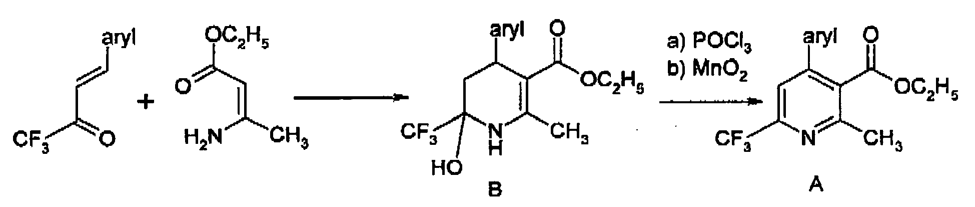

As a result of the uneconomic multi-step procedure, that process is not well suited to the large-scale preparation of 6-haloalkyl-3-nicotinic acid ethyl esters.

According to Heterocycles, Vol. 46, 1997, pages 129-132, 6-trifluoro-3-nicotinic acid methyl esters substituted by phenyl or alkyl in the 2-position, corresponding to formula C, 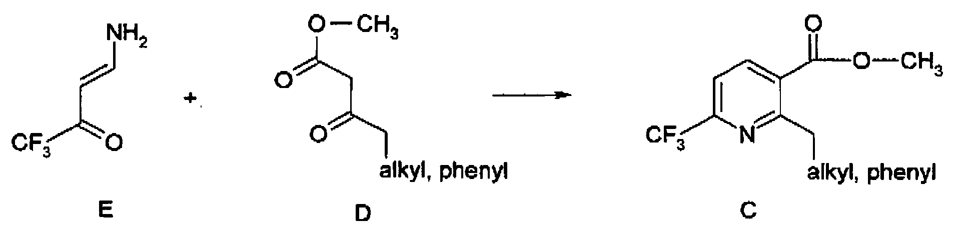 can be prepared by reacting a compound of formula E with a compound of formula D in benzene and in the presence of trifluoroacetic acid. In addition to unsatisfactory yields, that process has the serious disadvantage for large-scale preparation that the quality of the enamine (E) used as starting material continuously deteriorates during storage as a result of polymerisation reactions, making it considerably more difficult to ensure a consistent product quality.

The problem of the present invention is consequently to make available a novel process for the preparation of 6-haloalkyl-3-nicotinic acid esters which makes it possible to prepare those compounds at reasonable cost, in high yields and with good quality.

The present invention accordingly relates to a process for the preparation of compounds of formula I 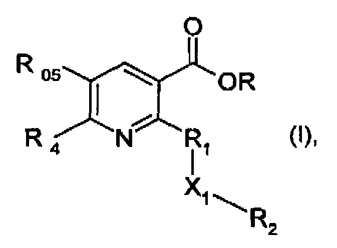 wherein
R is C₁-C₆alkyl;
R₀₅ is hydrogen;
R₁ is a C₁-C₆alkylene, C₃-C₆alkenylene or C₃-C₆alkynylene chain which may be substituted one or more times by halogen and/or by R₅, the unsaturated bonds of the chain not being attached directly to the substituent X₁:
R₄ is halomethy or haloethyl;
X₁ is oxygen, -O(CO)-, -(CO)O-, -O(CO)O-, -N(R₆)-O-, -O-NR₁₇-, thio, sulfinyl, sulfonyl, -SO₂NR₇-, -NR₁₈SO₂-, or -NR₈-;
R₂ is hydrogen or C₁-C₆alkyl, or is a C₁-C₆alkyl, C₃-C₆alkenyl or C₃-C₆alkynyl group which may be substituted one or more times by halogen, hydroxy, amino, formyl, nitro, cyano, mercapto, carbamoyl, C₁-C₆alkoxy, C₁-C₆alkoxycarbonyl, C₂-C₆alkenyl, C₂-C₆haloalkenyl, C₂-C₆alkynyl, C₂-C₆haloalkynyl, C₃-C₆cycloalkyl, halo-substituted C₃-C₆cycloalkyl, C₃-C₆alkenyloxy, C₃-C₆alkynyloxy, C₁-C₆haloalkoxy, C₃-C₆haloalkenyloxy, cyano-C₁-C₆alkoxy, C₁-C₆alkoxy-C₁-C₆alkoxy, C₁-C₆alkoxy-C₁-C₆alkoxy-C₁-C₆alkoxy, C₁-C₆alkylthio-C₁-C₆alkoxy, C₁-C₆alkylsulfinyl-C₁-C₆alkoxy, C₁-C₆alkylsulfonyl-C₁-C₆alkoxy, C₁-C₆-alkoxycarbonyl-C₁-C₆alkoxy, C₁-C₆alkylcarbonyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, oxiranyl (which may in turn be substituted by C₁-C₆alkyl), (3-oxetanyl)oxy (which may in turn be substituted by C₁-C₆alkyl), or by benzylthio, benzylsulfinyl, benzylsulfonyl, C₁-C₆alkylamino, di(C₁-C₆alkyl)amino, R₉S(O)₂O-, R₁₀N(R₁₁)SO₂-, rhodano, phenyl, phenoxy, phenylthio, phenylsulfinyl or by phenylsulfonyl;
it being possible for the phenyl- or benzyl-containing groups to be in turn substituted by one or more C₁-C₆alkyl, C₁-C₆haloalkyl, C₁-C₆alkoxy, C₁-C₆haloalkoxy, halogen, cyano, hydroxy or nitro groups, or
R₂ is phenyl which may be substituted one or more times by C₁-C₆alkyl, C₁-C₆haloalkyl, C₁-C₆alkoxy, C₁-C₆haloalkoxy, halogen, cyano, hydroxy or by nitro; or
R₂ is C₃-C₆cycloalkyl, C₁-C₆alkoxy- or C₁-C₆alkyl-substituted C₃-C₆cycloalkyl, 3-oxetanyl or C₁-C₆alkyl-substituted 3-oxetanyl; or
R₂ is a five- to ten-membered, monocyclic or fused bicyclic, ring system which may be aromatic, partially saturated or fully saturated and may contain from 1 to 4 hetero atoms selected from nitrogen, oxygen, sulfur, and/or may contain the group -C(=O)-, -C(=S)-, -C(=NR₁₉)-, -(N=O)-, -S(=O)- or -SO₂-, the ring system being attached to the substituent X₁ either directly or by way of a C₁-C₄alkylene, -N(R₁₂)-C₁-C₄alkylene, -SO-C₁-C₄alkylone or -SO₂-C₁-C₄alkylene group and each ring system containing no more than 2 oxygen atoms and no more than two sulfur atoms, and it being possible for each ring system itself to be substituted one or more times by C₁-C₆alkyl, C₁-C₆haloalkyl, C₂-C₆alkenyl, C₂-C₆haloalkenyl, C₂-C₆alkynyl, C₂-C₆haloalkynyl, C₁-C₆alkoxy, C₁-C₆haloalkoxy, C₃-C₆alkenyloxy, C₃-C₆alkynyloxy, mercapto, amino, hydroxy, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₆alkylthio, C₁-C₄alkylcarbonyl-C₁-C₃alkylthio, C₁-C₄alkoxycarbonyl-C₁-C₃alkylthio, cyano-C₁-C₃alkylthio, C₁-C₆alkylsuffinyl, C₁-C₆haloalkylsulfinyl, C₁-C₆alkylsulfonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano, nitro or by phenyl, it being possible for the phenyl group to be in turn substituted by hydroxy, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₁-C₄alkylcarbonyl-C₁-C₃alkykhio, C₁-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano or by nitro, and the substituents on nitrogen in a heterocyclic ring being other than halogen;
R₅ is hydroxy, C₁-C₆alkoxy, C₃-C₆cycloalkyloxy, C₁-C₆alkoxy-C₁-C₆alkoxy, C₁-C₆alkoxy-C₁-C₆alkoxy-C₁-C₆alkoxy or C₁-C₂alkylsulfonyloxy;
R₆, R₇, R₈, R₉, R₁₀ R₁₁, R₁₂, R₁₇, and R₁₈ are each independently of the others hydrogen, C₁-C₆alkyl, C₁-C₆haloalkyl, C₁-C₆alkoxycarbonyl, C₁-C₆alkylcarbonyl, C₁-C₆alkoxy-C₁-C₆alkyl, C₁-C₆alkoxy-C₁-C₆alkyl substituted by C₁-C₆alkoxy, benzyl; or phenyl, it being possible for phenyl and benzyl to be in turn substituted one or more times by C₁-C₆alkyl, C₁-C₆haloalkyl, C₁-C₆alkoxy, C₁-C₆haloalkoxy, halogen, cyano, hydroxy or by nitro; R₆ not being hydrogen when R₉ is hydrogen, C₁-C₆alkoxycarbonyl or C₁-C₆alkylcarbonyl;
or the group -R₁-X₁-R₂ together is C₁-C₆alkyl, C₂-C₆alkenyl, C₂-C₆haloalkenyl, C₂-C₆-alkynyl, C₂-C₆haloalkynyl, C₃-C₆cycloalkyl, C₁-C₆alkoxy, C₁-C₆haloalkoxy, C₁-C₆alkylthio, C₁-C₆alkylsulfinyl, C₁-C₆alkylsulfonyl, C₁-C₆haloalkyl, C₁-C₆haloalkylthio, C₁-C₆haloalkyl-sulfinyl, C₁-C₆haloalkylsulfonyl, C₁-C₆alkoxycarbonyl, C₁-C₆alkylcarbonyl, C₁-C₆alkylamino, di(C₁-C₆alkyl)amino, C₁-C₆alkylaminosulfonyl, di(C₁-C₆alkyl)aminosulfonyl, -NH-S-R₁₃, -N-(C₁-C₄alkylthio)-R₁₃, -NH-SO-R₁₄, -N-(C₁-C₄alkylsulfonyl)-R₁₄, -NH-SO₂-R₁₅, -N-(C₁-C₄alkylsulfonyl)-R₁₅, nitro, cyano, halogen, hydroxy, amino, formyl, rhodano-C₁-C₆-alkyl, cyano-C₁-C₆alkyl, oxiranyl, C₃-C₆alkenyloxy, C₃-C₆alkynyloxy, C₁-C₆alkoxy-C₁-C₆-alkoxy, cyano-C₁-C₆alkenyloxy, C₁-C₆alkoxycarbonyloxy-C₁-C₆alkoxy, C₃-C₆alkynyloxy, cyano-C₁-C₆alkoxy, C₁-C₆alkoxycarbonyl-C₁-C₆alkoxy, C₁-C₆alkylthio-C₁-C₆alkoxy, C₁-C₆alkoxycarbonyl-C₁-C₆alkylthio, C₁-C₆alkoxycarbonyl-C₁-C₆alkylsulfinyl, C₁-C₆alkoxycarbonyl-C₁-C₆alkylsulfonyl, C₁-C₆alkylsulfonyloxy, C₁-C₆haloalkylsulfonyloxy, phenyl, benzyl, phenoxy, phenylthio, phenylsulfinyl, phenylsulfonyl, benzylthio, benzylsulfinyl or benzylsulfonyl, it being possible for the phenyl groups to be substituted one or more times by halogen, methyl, ethyl, trifluoromethyl, methoxy or by nitro;
or the group -R₁-X₁-R₂ together is a five- to ten-membered, monocyclic or fused bicyclic, ring system, which may be aromatic or partially saturated and which may contain from 1 to 4 hetero atoms selected from nitrogen, oxygen and sulfur, the ring system either being directly attached to the pyridine ring being attached to the pyridine ring by way of a C₁-C₄alkylene group, and it being possible for each ring system to contain no more than 2 oxygen atoms and no more than two sulfur atoms, and/or contain the group -C(=O)-, - C(=S)-, -C(=NR₂₀)-, -(N=O)-, -S(=O)- or -SO₂-; and the ring system itself may be substituted one, two or three times by C₁-C₆alkyl, C₁-C₆haloalkyl, C₃-C₆alkenyl, C₃-C₆haloalkenyl, C₃-C₆alkynyl, C₃-C₆haloalkynyl, C₁-C₆alkoxy, C₁-C₆haloalkoxy, C₃-C₆alkenyloxy, C₃-C₆alkynyloxy, mercapto, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₁-C₄alkoxycarbonyl-C₁-C₃alkylthio, cyano-C₁-C₃alkylthio, C₁-C₆alkylsulfinyl, C₁-C₆halo-alkylsulfinyl, C₁-C₆alkylsulfonyl, C₁-C₆haloalkylsulfonyl, aminosulfonyl, C₁-C₂alkylamino-sulfonyl, di(C₁-C₆alkyl)aminosulfonyl, C₁-C₃alkylene-R₁₈, halogen, cyano, nitro, phenyl and benzylthio, it being possible for phenyl, benzyloxy and benzylthio to be in turn substituted on the phenyl ring by C₁-C₃alkyl, C₁-C₃haloalkyl, C₁-C₃alkoxy, C₁-C₃haloalkoxy, halogen, cyano or by nitro, and substituents on a nitrogen atom in a heterocyclic ring being other than halogen;
R₁₃ is N(H)-C₁-C₆alkyl, N(H)-C₁-C₆alkoxy, N-(C₁-C₆alkyl)-C₁-C₆alkyl, N-(C₁-C₆alkyl)-C₁-C₆alkoxy, C₁-C₆alkoxy, C₁-C₆haloalkoxy, C₁-C₆alkyl, C₁-C₆haloalkyl, C₃-C₆alkenyl, C₃-C₆haloalkenyl, C₃-C₆alkynyl, C₃-C₆haloalkynyl, C₃-C₆cycloalkyl or phenyl, it being possible for phenyl to be in turn substituted by C₁-C₃alkyl, C₁-C₃haloalkyl, C₁-C₃alkoxy, C₁-C₃haloalkoxy, halogen, cyano or by nitro;
R₁₄ is N(H)-C₁-C₆alkyl, N(H)-C₁-C₆alkoxy, N-(C₁-C₆alkyl)-C₁-C₆alkyl, N-(C₁-C₆alkyl)-C₁-C₆alkoxy, C₁-C₆alkoxy, C₁-C₆haloalkoxy, C₁-C₆alkyl, C₁-C₆haloalkyl, C₃-C₆alkenyl, C₃-C₆haloalkenyl, C₃-C₆alkynyl, C₃-C₆haloalkynyl, C₃-C₆cycloalkyl or phenyl, it being possible for phenyl to be in turn substituted by C₁-C₃alkyl, C₁-C₃haloalkyl, C₁-C₃alkoxy, C₁-C₃haloalkoxy, halogen, cyano or by nitro;
R₁₅ is N(H)-C₁-C₆alkyl, N(H)-C₁-C₆alkoxy, N-(C₁-C₆alkyl)-C₁-C₆alkyl, N-(C₁-C₆alkyl)-C₁-C₆alkoxy, C₁-C₆alkoxy, C₁-C₆haloalkoxy, C₁-C₆alkyl, C₁-C₆haloalkyl, C₃-C₆alkenyl, C₃-C₆haloalkenyl, C₃-C₆alkynyl, C₃-C₆haloalkynyl, C₃-C₆cycloalkyl or phenyl, it being possible for phenyl to be in turn substituted by C₁-C₃alkyl, C₁-C₃haloalkyl, C₁-C₃alkoxy, C₁-C₃haloalkoxy, halogen, cyano or by nitro;
R₁₆ is C₁-C₃alkoxy, C₂-C₄alkoxycarbonyl, C₁-C₃alkylthio, C₁-C₃alkylsulfinyl, C₁-C₃alkylsulfonyl or phenyl, it being possible for phenyl to be in turn substituted by C₁-C₃alkyl, C₁-C₃haloalkyl, C₁-C₃alkoxy, C₁-C₃haloalkoxy, halogen, cyano or by nitro; and
R₁₉ and R₂₀ are each independently of the other hydrogen, hydroxy, C₁-C₆alkyl, C₁-C₆haloalkyl, C₁-C₆alkoxy, C₁-C₆alkylcarbonyl, C₁-C₆alkoxycarbonyl or C₁-C₆alkylsulfonyl ; which process comprises reacting
a compound of formula II 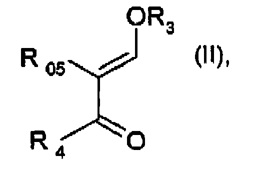
wherein R₃ is C₁-C₆alkyl or C₃-C₆cycloalkyl and R₄ and R₀₅ are as defined for formula I, with a compound of formula III 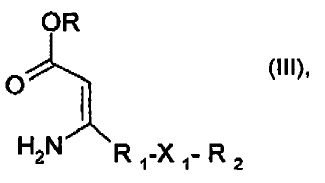 wherein R, R₁, R₂ and X₁ are as defined for formula I, in an inert solvent in the presence of a proton source.

Also disclosed is a process for the preparation of compounds of formula I 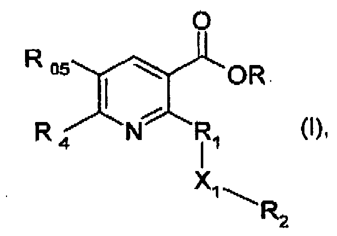 wherein
R is C₁-C₆alkyl;
R₀₅ is Hydrogen, C₁-C₃alkyl, C₁-C₃haloalkyl or C₁-C₃alkyl-C₁-C₃alkoxy;
R₁ is a C₁-C₆alkylene, C₃-C₆alkenylene or C₃-C₆alkynylene chain which may be substituted one or more times by halogen and/or by R₅, the unsaturated bonds of the chain not being attached directly to the substituent X₁;
R₄ is C₁-C₄haloalkyl;
X₁ is oxygen, -O(CO)-, -(CO)O-, -O(CO)O-, -N(R₆)-O-, -O-NR₁₇-, thio, sulfinyl, sulfonyl,
-SO₂NR₇-, -NR₁₈SO₂-, -N(SO₂R₁₈ₐ)-, -N(R_{18b})C(O)- or -NR₈-;
R₁₈ₐ is C₁-C₆alkyl;
R₂ is hydrogen or C₁-C₆alkyl, or is a C₁-C₆alkyl, C₃-C₆alkenyl or C₃-C₆alkynyl group which may be substituted one or more times by substituents selected from halogen, hydroxy, amino, formyl, nitro, cyano, mercapto, carbamoyl, C₁-C₆alkoxy, C₁-C₆alkoxycarbonyl, C₂-C₆alkenyl, C₂-C₆haloalkenyl, C₂-C₆alkynyl, C₂-C₆haloalkynyl, C₃-C₆cycloalkyl, halo-substituted C₃-C₆cycloalkyl, C₃-C₆alkenyloxy, C₃-C₆alkynyloxy, C₁-C₆haloalkow, C₃-C₆haloalkenyloxy, cyano-C₁-C₆alkoxy, C₁-C₆alkoxy-C₁-C₆alkoxy, C₁-C₆alkoxy-C₁-C₆alkoxy-C₁-C₆alkoxy, C₁-C₆alkylthio-C₁-C₆alkoxy, C₁-C₆alkylsulfinyl-C₁-C₆alkoxy, C₁-C₆alkylsulfonyl-C₁-C₆alkoxy, C₁-C₆alkoxycarbonyl-C₁-C₆alkoxy, C₁-C₆alkylcarbonyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, oxiranyl (which may in turn be substituted by C₁-C₆alkyl), (3-oxetanyl)oxy (which may in turn be substituted by C₁-C₆alkyl), benzyloxy, benzylthio, benzylsulfinyl, benzylsulfonyl, C₁-C₆alkylamino, di(C₁-C₆alkyl)amino, R₉S(O)₂O-, R₁₀N(R₁₁)SO₂-, rhodano, phenyl, phenoxy, phenylthio, phenylsulfinyl and phenylsulfonyl;
it being possible for the phenyl- or benzyl-containing groups to be in turn substituted by one or more C₁-C₆alkyl, C₁-C₆haloalkyl, C₁-C₆alkoxy, C₁-C₆haloalkoxy, halogen, cyano, hydroxy or nitro groups, or
R₂ is phenyl which may be substituted one or more times by C₁-C₆alkyl, C₁-C₆haloalkyl, C₁-C₆alkoxy, C₁-C₆haloalkoxy, halogen, cyano, hydroxy or by nitro; or
R₂ is C₃-C₆cycloalkyl, C₁-C₆alkoxy- or C₁-C₆alkyl-substituted C₃-C₆cycloalkyl, 3-oxetanyl or C₁-C₆alkyl-substituted 3-oxetanyl; or
R₂ is a three- to ten-membered, monocyclic or fused bicyclic, ring system which may be aromatic, partially saturated or fully saturated and may contain from 1 to 4 hetero atoms selected from nitrogen, oxygen, sulfur, and/or may contain the group -C(=O)-, -C(=S)-, -C(=NR₁₉)-, -(N=O)-, -S(=O)- or -SO₂-, the ring system being attached to the substituent X₁ either directly or by way of a C₁-C₄alkylene, C₂-C₄alkenylene, C₂-C₄alkynylene, -N(R₁₂)-C₁-C₄alkylene, -O-C₁-C₄alkylene, -S-C₁-C₄alkylene, -SO-C₁-C₄alkylene or -SO₂-C₁-C₄alkylene group and each ring system containing no more than 2 oxygen atoms and no more than two sulfur atoms, and it being possible for each ring system itself to be substituted one or more times by C₁-C₆alkyl, C₁-C₆haloalkyl, C₂-C₆alkenyl, C₂-C₆haloalkenyl, C₂-C₆alkynyl, C₂-C₆haloalkynyl, C₁-C₆alkoxy, C₁-C₆haloalkoxy, C₃-C₆alkenyloxy, C₃-C₆alkynyloxy, mercapto, amino, hydroxy, C₁-C₆alkylthio, C₁-C₆haloalkylthio, C₃-C₆-alkenylthio, C₃-C₆haloalkenylthio, C₃-C₆alkynylthio, C₁-C₃alkoxy-C₁-C₃alkylthio, C₁-C₄alkylcarbonyl-C₁-C₃alkylthio, C₁-C₄alkoxycarbonyl-C₁-C₃alkylthio, cyano-C₁-C₃alkylthio, C₁-C₆-alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano, nitro or by phenyl, it being possible for the phenyl group to be in turn substituted by hydroxy, C₁-C₆alkylthio, C₁-C₆haloalkylthio, C₃-C₆alkenylthio, C₃-C₆halo-alkenylthio, C₃-C₆alkynylthio, C₁-C₃alkoxy-C₁-C₃alkylthio, C₁-C₄alkylcarbonyl-C₁-C₃alkylthio, C₁-C₄alkoxycarbonyl-C₁-C₃alkylthio, cyano-C₁-C₃alkylthio, C₁-C₆alkylsulfinyl, C₁-C₆-haloalkylsulfinyl, C₁-C₆alkylsulfonyl, C₁-C₆haloalkylsulfonyl, aminosulfonyl, C₁-C₂alkyl-aminosulfonyl, N,N-di(C₁-C₂alkyl)aminosulfonyl, di(C₁-C₄alkyl)amino, halogen, cyano or by nitro, and the substituents on nitrogen in a heterocyclic ring being other than halogen;
R₅ is hydroxy, C₁-C₆alkoxy, C₃-C₆cycloalkyloxy, C₁-C₆alkoxy-C₁-C₆alkoxy, C₁-C₆alkoxy-C₁-C₆alkoxy-C₁-C₆alkoxy or C₁-C₂alkylsulfonyloxy;
R₆, R₇, R₈, R₉, R₁₀ R₁₁, R₁₂, R₁₇, R₁₈ and R_{18b} are each independently of the others hydrogen, C₁-C₆alkyl, C₁-C₆haloalkyl, C₁-C₆alkoxycarbonyl, C₁-C₆alkylcarbonyl, C₁-C₆-alkoxy-C₁-C₆alkyl, C₁-C₆alkoxy-C₁-C₆alkyl substituted by C₁-C₆alkoxy, benzyl, or phenyl, it being possible for phenyl and benzyl to be in turn substituted one or more times by C₁-C₆alkyl, C₁-C₆haloalkyl, C₁-C₆alkoxy, C₁-C₆haloalkoxy, halogen, cyano, hydroxy or by nitro; R₆ not being hydrogen when R₉ is hydrogen, C₁-C₆alkoxycarbonyl or C₁-C₆alkylcarbonyl;
or the group -R₁-X₁-R₂ together is C₁-C₆alkyl, C₂-C₆alkenyl, C₂-C₆haloalkenyl, C₂-C₆-alkynyl, C₂-C₆haloalkynyl, C₃-C₆cycloalkyl, C₁-C₆alkoxy, C₁-C₆haloalkoxy, C₁-C₆alkylthio, C₁-C₆alkylsulfinyl, C₁-C₆alkylsulfonyl, C₁-C₆haloalkyl, C₁-C₆haloalkylthio, C₁-C₆haloalkyl-sulfinyl, C₁-C₆haloalkylsulfonyl, C₁-C₆alkoxycarbonyl, C₁-C₆alkylcarbonyl, C₁-C₆alkylamino, di(C₁-C₆alkyl)amino, C₁-C₆alkylaminosulfonyl, di(C₁-C₆alkyl)aminosulfonyl, -NH-S-R₁₃, -N-(C₁-C₄alkylthio)-R₁₃, -NH-SO-R₁₄, -N-(C₁-C₄alkylsulfonyl)-R₁₄, -NH-SO₂-R₁₅, -N-(C₁-C₄alkylsulfonyl)-R₁₅, nitro, cyano, halogen, hydroxy, amino, formyl, rhodano-C₁-C₆-alkyl, cyano-C₁-C₆alkyl, oxiranyl, C₃-C₆alkenyloxy, C₃-C₆alkynyloxy, C₁-C₆alkoxy-C₁-C₆-alkoxy, cyano-C₁-C₆alkenyloxy, C₁-C₆alkoxycarbonyloxy-C₁-C₆alkoxy, C₃-C₆alkynyloxy, cyano-C₁-C₆alkoxy, C₁-C₆alkoxycarbonyl-C₁-C₆alkoxy, C₁-C₆alkylthio-C₁-C₆alkoxy, C₁-C₆alkoxycarbonyl-C₁-C₆alkylthio, C₁-C₆alkoxycarbonyl-C₁-C₆alkylsulfinyl, C₁-C₆alkoxycarbonyl-C₁-C₆alkylsulfonyl, C₁-C₆alkylsulfonyloxy, C₁-C₆haloalkylsulfonyloxy, phenyl, benzyl, phenoxy, phenylthio, phenylsulfinyl, phenylsulfonyl, benzylthio, benzylsulfinyl or benzylsulfonyl, it being possible for the phenyl groups to be substituted one or more times by halogen, methyl, ethyl, trifluoromethyl, methoxy or by nitro;
or the group -R₁-X₁-R₂ together is a three- to ten-membered, monocyclic or fused bicyclic, ring system, which may be aromatic, partially saturated or saturated and which may contain from 1 to 4 hetero atoms selected from nitrogen, oxygen and sulfur and/or may contain one or two groups selected from -C(=O)-, -C(=S)-, -C(=NR₂₀)-, -(N=O)-, -S(=O)-and -SO₂-, the ring system either being attached to the pyridine ring directly *via* a carbon atom or being attached to the pyridine ring *via* a carbon atom or *via* a nitrogen atom by way of a C₁-C₄alkylene, C₂-C₄alkenyl or C₂-C₄alkynyl chain, and it being possible for each ring system to contain no more than 2 oxygen atoms and no more than two sulfur atoms, and it being possible for the ring system itself to be substituted one, two or three times by substituents selected from C₁-C₆alkyl, C₁-C₆haloalkyl, C₃-C₆alkenyl, C₃-C₆haloalkenyl, C₃-C₆alkynyl, C₃-C₆haloalkynyl, C₃-C₆cycloalkyl, hydroxy, C₁-C₆alkoxy, C₁-C₆haloalkoxy, C₃-C₆alkenyloxy, C₃-C₆alkynyloxy, mercapto, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₁-C₃alkylcarbonyl-C₁-C₃alkylthio, C₁-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, di(C₁-C₆alkyl)aminosulfonyl, C₁-C₃alkylene-R₁₆, amino, C₁-C₆alkylamino, C₁-C₆alkoxyamino, di(C₁-C₆alkyl)amino, (N-C₁-C₆alkyl)-C₁-C₆alkoxyamino, halogen, cyano, nitro, phenyl, benzyloxy and benzylthio, it being possible for phenyl, benzyloxy and benzylthio to be in turn substituted on the phenyl ring by C₁-C₃alkyl, C₁-C₃haloalkyl, C₁-C₃alkoxy, C₁-C₃haloalkoxy, halogen, cyano or by nitro, and substituents on a nitrogen atom in a heterocyclic ring being other than halogen;
R₁₃ is N(H)-C₁-C₆alkyl, N(H)-C₁-C₆alkoxy, N-(C₁-C₆alkyl)-C₁-C₆alkyl, N-(C₁-C₆alkyl)-C₁-C₆alkoxy, C₁-C₆alkoxy, C₁-C₆haloalkoxy, C₁-C₆alkyl, C₁-C₆haloalkyl, C₃-C₆alkenyl, C₃-C₆haloalkenyl, C₃-C₆alkynyl, C₃-C₆haloalkynyl, C₃-C₆cycloalkyl or phenyl, it being possible for phenyl to be in turn substituted by C₁-C₃alkyl, C₁-C₃haloalkyl, C₁-C₃alkoxy, C₁-C₃haloalkoxy, halogen, cyano or by nitro;
R₁₄ is N(H)-C₁-C₆alkyl, N(H)-C₁-C₆alkoxy, N-(C₁-C₆alkyl)-C₁-C₆alkyl, N-(C₁-C₆alkyl)-C₁-C₆alkoxy, C₁-C₆alkoxy, C₁-C₆haloalkoxy, C₁-C₆alkyl, C₁-C₆haloalkyl, C₃-C₆alkenyl, C₃-C₆haloalkenyl, C₃-C₆alkynyl, C₃-C₆haloalkynyl, C₃-C₆cycloalkyl or phenyl, it being possible for phenyl to be in turn substituted by C₁-C₃alkyl, C₁-C₃haloalkyl, C₁-C₃alkoxy, C₁-C₃haloalkoxy, halogen, cyano or by nitro;
R₁₅ is N(H)-C₁-C₆alkyl, N(H)-C₁-C₆alkoxy, N-(C₁-C₆alkyl)-C₁-C₆alkyl, N-(C₁-C₆alkyl)-C₁-C₆alkoxy, C₁-C₆alkoxy, C₁-C₆haloalkoxy, C₁-C₆alkyl, C₁-C₆haloalkyl, C₃-C₆alkenyl, C₃-C₆haloalkenyl, C₃-C₆alkynyl, C₃-C₆haloalkynyl, C₃-C₆cycloalkyl or phenyl, it being possible for phenyl to be in turn substituted by C₁-C₃alkyl, C₁-C₃haloalkyl , C₁-C₃alkoxy, C₁-C₃haloalkoxy, halogen, cyano or by nitro;
R₁₆ is C₁-C₃alkoxy, C₂-C₄alkoxycarbonyl, C₁-C₃alkylthio, C₁-C₃alkylsulfinyl, C₁-C₃alkylsulfonyl or phenyl, it being possible for phenyl to be in turn substituted by C₁-C₃alkyl, C₁-C₃haloalkyl, C₁-C₃alkoxy, C₁-C₃haloalkoxy, halogen, cyano or by nitro; and
R₁₉ and R₂₀ are each independently of the other hydrogen, hydroxy, C₁-C₆alkyl, C₁-C₆haloalkyl, C₁-C₆alkoxy, cyano, C₁-C₆alkylcarbonyl, C₁-C₆alkoxycarbonyl or C₁-C₆alkylsulfonyl; which process comprises reacting a compound of formula II 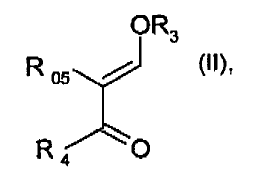
wherein R₃ is C₁-C₆alkyl or C₃-C₆cycloalkyl and R₄ and R₀₅ are as defined for formula I, with a compound of formula III 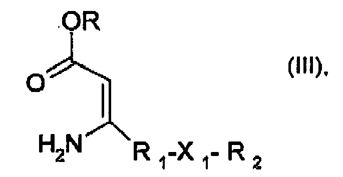 wherein R, R₁, R₂ and X₁ are as defined for formula I, in an inert solvent in the presence of a proton source.

The alkyl groups appearing in the substituent definitions may be straight-chained or branched and are, for example, methyl, ethyl, n-propyl, isopropyl, n-butyl, sec-butyl, isobutyl, tert-butyl, pentyl, hexyl, heptyl and octyl and also the branched isomers thereof. Alkoxy, alkenyl and alkynyl groups are derived from the mentioned alkyl groups. The alkenyl and alkynyl groups may be mono- or poly-unsaturated.

Halogen is generally fluorine, chlorine, bromine or iodine, preferably fluorine or chlorine. The same is also correspondingly true for halogen in conjunction with other meanings such as haloalkyl or halophenyl.

Haloalkyl groups preferably have a chain length of from 1 to 6 carbon atoms. Haloalkyl is, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl or 2,2,2-trichloroethyl; preferably trichloromethyl, difluorochloromethyl, difluoromethyl, trifluoromethyl or dichlorofluoromethyl.

As haloalkenyl there come into consideration alkenyl groups substituted one or more times by halogen, halogen being fluorine, chlorine, bromine or iodine, especially fluorine or chlorine, for example 2,2-difluoro-1-methylvinyl, 3-fluoropropenyl, 3-chloropropenyl, 3-bromopropenyl, 2,3,3-trifluoropropenyl, 2,3,3-trichloropropenyl and 4,4,4-trifluoro-but-2-en-1-yl. Among the C₃-C₆alkenyl groups substituted once, twice or three times by halogen, preference is given to those that have a chain length of from 3 to 5 carbon atoms.

As haloalkynyl there come into consideration alkynyl groups substituted one or more times by halogen, halogen being bromine, iodine or, especially, fluorine or chlorine, for example 3-fluoropropynyl, 3-chloropropynyl, 3-bromopropynyl, 3,3,3-trifluoropropynyl and 4,4,4-trifluoro-but-2-yn-1-yl. Among the alkynyl groups substituted one or more times by halogen, preference is given to those that have a chain length of from 3 to 5 carbon atoms.

Alkoxy groups preferably have a chain length of from 1 to 6 carbon atoms. Alkoxy is, for example, methoxy, ethoxy, propoxy, isopropoxy, n-butoxy, isobutoxy, sec-butoxy or tert-butoxy or the pentyloxy or hexyloxy isomers; preferably methoxy or ethoxy. Alkylcarbonyl preferably is acetyl or propionyl. Alkoxycarbonyl is, for example, methoxycarbonyl, ethoxycarbonyl, propoxycarbonyl, isopropoxycarbonyl, n-butoxycarbonyl, isobutoxycarbonyl, sec-butoxycarbonyl or tert-butoxycarbonyl; preferably methoxycarbonyl or ethoxycarbonyl. Haloalkoxy groups preferably have a chain length of from 1 to 8 carbon atoms. Haloalkoxy is, for example, fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy or 2,2,2-trichloroethoxy; preferably difluoromethoxy, 2-chlorethoxy or trifluoromethoxy. Alkylthio groups preferably have a chain length of from 1 to 8 carbon atoms. Alkylthio is, for example, methylthio, ethylthio, propylthio, isopropylthio, n-butylthio, isobutylthio, sec-butylthio or tert-butylthio, preferably methylthio or ethylthio. Alkylsulfinyl is, for example, methylsulfinyl, ethylsulfinyl, propylsulfinyl, isopropylsulfinyl, n-butylsulfinyl, isobutylsulfinyl, sec-butylsulfinyl or tert-butylsulfinyl; preferably methylsulfinyl or ethylsulfinyl.

Alkylsulfonyl is, for example, methylsulfonyl, ethylsulfonyl, propylsulfonyl, isopropylsulfonyl, n-butylsulfonyl, isobutylsulfonyl, sec-butylsulfonyl or tert-butylsulfonyl; preferably methylsulfonyl or ethylsulfonyl. Alkoxyalkoxy groups preferably have a chain length of from 1 to 8 carbon atoms. Examples of alkoxyalkoxy are: methoxymethoxy, methoxyethoxy, methoxypropoxy, ethoxymethoxy, ethoxyethoxy, propoxymethoxy and butoxybutoxy. Alkylamino is, for example, methylamino, ethylamino, n-propylamino, isopropylamino or the butylamine isomers. Dialkylamino is, for example, dimethylamino, methylethylamino, diethylamino, n-propylmethylamino, dibutylamino or diisopropylamino. Preference is given to alkylamino groups having a chain length of from 1 to 4 carbon atoms. Alkoxyalkyl groups preferably have a chain length of from 2 to 6 carbon atoms. Alkoxyalkyl is, for example, methoxymethyl, methoxyethyl, ethoxymethyl, ethoxyethyl, n-propoxymethyl, n-propoxyethyl, isopropoxymethyl or isopropoxyethyl. Alkylthioalkyl groups preferably have from 2 to 8 carbon atoms. Alkylhioalkyl is, for example, methylthiomethyl, methylthioethyl, ethylthiomethyl, ethylthioethyl, n-propylthiomethyl, n-propyl-thioethyl, isopropylthiomethyl, isopropylthioethyl, butylthiomethyl, butylthioethyl or butylthiobutyl. The cycloalkyl groups preferably have from 3 to 8 ring carbon atoms, for example cyclopropyl, cyclobutyl, cyclopentyl, cyclohexyl, cycloheptyl and cyclooctyl. Phenyl, including phenyl as part of a substituent such as phenoxy, benzyl, benzyloxy, benzoyl, phenylthio, phenylalkyl and phenoxyalkyl, may be present in substituted form, in which case the substituents may be in the ortho-, meta- and/or para-position(s). Preferred substituent positions are the positions ortho and para to the ring attachment position.

Also disclosed are those compounds of formula I wherein
R₄ is halomethyl or haloethyl;
R₀₅ is hydrogen;
X₁ is oxygen, -O(CO)-, -(CO)O-, -O(CO)O-, -N(R₆)-O-, -O-NR₁₇-, thio, sulfinyl, sulfonyl, -SO₂NR₇-, -NR₁₈SO₂- or -NR₈-;
R₂ is hydrogen or C₁-C₆alkyl, or a C₁-C₆alkyl, C₃-C₆alkenyl or C₃-C₆alkynyl group which is substituted one or more times by halogen, hydroxy, amino, formyl, nitro, cyano, mercapto, carbamoyl, C₁-C₆alkoxy, C₁-C₆alkoxycarbonyl, C₂-C₆alkenyl, C₂-C₆haloalkenyl, C₂-C₆-alkynyl, C₂-C₆haloalkynyl, C₃-C₆cycloalkyl, halo-substituted C₃-C₆cycloalkyl, or by C₃-C₆alkenyloxy, C₃-C₆alkynyloxy, C₁-C₆haloalkoxy, C₃-C₆haloalkenyloxy, cyano-C₁-C₆alkoxy, C₁-C₆alkoxy-C₁-C₆alkoxy, C₁-C₆alkoxy-C₁-C₆alkoxy-C₁-C₆alkoxy, C₁-C₆alkylthio-C₁-C₆alkoxy, C₁-C₆alkylsulfinyl-C₁-C₆alkoxy, C₁-C₆alkylsulfonyl-C₁-C₆alkoxy, C₁-C₆-alkoxycarbonyl-C₁-C₆alkoxy, C₁-C₆alkoxycarbonyl, C₁-C₆alkylcarbonyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆-haloalkylsulfonyl, oxiranyl (which may in turn be substituted by C₁-C₆alkyl), or by (3-oxetanyl)oxy (which may in turn be substituted by C₁-C₆alkyl), or by benzylthio, benzylsulfinyl, benzylsulfonyl, C₁-C₆alkylamino, di(C₁-C₆alkyl)amino, R₉S(O)₂O-, R₁₀N(R₁₁)SO₂-, rhodano, phenyl, phenoxy, phenylthio, phenylsulfinyl or by phenylsulfonyl; it being possible for the phenyl- or benzyl-containing groups to be in turn substituted by one or more C₁-C₆alkyl, C₁-C₆haloalkyl, C₁-C₆alkoxy, C₁-C₆haloalkoxy, halogen, cyano, hydroxy or nitro groups, or
R₂ is phenyl which may be substituted one or more times by C₁-C₆alkyl, C₁-C₆haloalkyl, C₁-C₆alkoxy, C₁-C₆haloalkoxy, halogen, cyano, hydroxy or by nitro; or
R₂ is C₃-C₆cycloalkyl, C₁-C₆alkoxy- or C₁-C₆alkyl-substituted C₃-C₆cycloalkyl, 3-oxetanyl or C₁-C₆alkyl-substituted 3-oxetanyl;
or R₂ is a five- to ten-membered, monocyclic or fused bicyclic, ring system which may be aromatic, partially saturated or fully saturated and may contain from 1 to 4 hetero atoms selected from nitrogen, oxygen, sulfur, and/or may contain the group -C(=O)-, -C(=S)-, -C(=NR₁₉)-, -(N=O)-, -S(=O)- or -SO₂-, the ring system being attached to the substituent X₁ directly or by way of a C₁-C₄alkylene, C₂-C₄alkenyl-C₁-C₄alkylene, C₂-C₄alkynyl-C₁-C₄alkylene, -N(R₁₂)-C₁-C₄alkylene, -SO-C₁-C₄alkylene or -SO₂-C₁-C₄alkylene group and each ring system containing no more than 2 oxygen atoms and no more than two sulfur atoms, and it being possible for each ring system itself to be substituted one or more times by C₁-C₆alkyl, C₁-C₆haloalkyl, C₂-C₆alkenyl, C₂-C₆haloalkenyl, C₂-C₆alkynyl, C₂-C₆haloalkynyl, C₁-C₆alkoxy, C₁-C₆haloalkoxy, C₃-C₆alkenyloxy, C₃-C₆alkynyloxy, mercapto, amino, hydroxy, C₁-C₆alkylthio, C₁-C₆haloalkylthio, C₃-C₆alkenylthio, C₃-C₆halo-alkenylthio, C₃-C₆alkynylthio, C₁-C₃alkoxy-C₁-C₃alkylthio, C₁-C₄alkylcarbonyl-C₁-C₃alkylthio, C₁-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano, nitro or by phenyl, it being possible for the phenyl group to be in turn substituted by hydroxy, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₁-C₄alkylcarbonyl-C₁-C₃alkylthio, C₁-C₄-alkoxycarbonyl-C₁-C₃alkylthio, cyano-C₁-C₃alkylthio, C₁-C₆alkylsulfinyl, C₁-C₆haloalkyl-sulfinyl, C₁-C₆alkylsulfonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano or by nitro, and the substituents on nitrogen in a heterocyclic ring being other than halogen;
R₆, R₇, R₈, R₉, R₁₀ R₁₀, R₁₂, R₁₇ and R₁₈ are each independently of the others hydrogen, C₁-C₆alkyl, C₁-C₆haloalkyl, C₁-C₆alkoxycarbonyl, C₁-C₆alkylcarbonyl, C₁-C₆alkoxy-C₁-C₆alkyl, C₁-C₆alkoxy-C₁-C₆alkyl substituted by C₁-C₆alkoxy, benzyl, or phenyl, it being possible for phenyl and benzyl to be in turn substituted one or more times by C₁-C₆alkyl, C₁-C₆haloalkyl, C₁-C₆alkoxy, C₁-C₆haloalkoxy, halogen, cyano, hydroxy or by nitro; R₆ not being hydrogen when R₉ is hydrogen, C₁-C₆alkoxycarbonyl or C₁-C₆alkylcarbonyl;
or the group -R₁-X₁-R₂ together is C₁-C₆alkyl, C₂-C₆alkenyl, C₂-C₆haloalkenyl, C₂-C₆-alkynyl, C₂-C₆haloalkynyl, C₃-C₆cycloalkyl, C₁-C₆alkoxy, C₁-C₆haloalkoxy, C₁-C₆alkylthio, C₁-C₆alkylsulfinyl, C₁-C₆alkylsulfonyl, C₁-C₆haloalkyl, C₁-C₆haloalkylthio, C₁-C₆haloalkyl-sulfinyl, C₁-C₆haloalkylsulfonyl, C₁-C₆alkoxycarbonyl, C₁-C₆alkylcarbonyl, C₁-C₆alkylamino, di(C₁-C₆alkyl)amino, C₁-C₆alkylaminosulfonyl, di(C₁-C₆alkyl)aminosulfonyl, -NH-S-R₁₃, -N-(C₁-C₄alkylthio)-R₁₃, -NH-SO-R₁₄, -N-(C₁-C₄alkylsulfonyl)-R₁₄, -NH-SO₂-R₁₅, -N-(C₁-C₄alkylsulfonyl)-R₁₅, nitro, cyano, halogen, hydroxy, amino, formyl, rhodano-C₁-C₆alkyl; cyano-C₁-C₆alkyl, oxiranyl, C₃-C₆alkenyloxy, C₃-C₆alkynyloxy, C₁-C₆alkoxy-C₁-C₆alkoxy, cyano-C₁-C₆alkenyloxy, C₁-C₆alkoxycarbonyloxy-C₁-C₆alkoxy, C₃-C₆alkynyl-oxy, cyano-C₁-C₆alkoxy, C₁-C₆alkoxycarbonyl-C₁-C₆alkoxy, C₁-C₆alkylthio-C₁-C₆alkoxy, alkoxycarbonyl-C₁-C₆alkylthio, alkoxycarbonyl-C₁-C₆alkylsulfinyl, alkoxycarbonyl-C₁-C₆alkylsulfonyl, C₁-C₆alkylsulfonyloxy, C₁-C₆haloalkylsulfonyloxy, phenyl, benzyl, phenoxy, phenylthio, phenylsulfinyl, phenylsulfonyl, benzylthio, benzylsulfinyl or benzylsulfonyl, it being possible for the phenyl groups to be substituted one or more times by halogen, methyl, ethyl, trifluoromethyl, methoxy or by nitro;
or the group -R₁-X₁-R₂ together is a five- to ten-membered, monocyclic or fused bicyclic, ring system, which may be aromatic or partially saturated and which may contain from 1 to 4 hetero atoms selected from nitrogen, oxygen and sulfur, the ring system either being directly attached to the pyridine ring or being attached to the pyridine ring by way of a C₁-C₄alkylene group, and it being possible for each ring system to contain no more than 2 oxygen atoms and no more than two sulfur atoms, and/or to contain the group -C(=O)-, -C(=S)-, -C(=NR₂₀)-, -(N=O)-, -S(=O)- or -SO₂-;
and the ring system itself may be substituted one, two or three times by C₁-C₆alkyl, C₁-C₆-haloalkyl, C₃-C₆alkenyl, C₃-C₆haloalkenyl, C₃-C₆alkynyl, C₃-C₆haloalkynyl, C₁-C₆alkoxy, C₁-C₆haloalkoxy, C₃-C₆alkenyloxy, C₃-C₆alkynyloxy, mercapto, C₁-C₆alkylthio, C₁-C₆-haloalkylthio, C₃-C₆alkenylthio, C₃-C₆haloalkenylthio, C₃-C₆alkynylthio, C₂-C₅alkoxyalkyl-thio, C₃-C₅acetylalkylthio, C₃-C₆alkoxycarbonylalkylthio, C₂-C₄cyanoalkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, C₂-C₄dialkylaminosulfonyl, C₁-C₃alkylene-R₁₆, N(H)-C₁-C₆alkyl, N(H)-C₁-C₆alkoxy, N-(C₁-C₆alkyl)-C₁-C₆alkyl, N-(C₁-C₆alkyl)-C₁-C₆alkoxy, halogen, cyano, nitro, phenyl and by benzylthio, it being possible for phenyl and benzylthio to be in turn substituted on the phenyl ring by C₁-C₃alkyl, C₁-C₃haloalkyl, C₁-C₃alkoxy, C₁-C₃haloalkoxy, halogen, cyano or by nitro, and substituents on nitrogen in a heterocyclic ring being other than halogen; and
R₁₉ and R₂₀ are each independently of the other hydrogen, hydroxy, C₁-C₆alkyl, C₁-C₆-haloalkyl, C₁-C₆alkoxy, C₁-C₆alkylcarbonyl, C₁-C₆alkoxycarbonyl or C₁-C₆alkylsulfonyl.

The process according to the invention is especially suitable for the preparation of those compounds of formula I wherein R₁ is -CH₂-, -CH₂CH₂-, -CH₂CH₂CH₂-, -CF₂-, -CH=CHCH₂-, -CH(CH₃)- or -C≡CCH₂-, but preferably -CH₂-, the free valency on the left in each case being attached to the pyridine ring.

Preference is furthermore given to the preparation of those compounds of formula I wherein X₁ is oxygen, sulfonyl or a group -NR₁₈SO₂-, especially oxygen.

In accordance with the process according to the invention, special preference is given to the preparation of those compounds of formula I wherein R₂ is CH₃, CH₂CH₃, CH₂OCH₃, CH₂OCH₂CH₃, CH₂CH₂OCH₃, CH₂CH₂OCH₂CH₃, CH₂CF₃, propargyl, cyclopropylmethyl, benzyl, CH₂CH₂SO₂CH₃ or CH₂CH₂OCH₂CH₂OCH₃, but preferably CH₂CH₂OCH₃, with very special preference being given to those compounds wherein X₁ is oxygen and R₁ is -CH₂-.

In accordance with the process according to the invention, preference is given to the preparation of those compounds of formula I wherein R₀₅ is hydrogen, CH₃, CH₂Cl, CH₂Br or CH₂OCH₃, but especially hydrogen.

From that group, those compounds wherein R is ethoxy or methoxy may be prepared especially advantageously.

Furthermore, in accordance with the process according to the invention there may be advantageously prepared compounds of formula I wherein R₂ is

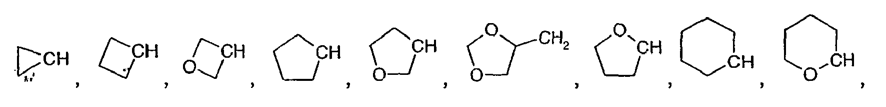

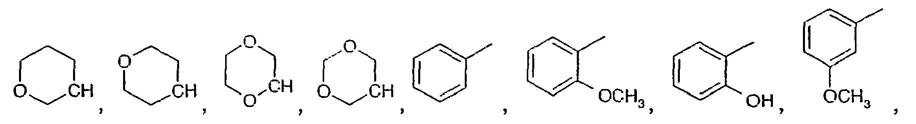

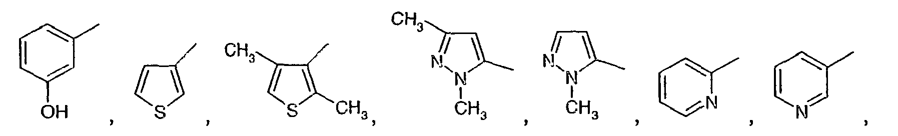

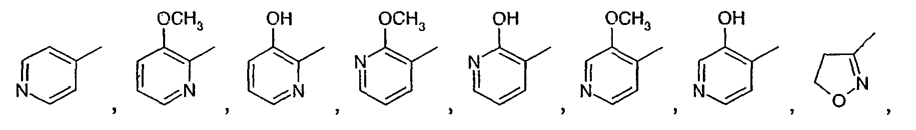

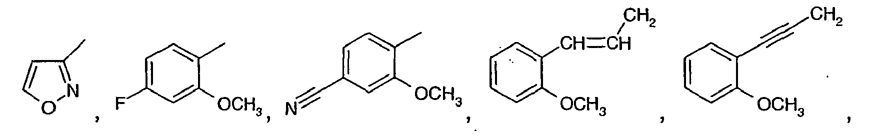

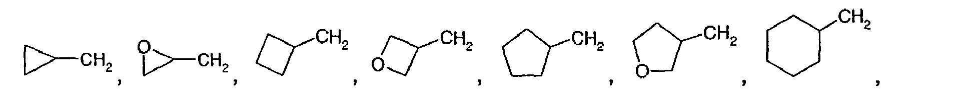

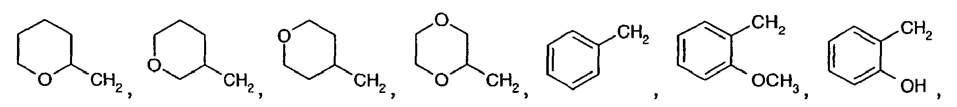

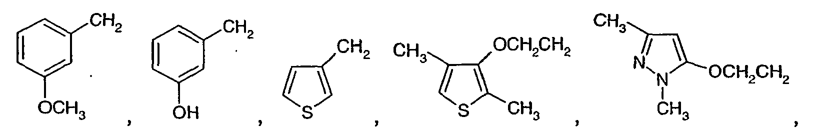

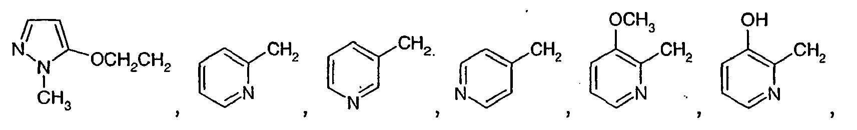

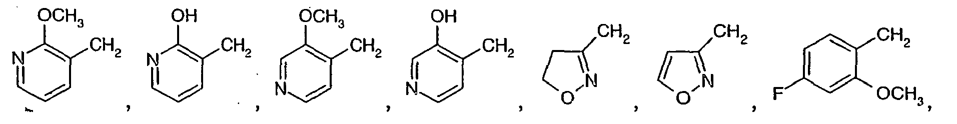

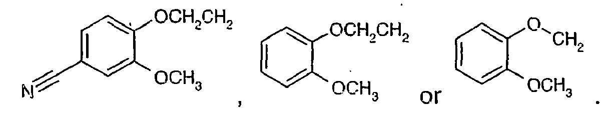

Where no free valency is indicated in those preferred meanings of R₂, as in the case of, for example, 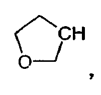 the attachment position is at the carbon atom marked "CH".

Furthermore, there may also be advantageously prepared those compounds wherein the group -R₁-X₁-R₂ together is a four- to ten-membered, monocyclic or fused bicyclic, ring system, which may be aromatic, partially saturated or saturated and contains from 1 to 4. hetero atoms selected from nitrogen, oxygen and sulfur and/or contains one or two groups selected from -C(=O)-, -C(=S)-, -C(=NR₂₀)- and -SO₂-, the ring system being attached to the pyridine ring via a carbon atom or preferably *via* a nitrogen atom by way of a C₁-C₄alkylene chain, especially a methylene chain. Among such ring systems special mention may be made of the following preferred, four- to seven-membered ring systems attached via a nitrogen atom to the methylene group, the attachment position being shown in each case at the bottom left: 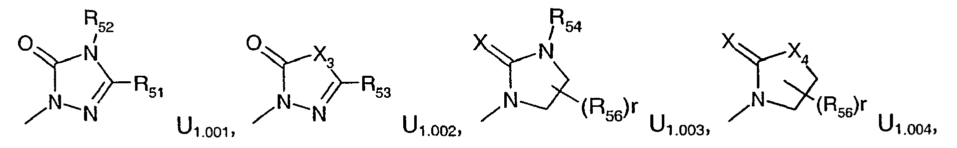 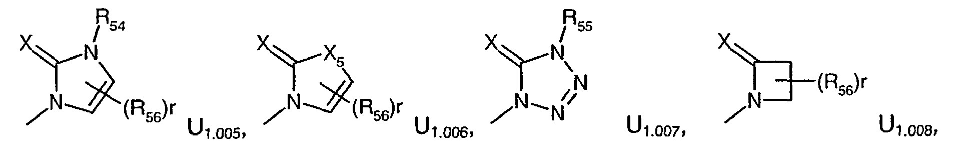 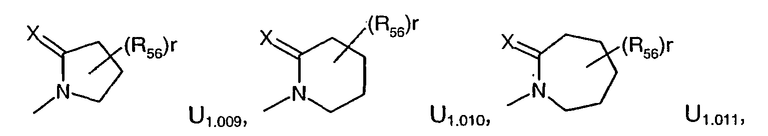 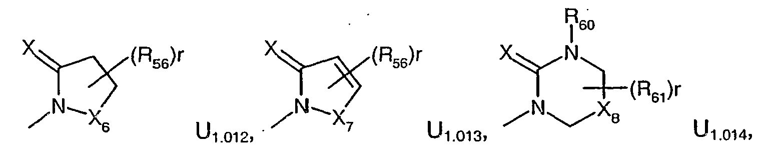 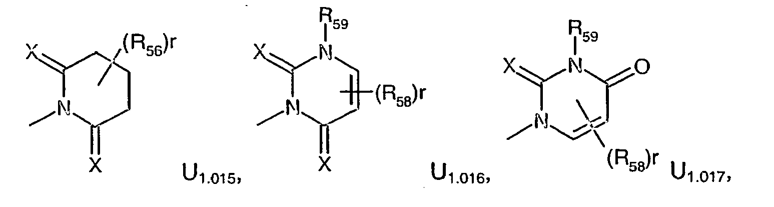 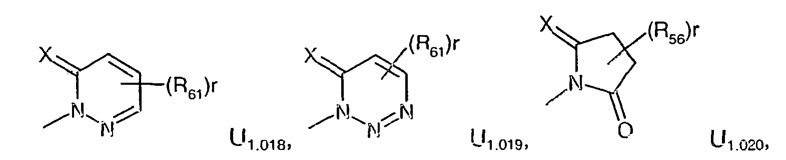 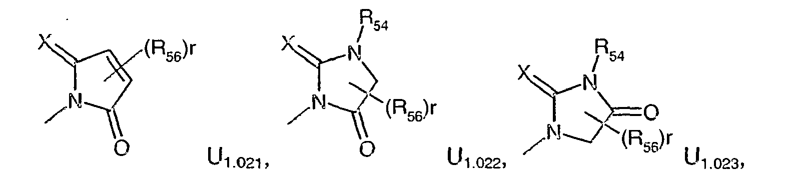 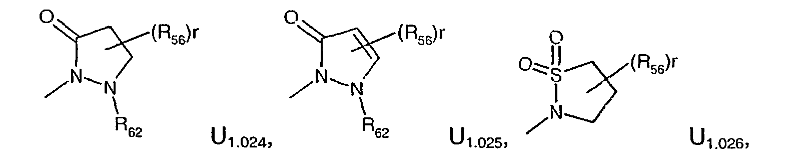 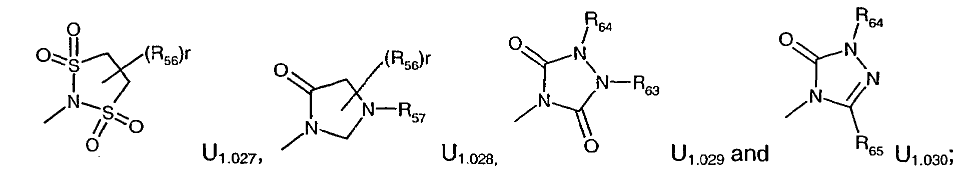 wherein r is 0, 1 or 2; R₅₁, R₅₃, R₅₆ and R₆₅ are each independently of the others hydrogen, halogen, C₁-C₆alkyl, C₁-C₆haloalkyl, C₃-C₆cycloalkyl, C₃-C₆alkenyl, C₃-C₆alkynyl, C₁-C₃alkoxy-C₁-C₃alkyl, C₁-C₆alkoxy, C₃-C₆alkenyloxy, C₃-C₆alkynyloxy, C₁-C₆alkylthio, C₁-C₆alkylsulfinyl, C₁-C₆alkylsulfonyl, C₃-C₆alkenylthio or C₃-C₆alkynylthio; R₅₂ is hydrogen, C₁-C₆alkyl, C₁-C₆haloalkyl, C₃-C₆cycloalkyl, C₃-C₆alkenyl, C₃-C₆alkynyl, C₁-C₆alkoxy, amino, or phenyl which may in turn be substituted by R₇₀; R₅₄, R₅₅ and R₆₀ are each independently of the others hydrogen, C₁-C₆alkyl, C₁-C₆haloalkyl, C₃-C₆alkenyl, C₃-C₆alkynyl or C₃-C₆cycloalkyl; R₅₇, R₆₃, R₆₆, R₆₇, R₆₈ and R₆₉ are each independently of the others C₁-C₆alkyl, or phenyl which may in turn be substituted by R₇₀; R₆₄ is C₁-C₆alkyl, C₁-C₆haloalkyl, C₃-C₆cycloalkyl, C₃-C₆alkenyl, C₃-C₆alkynyl, or phenyl which may in turn be substituted by R₇₀; R₅₈ and R₆₁ are hydrogen, halogen, C₁-C₆alkyl or C₁-C₆haloalkyl; R₅₉ is C₁-C₆alkyl, C₁-C₆haloalkyl, C₁-C₃alkoxy-C₁-C₃alkyl, C₃-C₆alkenyl or C₃-C₆alkynyl; R₆₂ is hydrogen, C₁-C₆alkyl, C₁-C₄alkoxycarbonyl or C₁-C₄alkylthiocarbonyl; or R₅₁ together with R₅₂, or R₅₄ together with an adjacent group R₅₆, or R₅₈ together with an adjacent group R₅₉, or R₆₀ together with an adjacent group R₆₁, or, when r is 2, two adjacent groups R₅₆ or two adjacent groups R₆₁ together may form a saturated or unsaturated C₁-C₅alkylene or C₃-C₄alkenylene bridge, which may in turn be substituted by a group R₇₀ or interrupted by oxygen, sulfur or nitrogen; each R₇₀ is independently halogen, C₁-C₃alkyl, C₁-C₃haloalkyl, hydroxy, C₁-C₃alkoxy, C₁-C₃haloalkoxy, cyano or nitro; X is oxygen, sulfur or NR₆; X₃, X₄ and X₅ are oxygen or sulfur; X₆ and X₇ are oxygen, sulfur, S(O) or SO₂; and X₈ is CH₂, oxygen, sulfur, S(O), SO₂ or NR₇₁, wherein R₇₁ is hydrogen or C₁-C₆alkyl.

In the context of the present invention, preference is given to the group R₁-X₁-R₂ together being C₁-C₆alkyl, C₁-C₆haloalkyl, C₂-C₆alkenyl, C₁-C₆haloalkenyl, C₂-C₆alkynyl, C₂-C₆haloalkynyl, C₃-C₆cycloalkyl, C₁-C₆alkoxy or C₁-C₆alkylthio, e.g. methyl, ethyl, n-propyl, isopropyl, cyclopropyl, n-butyl, fluoromethyl, 2-fluoroethyl, difluoroethyl, trifluoroethyl, vinyl, 1-propenyl, methoxy, ethoxy, methylthio or ethylthio.

In the context of the present invention, preference is given to R being methyl, ethyl, n-propyl or isopropyl, especially ethyl.

R₃ is preferably methyl or ethyl, very especially ethyl.

R₄ is preferably trifluoromethyl, difluoromethyl, chlorodifluoromethyl, pentafluoroethyl, 2,2,2-trifluoroethyl, especially trifluoromethyl, chlorodifluoromethyl or difluoromethyl.

As inert solvents for the method according to the invention there are suitable, for example, aromatic solvents such as benzene, chlorobenzene, fluorobenzene, xylenes, toluene, or alcohols such as methanol or ethanol, and also ethyl acetate, acetonitrile, dimethyl sulfoxide, dimethylformamide, dimethylacetamide, N-methyl-2-pyrrolidone, acetone, butanone, halogenated solvents such as, for example, methylene chloride, trichloromethane, dichloroethylene or trichlorethane, ethers such as tetrahydrofuran, diethyl ether, 1,2-dimethoxyethane, dioxane or methyl tert-butyl ether. Ethanol and toluene are especially preferred.

Organic or mineral acids are suitable as the proton source. Examples of suitable proton sources are HCl, HBr, H₂SO₄, carboxylic acids such as acetic acid and derivatives thereof such as trifluoroacetic acid and trichloroacetic acid, sulfonic acids such as methanesulfonic acid or p-toluenesulfonic acid and also carbonic acid. As the proton source for the process according to the invention special preference is given to trifluoroacetic acid.

The reactions can be carried out at ambient temperature or at elevated temperature. In general, addition of the reactants is carried out at a temperature from ambient temperature to the boiling point of the solvents, especially from 20 to 14.0°C, preferably from 40 to 120°C, with subsequent heating of the reaction mixture, advantageously to the boiling point of the solvent.

The compounds of formula II are known or are accessible by known methods. Processes for the preparation of compounds of formula II are described, for example, in J. Org Chem. (1995) vol 95, 3523, in H. Amil, T. Kobayashi, H. Terasawa, K. Uneyama, Org. Lett. 3(20), 3103-3105 (2001) and also A. Colla, G. Clar, S. Krimmer, P. Fischer, M.A.P. Martins, Synthesis-Stuttgart (6),483-486 (1991).

Some of the compounds of formula III are known. The preparation of such compounds is described in H. G. O. Becker, J. Prakt. Chem. (1961), Vol 12, 294., in WO 00/24714 and also in D.H. Wu, W. Wang, J. Labelled Compd. Rad 39(2),105-107(1997).

The compounds of formula III wherein -R₁-X₁-R₂ is -CH₂-O-CH₂-CH₂-O-CH₃, that is to say compounds of formula IIIa 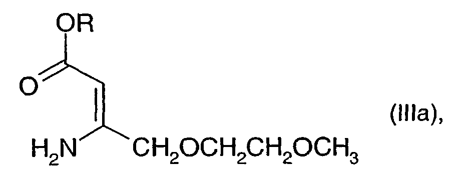 wherein R is as defined for formula III, are novel and were developed specifically for the preparation of compounds of formula I, and the present invention accordingly relates thereto. In a preferred compound of formula IIIa, R is methyl or ethyl.

Compounds of formula III can be prepared using processes known to the person skilled in the art, for example by reacting the unsaturated ketones on which they are based with ammonia gas as described in Preparation Example P1 hereinbelow.

In a preferred embodiment of the process according to the invention, the starting compounds of formula III are prepared from the 3-oxo-carboxylic acid esters on which they are based by introducing ammonia gas and then, without further isolation, reacting directly with the compounds of formula II. That process is especially advantageous for the large-scale preparation of compounds of formula I.

The compounds of formula I either may be used directly in the reaction mixture for further reactions or alternatively may be isolated. Isolation of the compounds of formula I can be carried out, for Example, by extraction of the reaction mixture, and subsequent removal of the solvent from the product-containing phase by customary methods.

The process according to the invention will be explained in greater detail in the hollowing Preparation Examples:

### Example P1: Preparation of 3-amino-4-methoxyethoxy-but-2-enoic acid ethyl ester:

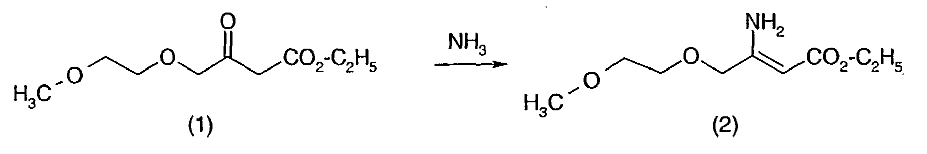

A mixture of 1.37 g (6 mmol) of 3-oxo-4-methoxyethoxy-butanoic acid ethyl ester (1) in 13 ml of ethanol is introduced into a reaction vessel and cooled to a temperature of 0°C using an ice/water bath.

Ammonia gas is then introduced for a period of 30 minutes, with stirring, and the reaction mixture is stirred for a further 20 minutes at a temperature of 0°C. After removing the cooling bath, the reaction mixture is allowed to warm up to a temperature of 20°C and ammonia gas is then introduced for a further hour. The reaction mixture is then stirred for 20 hours.

After removal of the solvent *in vacuo,* there are obtained 1.3 g (95 % of theory) of 3-amino-4-methoxyethoxy-but-2-enoic acid ethyl ester (2) in the form of an orange-coloured oil.

¹H NMR (CDCl₃): 1.30 (t, 3H, CH₃CH₂O-), 3.40 (s, 3H, CH₃O-), 3.55 (m, 2H, OCH₂CH₂O), 3.60 (m, 2H, OCH₂CH₂O), 4.10 (s, 2H, C=CCH₂O-), 4.15 (q, 2H, CH₃CH₂O-), 4.50 (s, 1H, CH=CNH₂).

¹³C NMR (CDCl₃): 14.7 (CH₃), 58.9 (CH₂), 59.2 (CH₃), 70.0 (CH₂), 71.0 (CH₂), 71.8 (CH₂), 81.9 (CH), 159.7 (C), 170.3 (C).

MS: 203 (M⁺), 158, 157, 144, 129, 114, 100, 98, 83, 71, 59, 45.

### Example P2: Preparation of 2-methoxyethoxymethyl-3-ethoxycarbonyl-6-trifluoromethylpyridine (4):

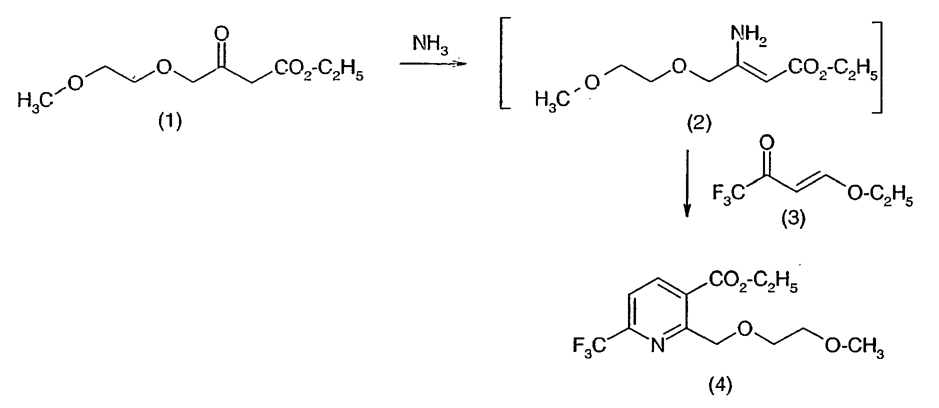

A mixture of 52.3 g (0.24 mol) of 3-oxo-4-methoxyethoxy-butanoic acid ethyl ester (1) in 150 ml of toluene is introduced into a reaction vessel equipped with a water separator.

Ammonia gas is then introduced into the reaction mixture for 2 hours, with stirring. Refluxing is then carried out for 30 minutes and the water is collected in the separator. After cooling the reaction mixture to a temperature of 20°C, the procedure is repeated. Ammonia gas is again introduced for 1.5 hours, with stirring, and the reaction mixture is then refluxed in order to separate off the water.

After cooling the reaction mixture, which contains 3-amino-4-methoxyethoxy-but-2-enoic acid ethyl ester (2), to a temperature of 20°C, 48 g (0.248 mol) of 1-ethoxy-3-oxo-4-trifluorobutene (3) are added and stirring is carried out at a temperature of 20°C for 18 hours. 1.5 ml of trifluoroacetic acid are then added, stirring is carried out at a temperature of 20°C for 2 hours and refluxing is carried out for a further 2 hours.

The reaction mixture is then allowed to cool down to a temperature of 20°C and is then washed with 100 ml of 1M NaHCO₃. The aqueous phase is separated off and is then extracted with 150 ml of toluene and the combined organic phases are then dried over MgSO₄.

After removal of the solvent *in vacuo*, there are obtained 65.4 g (62 % of theory) of 2-methoxyethoxymethyl-3-ethoxycarbonyl-6-trifluoromethylpyridine in the form of a dark-brown oil.

¹H NMR (CDCl₃): 1.40 (t, 3H, CH₃CH₂O-), 3.35 (s, 3H, CH₃O-), 3.55 (m, 2H, OCH₂CH₂O), 3.70 (m, 2H, OCH₂CH₂O), 4.45 (q, 2H, CH₃CH₂O-), 5.00 (s, 2H, ArCH₂O-), 7.70 (s, 1H, ArH), 8.30 (s, 1H, ArH).

### MS: 307 (M⁺), 262, 248, 233, 204, 202, 161, 128, 109, 59, 45

The other compounds listed in Table 1 can also be prepared in that manner.

In the following Table, the valency on the left of the radical R₁ is attached to the pyridine ring. When no free valency is indicated in the case of the substituent R₂, as in the case of, for example, 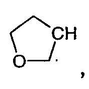 the attachment position is at the carbon atom marked "CH".

**Table 1: Compounds of formula la**

| | | | | |
|---|---|---|---|---|
| 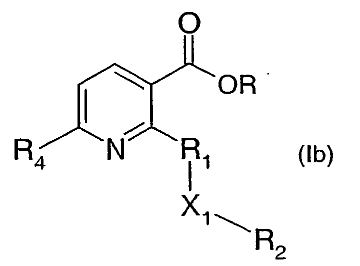 | | | | |
| wherein R is methyl or ethyl: | | | | |

| Comp. no. | R₄ | R₁ | R₂ | X₁ |
|---|---|---|---|---|
| | | | | |
| A1 | CF₃ | CH₂ | CH₃ | O |
| A2 | CF₃ | CH₂ | CH₂CH₃ | O |
| A3 | CF₃ | CH₂ | (CH₃)₂CH | O |
| A4 | CF₃ | CH₂ | PhCH₂ | O |
| A5 | CF₃ | CH₂ | CH₃ | S |
| A6 | CF₃ | CH₂ | CH₃ | SO |
| A7 | CF₃ | CH₂ | CH₃ | SO₂ |
| A8 | CF₃ | CH₂ | CH₃OCH₂ | O |
| A9 | CF₃ | CH₂ | CH₃CH₂OCH₂ | O |
| A10 | CF₃ | CH₂ | CH₃OCH₂CH₂ | O |
| A11 | CF₃ | CH₂ | CH₃CH₂OCH₂CH₂ | O |
| A12 | CF₃ | CH₂ | CH₃OC(CH₃)₂CH₂ | O |
| A13 | CF₃ | CH₂ | CH₃OCH(CH₃)CH₂ | O |
| A14 | CF₃ | CH₂ | CH₃OCH₂CH(CH₃) | O |
| A15 | CF₃ | CH₂ | CH₃OCH₂C(CH₃)₂ | O |
| A16 | CF₃ | CH₂ | CH₃OCH(CH₃) | O |
| A17 | CF3 | CH₂ | CH₃OC(CH₃)₂ | O |
| A18 | CF₃ | CH₂ | HC≡CCH₂ | O |
| A19 | CF₃ | CH₂ | H₂C=CHCH₂ | O |
| A20 | CF₃ | CH₂ | CH₃C≡CCH₂ | O |
| A21 | CF₃ | CH₂ | 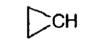 | O |
| A22 | CF₃ | CH₂CH₂CH₂ | 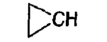 | O |
| A23 | CF₃ | CH₂ | 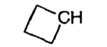 | O |
| A24 | CF₃ | CH₂ | 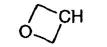 | O |
| A25 | CF₃ | CH₂ | 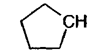 | O |
| A26 | CF₃ | CH₂ | 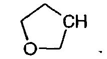 | O |
| A27 | CF₃ | CH₂ | 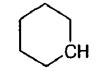 | O |
| A28 | CF₃ | CH₂ | 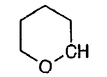 | O |
| A29 | CF₃ | CH₂ | 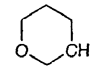 | O |
| A30 | CF₃ | CH₂ | 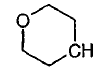 | O |
| A31 | CF₃ | CH₂ | 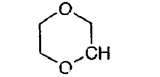 | O |
| A32 | CF₃ | CH₂ | 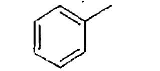 | O |
| A33 | CF₃ | CH₂ | 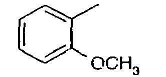 | O |
| A34 | CF₃ | CH₂ | 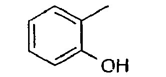 | O |
| A35 | CF₃ | CH₂ | 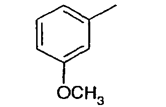 | O |
| A36 | CF₃ | CH₂ | 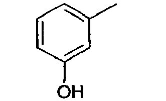 | O |
| A37 | CF₃ | CH₂ | 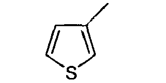 | O |
| A38 | CF₃ | CH₂ | 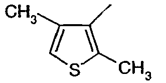 | O |
| A39 | CF₃ | CH₂ | 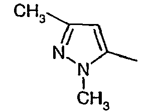 | O |
| A40 | CF₃ | CH₂ | 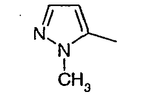 | O |
| A41 | CF₃ | CH₂ | 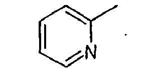 | O |
| A42 | CF₃ | CH₂ | 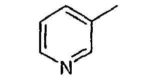 | O |
| A43 | CF₃ | CH₂ | 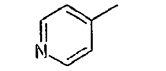 | O |
| A44 | CF₃ | CH₂ | 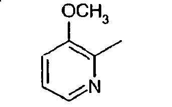 | O |
| A45 | CF₃ | CH₂ | 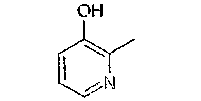 | O |
| A46 | CF₃ | CH₂ | 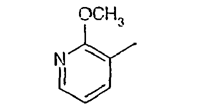 | O |
| A47 | CF₃ | CH₂ | 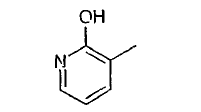 | O |
| A48 | CF₃ | CH₂ | 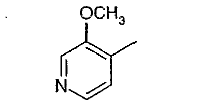 | O |
| A49 | CF₃ | CH₂ | 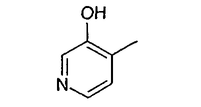 | O |
| A50 | CF₃ | CH₂ | 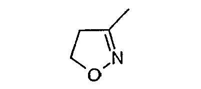 | O |
| A51 | CF₃ | CH₂ | 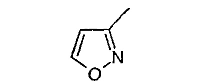 | O |
| A52 | CF₃ | CH₂ | 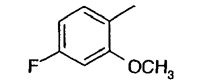 | O |
| A53 | CF₃ | CH₂ | 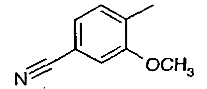 | O |
| A54 | CF₃ | CH₂ | 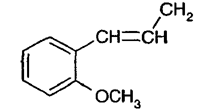 | O |
| A55 | CF₃ | CH₂ | 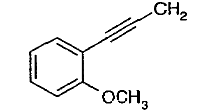 | O |
| A56 | CF₃ | CH₂ | 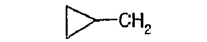 | O |
| A57 | CF₃ | CH₂ | 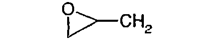 | O |
| A58 | CF₃ | CH₂ | 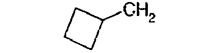 | O |
| A59 | CF₃ | CH₂ | 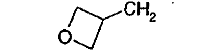 | O |
| A60 | CF₃ | CH₂ | 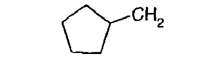 | O |
| A61 | CF₃ | CH₂ CH₂ | 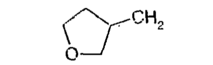 | O |
| A62 | CF₃ | CH₂ | 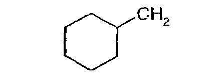 | O |
| A63 | CF₃ | CH₂ | 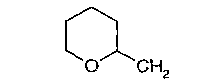 | O |
| A64 | CF₃ | CH₂ | 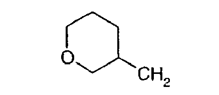 | O |
| A65 | CF₃ | CH₂ | 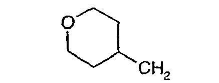 | O |
| A66 | CF₃ | CH₂ | 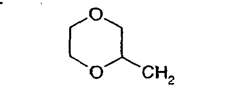 | O |
| A67 | CF₃ | CH₂ | 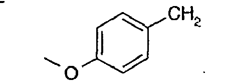 | O |
| A68 | CF₃ | CH₂ | 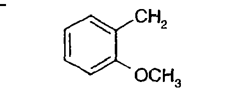 | O |
| A69 | CF₃ | CH₂ | 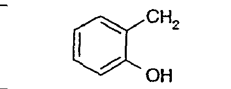 | O |
| A70 | CF₃ | CH₂ | 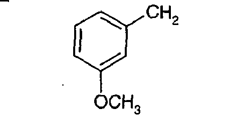 | O |
| A71 | CF₃ | CH₂ | 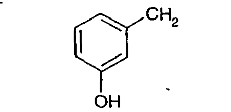 | O |
| A72 | CF₃ | CH₂ | 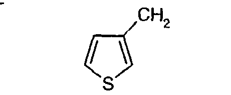 | O |
| A73 | CF₃ | CH₂ | 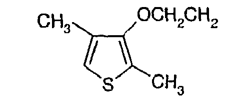 | O |
| A74 | CF₃ | CH₂ | 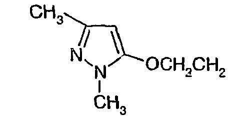 | O |
| A75 | CF₃ | CH₂ | 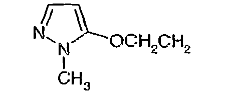 | O |
| A76 | CF₃ | CH₂ | 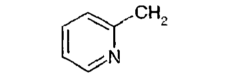 | O |
| A77 | CF₃ | CH₂ | 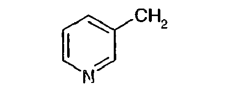 | O |
| A78 | CF₃ | CH₂ | 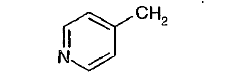 | O |
| A79 | CF₃ | CH₂ | 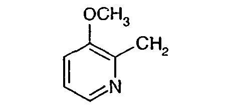 | O |
| A80 | CF₃ | CH₂ | 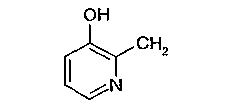 | O |
| A81 | CF₃ | CH₂ | 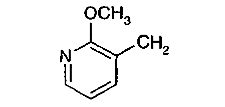 | O |
| A82 | CF₃ | CH₂ | 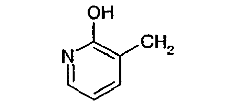 | O |
| A83 | CF₃ | CH₂ | 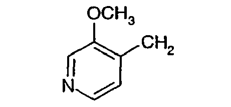 | O |
| A84 | CF₃ | CH₃ | 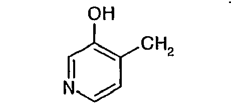 | O |
| A85 | CF₃ | CH₂ | 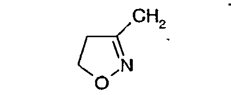 | O |
| A86 | CF₃ | CH₂ | 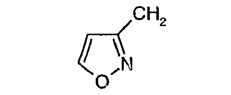 | O |
| A87 | CF₃ | CH₂ | 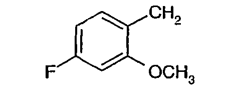 | O |
| A88 | CF₃ | CH₂ | 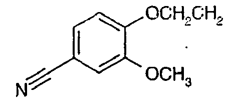 | O |
| A89 | CF₃ | CH₂ | 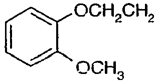 | O |
| A90 | CF₃ | CH₂ | 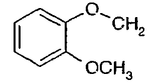 | O |
| A91 | CF₃ | CH₂CH₂ | CH₃ | O |
| A92 | CF₃ | CH₂CH₂ | CH₃CH₂ | O |
| A93 | CF₃ | CH₂CH₂ | (CH₃)₂CH | O |
| A94 | CF₃ | CH₂CH₂ | PhCH₂ | O |
| A95 | CF₃ | CH₂CH₂ | CH₃ | O |
| A96 | CF₃ | CH₂CH₂ | CH₃ | SO |
| A97 | CF₃ | CH₂CH₂ | CH₃ | SO₂ |
| A98 | CF₃ | CH₂CH₂ | (CH₃)₂CHCH₂ | O |
| A99 | CF₃ | CH₂CH₂ | CH₃OCH₂ | O |
| A100 | CF₃ | CH₂CH₂ | CH₃CH₂OCH₂ | O |
| A101 | CF₃ | CH₂CH₂ | CH₃OCH₂CH₂ | O |
| A102 | CF₃ | CH₂CH₂ | CH₃CH₂OCH₂CH₂ | O |
| A103 | CF₃ | CH₂CH₂ | CH₃OC(CH₃)₂CH₂ | O |
| A104 | CF₃ | CH₂CH₂ | CH₃OCH(CH₃)CH₂ | O |
| A105 | CF₃ | CH₂CH₂ | CH₃OCH₂CH(CH₃) | O |
| A106 | CF₃ | CH₂CH₂ | CH₃OCH₂C(CH₃)₂ | O |
| A107 | CF₃ | CH₂CH₂ | CH₃OCH(CH₃) | O |
| A108 | CF₃ | CH₂CH₂ | CH₃OC(CH₃)₂ | O |
| A109 | CF₃ | CH₂CH₂ | HC≡CCH₂ | O |
| A110 | CF₃ | CH₂CH₂ | H₂C=CHCH₂ | O |
| A111 | CF₃ | CH₂CH₂ | CH₃C≡CCH₂ | O |
| A112 | CF₃ | CH₂CH₂ | 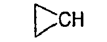 | O |
| A113 | CF₃ | CH₂CH₂CH₂ | 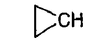 | O |
| A114 | CF₃ | CH₂CH₂ | 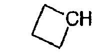 | O |
| A115 | CF₃ | CH₂CH₂ | 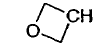 | O |
| A116 | CF₃ | CH₂CH₂ | 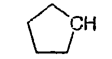 | O |
| A117 | CF₃ | CH₂CH₂ | 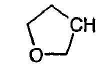 | O |
| A118 | CF₃ | CH₂CH₂ | 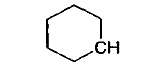 | O |
| A119 | CF₃ | CH₂CH₂ | 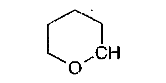 | O |
| A120 | CF₃ | CH₂CH₂ | 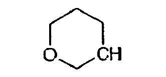 | O |
| A121 | CF₃ | CH₂CH₂ | 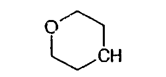 | O |
| A122 | CF₃ | CH₂CH₂ | 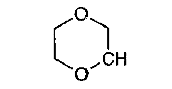 | O |
| A123 | CF₃ | CH₂CH₂ | 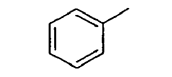 | O |
| A124 | CF₃ | CH₂CH₂ | 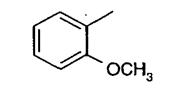 | O |
| A125 | CF₃ | CH₂CH₂ | 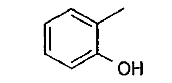 | O |
| A126 | CF₃ | CH₂CH₂ | 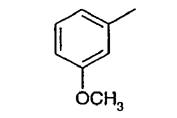 | O |
| A127 | CF₃ | CH₂CH₂ | 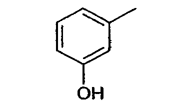 | O |
| A128 | CF₃ | CH₂CH₂ | 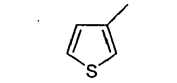 | O |
| A129 | CF₃ | CH₂CH₂ | 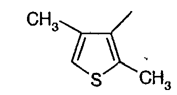 | O |
| A130 | CF₃ | CH₂CH₂ | 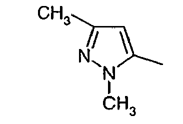 | O |
| A131 | CF₃ | CH₂CH₂ | 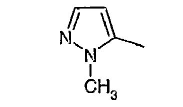 | O |
| A132 | CF₃ | CH₂CH₂ | 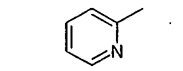 | O |
| A133 | CF₃ | CH₂CH₂ | 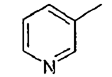 | O |
| A134 | CF₃ | CH₂CH₂ | 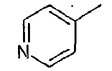 | O |
| A135 | CF₃ | CH₂CH₂ | 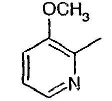 | O |
| A136 | CF₃ | CH₂CH₂ | 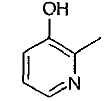 | O |
| A137 | CF₃ | CH₂CH₂ | 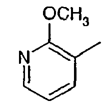 | O |
| A138 | CF₃ | CH₂CH₂ | 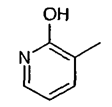 | O |
| A139 | CF₃ | CH₂CH₂ | 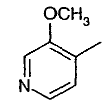 | O |
| A140 | CF₃ | CH₂CH₂ | 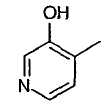 | O |
| A141 | CF₃ | CH₂CH₂ | 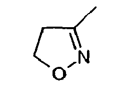 | O |
| A142 | CF₃ | CH₂CH₂ | 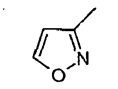 | O |
| A143 | CF₃ | CH₂CH₂ | 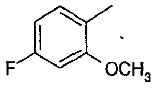 | O |
| A144 | CF₃ | CH₂CH₂ | 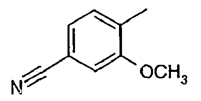 | O |
| A145 | CF₃ | CH₂CH₂ | 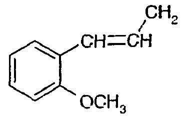 | O |
| A146 | CF₃ | CH₂CH₂ | 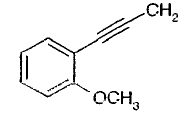 | O |
| A147 | CF₃ | CH₂CH₂ | 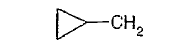 | O |
| A148 | CF₃ | CH₂CH₂ | 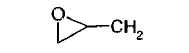 | O |
| A149 | CF₃ | CH₂CH₂ | 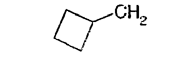 | O |
| A150 | CF₃ | CH₂CH₂ | 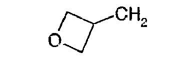 | O |
| A151 | CF₃ | CH₂CH₂ | 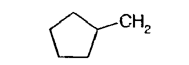 | O |
| A152 | CF₃ | CH₂CH₂ | 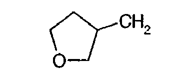 | O |
| A153 | CF₃ | CH₂CH₂ | 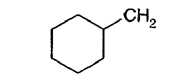 | O |
| A154 | CF₃ | CH₂CH₂ | 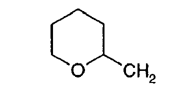 | O |
| A155 | CF₃ | CH₂CH₂ | 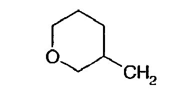 | O |
| A156 | CF₃ | CH₂CH₂ | 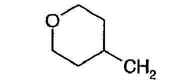 | O |
| A157 | CF₃ | CH₂CH₂ | 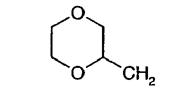 | O |
| A158 | CF₃ | CH₂CH₂ | 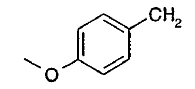 | O |
| A159 | CF₃ | CH₂CH₂ | 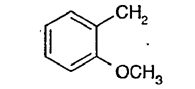 | O |
| A160 | CF₃ | CH₂CH₂ | 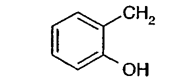 | O |
| A161 | CF₃ | CH₂CH₂ | 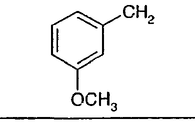 | O |
| A162 | CF₃ | CH₂CH₂ | 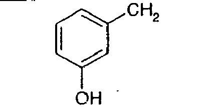 | O |
| A163 | CF₃ | CH₂CH₂ | 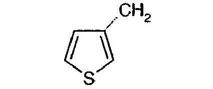 | O |
| A164 | CF₃ | CH₂CH₂ | 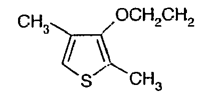 | O |
| A165 | CF₃ | CH₂CH₂ | 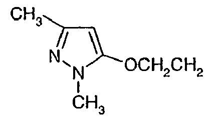 | O |
| A166 | CF₃ | CH₂CH₂ | 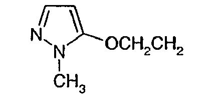 | O |
| A167 | CF₃ | CH₂CH₂ | 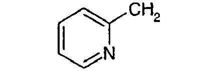 | O |
| A168 | CF₃ | CH₂CH₂ | 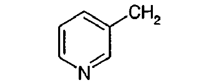 | O |
| A169 | CF₃ | CH₂CH₂ | 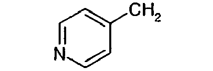 | O |
| A170 | CF₃ | CH₂CH₂ | 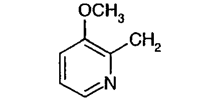 | O |
| A171 | CF₃ | CH₂CH₂ | 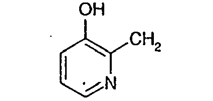 | O |
| A172 | CF₃ | CH₂CH₂ | 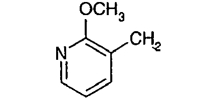 | O |
| A173 | CF₃ | CH₂CH₂ | 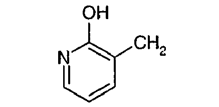 | O |
| A174 | CF₃ | CH₂CH₂ | 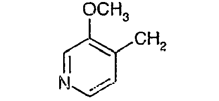 | O |
| A175 | CF₃ | CH₂CH₂ | 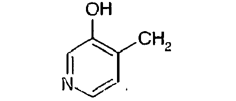 | O |
| A176 | CF₃ | CH₂CH₂ | 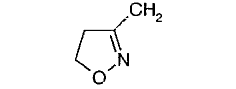 | O |
| A177 | CF₃ | CH₂CH₂ | 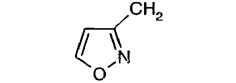 | O |
| A178 | CF₃ | CH₂CH₂ | 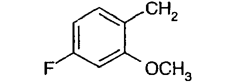 | O |
| A179 | CF₃ | CH₂CH₂ | 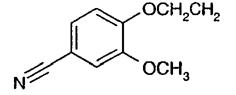 | O |
| A180 | CF₃ | CH₂CH₂ | 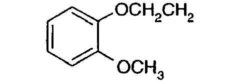 | O |
| A181 | CF₃ | CH₂CH₂ | 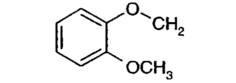 | O |
| A182 | CF₃ | CH(OCH₃)CH₂ | CH₃ | O |
| A183 | CF₃ | CH(OCH₃)CH₂ | CH₃CH₂ | O |
| A184 | CF₃ | CH(OCH₃)CH₂ | (CH₃)₂CH | O |
| A185 | CF₃ | CH(OCH₃)CH₂ | PhCH₂ | O |
| A186 | CF₃ | CH(OCH₃)CH₂ | CH₃ | S |
| A187 | CF₃ | CH(OCH₃)CH₂ | CH₃ | SO |
| A188 | CF₃ | CH(OCH₃)CH₂ | CH₃ | SO₂ |
| A189 | CF₃ | CH(OCH₃)CH₂ | CH₃CH₂CH₂ | O |
| A190 | CF₃ | CH(OCH₃)CH₂ | CH₃OCH₂ | O |
| A191 | CF₃ | CH(OCH₃)CH₂ | CH₃CH₂OCH₂ | O |
| A192 | CF₃ | CH(OCH₃)CH₂ | CH₃OCH₂CH₂ | O |
| A193 | CF₃ | CH(OCH₃)CH₂ | CH₃CH₂OCH₂CH₂ | O |
| A194 | CF₃ | CH(OCH₃)CH₂ | CH₃OC(CH₃)₂CH₂ | O |
| A195 | CF₃ | CH(OCH₃)CH₂ | CH₃OCH(CH₃)CH₂ | O |
| A196 | CF₃ | CH(OCH₃)CH₂ | CH₃OCH₂CH(CH₃) | O |
| A197 | CF₃ | CH(OCH₃)CH₂ | CH₃OCH₂C(CH₃)₂ | O |
| A198 | CF₃ | CH(OCH₃)CH₂ | CH₃OCH(CH₃) | O |
| A199 | CF₃ | CH(OCH₃)CH₂ | CH₃OC(CH₃)₂ | O |
| A200 | CF₃ | CH(OCH₃)CH₂ | HC≡CCH₂ | O |
| A201 | CF₃ | CH(OCH₃)CH₂ | H₂C=CHCH₂ | O |
| A202 | CF₃ | CH(OCH₃)CH₂ | CH₃C≡CCH₂ | O |
| A203 | CF₃ | CH(OCH₃)CH₂ | 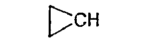 | O |
| A204 | CF₃ | CH₂CH₂CH₂ | 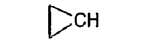 | O |
| A205 | CF₃ | CH(OCH₃)CH₂ | 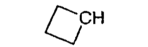 | O |
| A206 | CF₃ | CH(OCH₃)CH₂ | 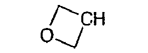 | O |
| A207 | CF₃ | CH(OCH₃)CH₂ | 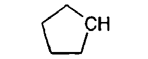 | O |
| A208 | CF₃ | CH(OCH₃)CH₂ | 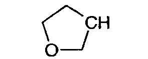 | O |
| A209 | CF₃ | CH(OCH₃)CH₂ | 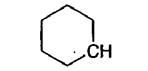 | O |
| A210 | CF₃ | CH(OCH₃)CH₂ | 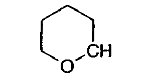 | O |
| A211 | CF₃ | CH(OCH₃)CH₂ | 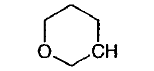 | O |
| A212 | CF₃ | CH(OCH₃)CH₂ | 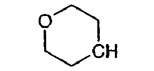 | O |
| A213 | CF₃ | CH(OCH₃)CH₂ | 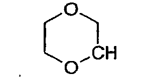 | O |
| A214 | CF₃ | CH(OCH₃)CH₂ | 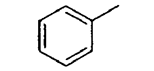 | O |
| A215 | CF₃ | CH(OCH₃)CH₂ | 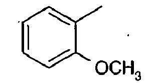 | O |
| A216 | CF₃ | CH(OCH₃)CH₂ | 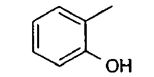 | O |
| A217 | CF₃ | CH(OCH₃)CH₂ | 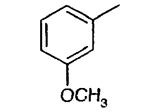 | O |
| A218 | CF₃ | CH(OCH₃)CH₂ | 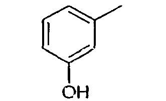 | O |
| A219 | CF₃ | CH(OCH₃)CH₂ | 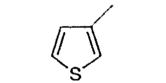 | O |
| A220 | CF₃ | CH(OCH₃)CH₂ | 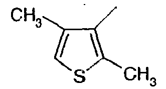 | O |
| A221 | CF₃ | CH(OCH₃)CH₂ | 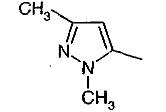 | O |
| A222 | CF₃ | CH(OCH₃)CH₂ | 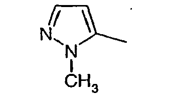 | O |
| A223 | CF₃ | CH(OCH₃)CH₂ | 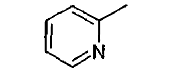 | O |
| A224 | CF₃ | CH(OCH₃)CH₂ | 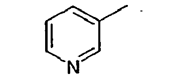 | O |
| A225 | CF₃ | CH(OCH₃)CH₂ | 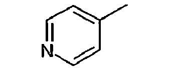 | O |
| A226 | CF₃ | CH(OCH₃)CH₂ | 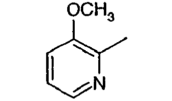 | O |
| A227 | CF₃ | CH(OCH₃)CH₂ | 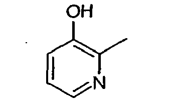 | O |
| A228 | CF₃ | CH(OCH₃)CH2 | 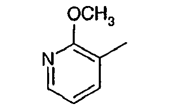 | O |
| A229 | CF₃ | CH(OCH₃)CH₂ | 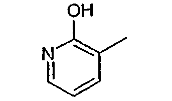 | O |
| A230 | CF₃ | CH(OCH₃)CH₂ | 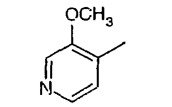 | O |
| A231 | CF₃ | CH(OCH₃)CH₂ | 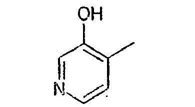 | O |
| A232 | CF₃ | CH(OCH₃)CH₂ | 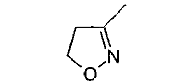 | O |
| A233 | CF₃ | CH(OCH₃)CH₂ | 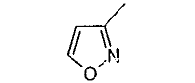 | O |
| A234 | CF₃ | CH(OCH₃)CH₂ | 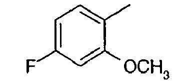 | O |
| A235 | CF₃ | CH(OCH₃)CH₂ | 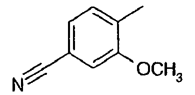 | O |
| A236 | CF₃ | CH(OCH₃)CH₂ | 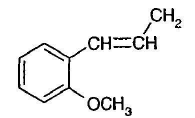 | O |
| A237 | CF₃ | CH(OCH₃)CH₂ | 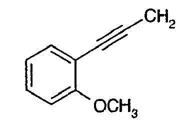 | O |
| A238 | CF₃ | CH(OCH₃)CH₂ | 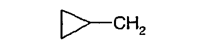 | O |
| A239 | CF₃ | CH(OCH₃)CH₂ | 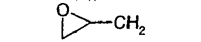 | O |
| A240 | CF₃ | CH(OCH₃)CH₂ | 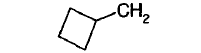 | O |
| A241 | CF₃ | CH(OCH₃)CH₂ | 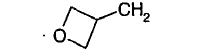 | O |
| A242 | CF₃ | CH(OCH₃)CH₂ | 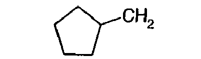 | O |
| A243 | CF₃ | CH(OCH₃)CH₂ | 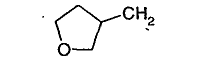 | O |
| A244 | CF₃ | CH(OCH₃)CH₂ | 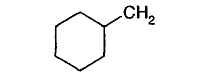 | O |
| A245 | CF₃ | CH(OCH₃)CH₂ | 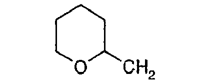 | O |
| A246 | CF₃ | CH(OCH₃)CH₂ | 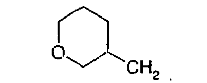 | O |
| A247 | CF₃ | CH(OCH₃)CH₂ | 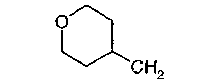 | O |
| A248 | CF₃ | CH(OCH₃)CH₂ | 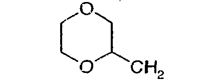 | O |
| A249 | CF₃ | CH(OCH₃)CH₂ | 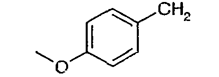 | O |
| A250 | CF₃ | CH(OCH₃)CH₂ | 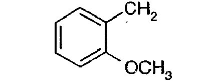 | O |
| A251 | CF₃ | CH(OCH₃)CH₂ | 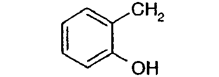 | O |
| A252 | CF₃ | CH(OCH₃)CH₂ | 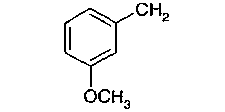 | O |
| A253 | CF₃ | CH(OCH₃)CH₂ | 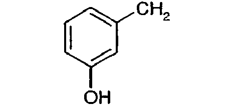 | O |
| A254 | CF₃ | CH(OCH₃)CH₂ | 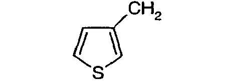 | O |
| A255 | CF₃ | CH(OCH₃)CH₂ | 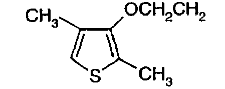 | O |
| A256 | CF₃ | CH(OCH₃)CH₂ | 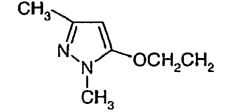 | O |
| A257 | CF₃ | CH(OCH₃)CH2 | 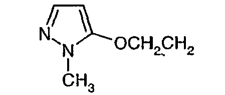 | O |
| A258 | CF₃ | CH(OCH₃)CH₂ | 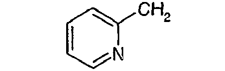 | O |
| A259 | CF₃ | CH(OCH₃)CH₂ | 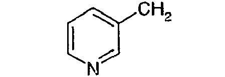 | O |
| A260 | CF₃ | CH(OCH₃)CH₂ | 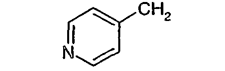 | O |
| A261 | CF₃ | CH(OCH₃)CH₂ | 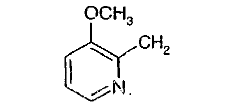 | O |
| A252 | CF₃ | CH(OCH₃)CH₂ | 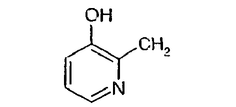 | O |
| A263 | CF₃ | CH(OCH₃)CH₂ | 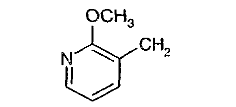 | O |
| A264 | CF₃ | CH(OCH₃)CH₂ | 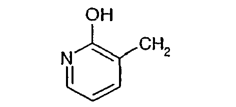 | O |
| A265 | CF₃ | CH(OCH₃)CH₂ | 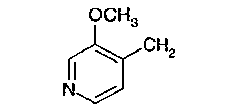 | O |
| A266 | CF₃ | CH(OCH₃)CH₂ | 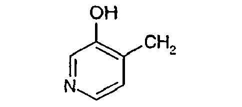 | O |
| A267 | CF₃ | CH(OCH₃)CH₂ | 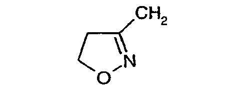 | O |
| A268 | CF₃ | CH(OCH₃)CH₂ | 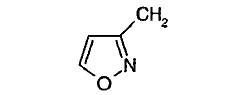 | O |
| A269 | CF₃ | CH(OCH₃)CH₂ | 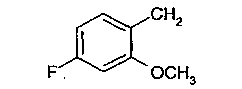 | O |
| A270 | CF₃ | CH(OCH₃)CH₂ | 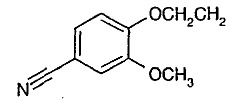 | O |
| A271 | CF₃ | CH(OCH₃)CH₂ | 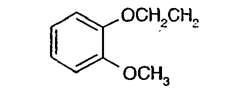 | O |
| A272 | CF₃ | CH(OCH₃)CH₂ | 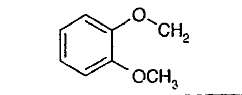 | O |
| A273 | CF₃ | CH₂CH(OCH₃)CH₂ | CH₃ | O |
| A274 | CF₃ | CH₂CH(OCH₃)CH₂ | CH₃CH₂ | O |
| A275 | CF₃ | CH₂CH(OCH₃)CH₂ | (CH₃)₂CH | O |
| A276 | CF₃ | CH₂CH(OCH₃)CH₂ | PhCH₂ | O |
| A277 | CF₃ CF₃ | CH₂CH(OCH₃)CH₂ | CH₃ CH₃ | S |
| A278 | CF₃ | CH₂CH(OCH₃)CH₂ | CH₃ | SO |
| A279 | CF₃ | CH₂CH(OCH₃)CH₂ | CH₃ | SO₂ |
| A280 | CF₃ | CH₂CH(OCH₃)CH₂ | CH₃CH₂CH₂ | O |
| A281 | CF₃ | CH₂CH(OCH₃)CH₂ | CH₃OCH₂ | O |
| A282 | CF₃ | CH₂CH(OCH₃)CH₂ | CH₃CH₂OCH₂ | O |
| A283 | CF₃ | CH₂CH(OCH₃)CH₂ | CH₃OCH₂CH₂ | O |
| A284 | CF₃ | CH₂CH(OCH₃)CH₂ | CH₃CH₂OCH₂CH₂ | O |
| A285 | CF₃ | CH₂CH(OCH₃)CH₂ | CH₃OC(CH₃)₂CH₂ | O |
| A286 | CF₃ | CH₂CH(OCH₃)CH₂ | CH₃OCH(CH₃)CH₂ | O |
| A287 | CF₃ | CH₂CH(OCH₃)CH₂ | CH₃OCH₂CH(CH₃) | O |
| A288 | CF₃ | CH₂CH(OCH₃)CH₂ | CH₃OCH₂C(CH₃)₂ | O |
| A289 | CF₃ | CH₂CH(OCH₃)CH₂ | CH₃OCH(CH₃) | O |
| A290 | CF₃ | CH₂CH(OCH₃)CH₂ | CH₃OC(CH₃)₂ | O |
| A291 | CF₃ | CH₂CH(OCH₃)CH₂ | HC≡CCH₂ | O |
| A292 | CF₃ | CH₂CH(OCH₃)CH₂ | H₂C=CHCH₂ | O |
| A293 | CF₃ | CH₂CH(OCH₃)CH₂ | CH₃C=CCH₂ | O |
| A294 | CF₃ | CH₂CH(OCH₃)CH₂ | 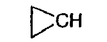 | O |
| A295 | CF₃ | CH₂CH₂CH₂ | 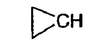 | O |
| A296 | CF₃ | CH₂CH(OCH₃)CH₂ | 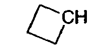 | O |
| A297 | CF₃ | CH₂CH(OCH₃)CH₂ | 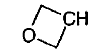 | O |
| A298 | CF₃ | CH₂CH(OCH₃)CH₂ | 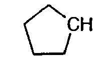 | O |
| A299 | CF₃ | CH₂CH(OCH₃)CH₂ | 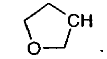 | O |
| A300 | CF₃ | CH₂CH(OCH₃)CH₂ | 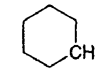 | O |
| A301 | CF₃ | CH₂CH(OCH₃)CH₂ | 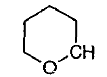 | O |
| A302 | CF₃ | CH₂CH(OCH₃)CH₂ | 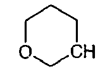 | O |
| A303 | CF₃ | CH₂CH(OCH₃)CH₂ | 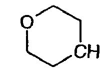 | O |
| A304 | CF₃ | CH₂CH(OCH₃)CH₂ | 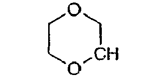 | O |
| A305 | CF₃ | CH₂CH(OCH₃)CH₂ | 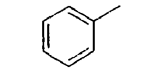 | O |
| A306 | CF₃ | CH₂CH(OCH₃)CH₂ | 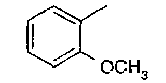 | O |
| A307 | CF₃ | CH₂CH(OCH₃)CH₂ | 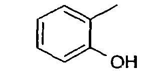 | O |
| A308 | CF₃ | CH₂CH(OCH₃)CH₂ | 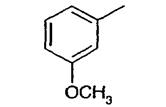 | O |
| A309 | CF₃ | CH₂CH(OCH₃)CH₂ | 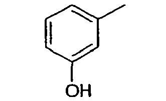 | O |
| A310 | CF₃ | CH₂CH(OCH₃)CH₂ | 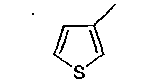 | O |
| A311 | CF₃ | CH₂CH(OCH₃)CH₂ | 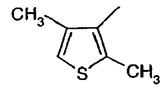 | O |
| A312 | CF₃ | CH₂CH(OCH₃)CH₂ | 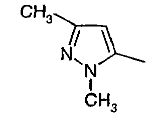 | O |
| A313 | CF₃ | CH₂CH(OCH₃)CH₂ | 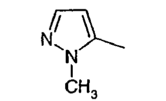 | O |
| A314 | CF₃ | CH₂CH(OCH₃)CH₂ | 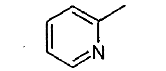 | O |
| A315 | CF₃ | CH₂CH(OCH₃)CH₂ | 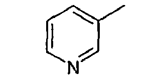 | O |
| A316 | CF₃ | CH₂CH(OCH₃)CH₂ | 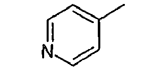 | O |
| A317 | CF₃ | CH₂CH(OCH₃)CH₂ | 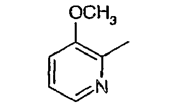 | O |
| A318 | CF₃ | CH₂CH(OCH₃)CH₂ | 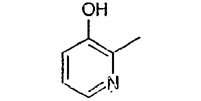 | O |
| A319 | CF₃ | CH₂CH(OCH₃)CH₂ | 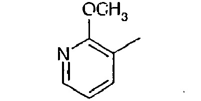 | O |
| A320 | CF₃ | CH₂CH(OCH₃)CH₂ | 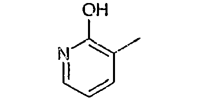 | O |
| A321 | CF₃ | CH₂CH(OCH₃)CH₂ | 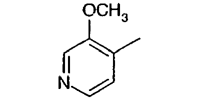 | O |
| A322 | CF₃ | CH₂CH(OCH₃)CH₂ | 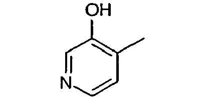 | O |
| A323 | CF₃ | CH₂CH(OCH₃)CH₂ | 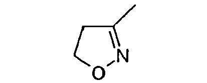 | O |
| A324 | CF₃ | CH₂CH(OCH₃)CH₂ | 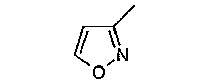 | O |
| A325 | CF₃ | CH₂CH(OCH₃)CH₂ | 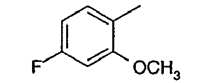 | O |
| A326 | CF₃ | CH₂CH(OCH₃)CH₂ | 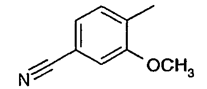 | O |
| A327 | CF₃ | CH₂CH(OCH₃)CH₂ | 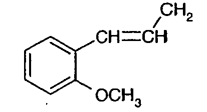 | O |
| A328 | CF₃ | CH₂CH(OCH₃)CH₂ | 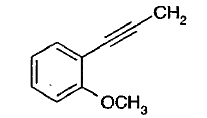 | O |
| A329 | CF₃ | CH₂CH(OCH₃)CH₂ | 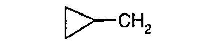 | O |
| A330 | CF₃ | CH₂CH(OCH₃)CH₂ | 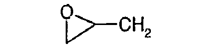 | O |
| A331 | CF₃ | CH₂CH(OCH₃)CH₂ | 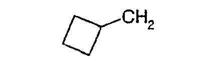 | O |
| A332 | CF₃ | CH₂CH(OCH₃)CH₂ | 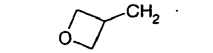 | O |
| A333 | CF₃ | CH₂CH(OCH₃)CH₂ | 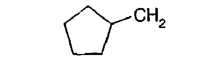 | O |
| A334 | CF₃ | CH₂CH(OCH₃)CH₂ | 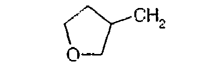 | O |
| A335 | CF₃ | CH₂CH(OCH₃)CH₂ | 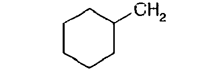 | O |
| A336 | CF₃ | CH₂CH(OCH₃)CH₂ | 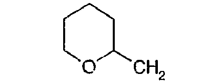 | O |
| A337 | CF₃ | CH₂CH(OCH₃)CH₂ | 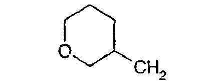 | O |
| A338 | CF₃ | CH₂CH(OCH₃)CH₂ | 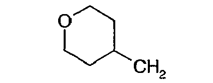 | O |
| A339 | CF₃ | CH₂CH(OCH₃)CH₂ | 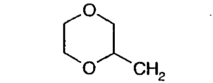 | O |
| A340 | CF₃ | CH₂CH(OCH₃)CH₂ | 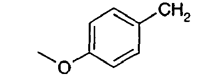 | O |
| A341 | CF₃ | CH₂CH(OCH₃)CH₂ | 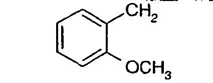 | O |
| A342 | CF₃ | CH₂CH(OCH₃)CH₂ | 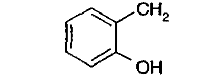 | O |
| A343 | CF₃ | CH₂CH(OCH₃)CH₂ | 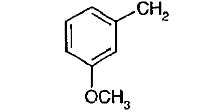 | O |
| A344 | CF₃ | CH₂CH(OCH₃)CH₂ | 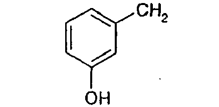 | O |
| A345 | CF₃ | CH₂CH(OCH₃)CH₂ | 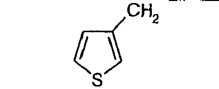 | O |
| A346 | CF₃ | CH₂CH(OCH₃)CH₂ | 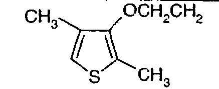 | O |
| A347 | CF₃ | CH₂CH(OCH₃)CH₂ | 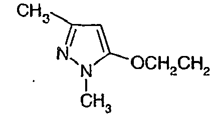 | O |
| A348 | CF₃ | CH₂CH(OCH₃)CH₂ | 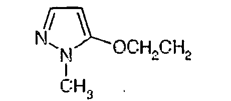 | O |
| A349 | CF₃ | CH₂CH(OCH₃)CH₂ | 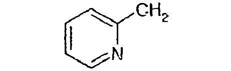 | O |
| A350 | CF₃ | CH₂CH(OCH₃)CH₂ | 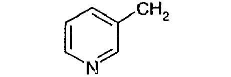 | O |
| A351 | CF₃ | CH₂CH(OCH₃)CH₂ | 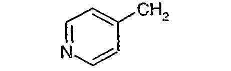 | O |
| A352 | CF₃ | CH₂CH(OCH₃)CH₂ | 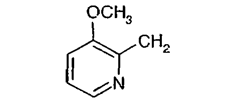 | O |
| A353 | CF₃ | CH₂CH(OCH₃)CH₂ | 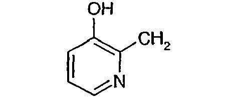 | O |
| A354 | CF₃ | CH₂CH(OCH₃)CH₂ | 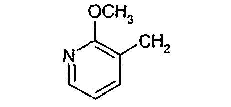 | O |
| A355 | CF₃ | CH₂CH(OCH₃)CH₂ | 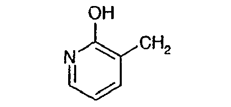 | O |
| A356 | CF₃ | CH₂CH(OCH₃)CH₂ | 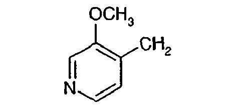 | O |
| A357 | CF₃ | CH₂CH(OCH₃)CH₂ | 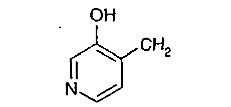 | O |
| A358 | CF₃ | CH₂CH(OCH₃)CH₂ | 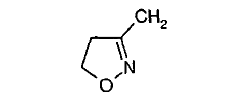 | O |
| A359 | CF₃ | CH₂CH(OCH₃)CH₂ | 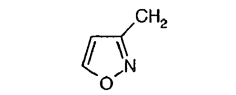 | O |
| A360 | CF₃ | CH₂CH(OCH₃)CH₂ | 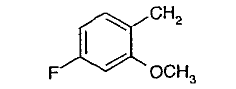 | O |
| A361 | CF₃ | CH₂CH(OCH₃)CH₂ | 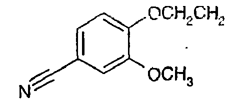 | O |
| A362 | CF₃ | CH₂CH(OCH₃)CH₂ | 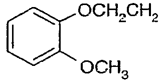 | O |
| A363 | CF₃ | CH₂CH(OCH₃)CH₂ | 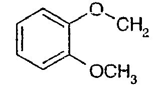 | O |
| A364 | CF₃ | CH=CHCH₂ | CH₃ | O |
| A365 | CF₃ | CH=CHCH₂ | CH₃CH₂ | O |
| A366 | CF₃ | CH=CHCH₂ | (CH₃)₂CH | O |
| A367 | CF₃ | CH=CHCH₂ | PhCH₂ | O |
| A368 | CF₃ | CH=CHCH₂ | CH₃ | S |
| A369 | CF₃ | CH=CHCH₂ | CH₃ | SO |
| A370 | CF₃ | CH=CHCH₂ | CH₃ | SO₂ |
| A371 | CF₃ | CH=CHCH₂ | CH₃CH₂CH₂ | O |
| A372 | CF₃ | CH=CHCH₂ | CH₃OCH₂ | O |
| A373 | CF₃ | CH=CHCH₂ | CH₃CH₂OCH₂ | O |
| A374 | CF₃ | CH=CHCH₂ | CH₃OCH₂CH₂ | O |
| A375 | CF₃ | CH=CHCH₂ | CH₃CH₂OCH₂CH₂ | O |
| A376 | CF₃ | CH=CHCH₂ | CH₃OC(CH₃)CH₂ | O |
| A377 | CF₃ | CH=CHCH₂ | CH₃OCH(CH₃)₂CH₂ | O |
| A378 | CF₃ | CH=CHCH₂ | CH₃OCH₂CH(CH₃) | O |
| A379 | CF₃ | CH=CHCH₂ | CH₃OCH₂C(CH₃)₂ | O |
| A380 | CF₃ | CH=CHCH₂ | CH₃OCH(CH₃) | O |
| A381 | CF₃ | CH=CHCH₂ | CH₃OC(CH₃)₂ | O |
| A382 | CF₃ | CH=CHCH₂ | HC≡CCH₂ | O |
| A383 | CF₃ | CH=CHCH₂ | H₂C=CHCH₂ | O |
| A384 | CF₃ | CH=CHCH₂ | CH₃C≡CCH₂ | O |
| A385 | CF₃ | CH=CHCH₂ | 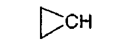 | O |
| A386 | CF₃ | CH=CHCH₂ | 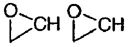 | O |
| A387 | CF₃ | CH=CHCH₂ | 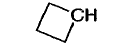 | O |
| A388 | CF₃ | CH=CHCH₂ | 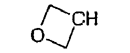 | O |
| A389 | CF₃ | CH=CHCH₂ | 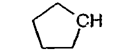 | O |
| A390 | CF₃ | CH=CHCH₂ | 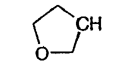 | O |
| A391 | CF₃ | CH=CHCH₂ | 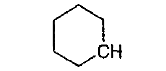 | 0 |
| A392 | CF₃ | CH=CHCH₂ | 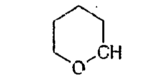 | 0 |
| A393 | CF₃ | CH=CHCH₂ | 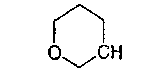 | 0 |
| A394 | CF₃ | CH=CHCH₂ | 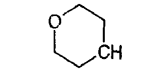 | 0 |
| A395 | CF₃ | CH=CHCH₂ | 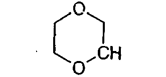 | 0 |
| A396 | CF₃ | CH=CHCH₂ | 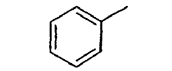 | 0 |
| A397 | CF₃ | CH=CHCH₂ | 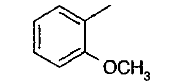 | 0 |
| A398 | CF₃ | CH=CHCH₂ | 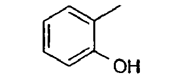 | 0 |
| A399 | CF₃ | CH=CHCH₂ | 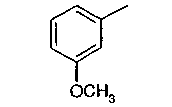 | 0 |
| A400 | CF₃ | CH=CHCH₂ | 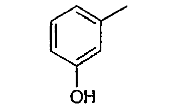 | 0 |
| A401 | CF₃ | CH=CHCH₂ | 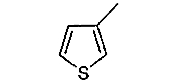 | 0 |
| A402 | CF₃ | CH=CHCH₂ | 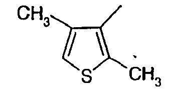 | 0 |
| A403 | CF₃ | CH=CHCH₂ | 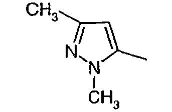 | 0 |
| A404 | CF₃ | CH=CHCH₂ | 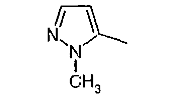 | 0 |
| A405 | CF₃ | CH=CHCH₂ | 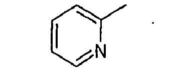 | 0 |
| A406 | CF₃ | CH=CHCH₂ | 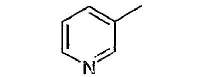 | O |
| A407 | CF₃ | CH=CHCH₂ | 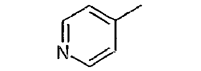 | O |
| A408 | CF₃ | CH=CHCH₂ | 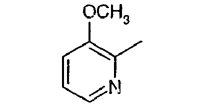 | O |
| A409 | CF₃ | CH=CHCH₂ | 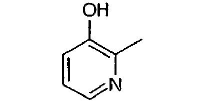 | O |
| A410 | CF₃ | CH=CHCH₂ | 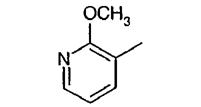 | O |
| A411 | CF₃ | CH=CHCH₂ | 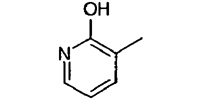 | O |
| A412 | CF₃ | CH=CHCH₂ | 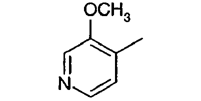 | O |
| A413 | CF₃ | CH=CHCH₂ | 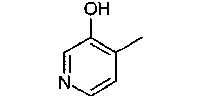 | O |
| A414 | CF₃ | CH=CHCH₂ | 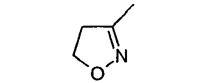 | O |
| A415 | CF₃ | CH=CHCH₂ | 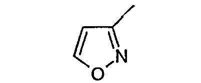 | O |
| A416 | CF₃ | CH=CHCH₂ | 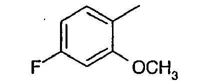 | O |
| A417 | CF₃ | CH=CHCH₂ | 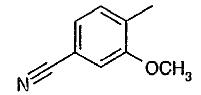 | O |
| A418 | CF₃ | CH=CHCH₂ | 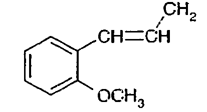 | O |
| A419 | CF₃ | CH=CHCH₂ | 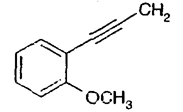 | O |
| A420 | CF₃ | CH=CHCH₂ | 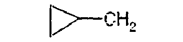 | O |
| A421 | CF₃ | CH=CHCH₂ | 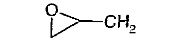 | O |
| A422 | CF₃ | CH=CHCH₂ | 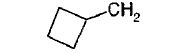 | O |
| A423 | CF₃ | CH=CHCH₂ | 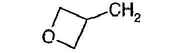 | O |
| A424 | CF₃ | CH=CHCH₂ | 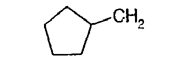 | O |
| A425 | CF₃ | CH=CHCH₂ | 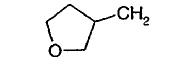 | O |
| A426 | CF₃ | CH=CHCH₂ | 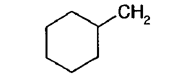 | O |
| A427 | CF₃ | CH=CHCH₂ | 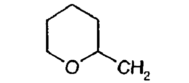 | O |
| A428 | CF₃ | CH=CHCH₂ | 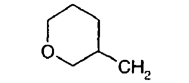 | O |
| A429 | CF₃ | CH=CHCH₂ | 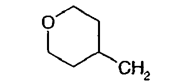 | O |
| A430 | CF₃ | CH=CHCH₂ | 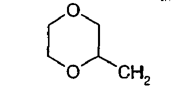 | O |
| A431 | CF₃ | CH=CHCH₂ | 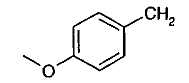 | O |
| A432 | CF₃ | CH=CHCH₂ | 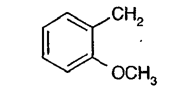 | O |
| A433 | CF₃ | CH=CHCH₂ | 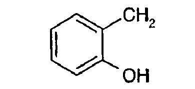 | O |
| A434 | CF₃ | CH=CHCH₂ | 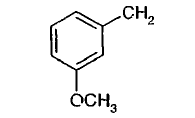 | O |
| A435 | CF₃ | H=CHCH₂ | 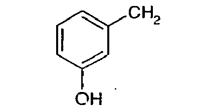 | O |
| A436 | CF₃ | CH=CHCH₂ | 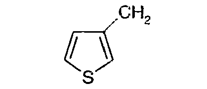 | O |
| A437 | CF₃ | CH=CHCH₂ | 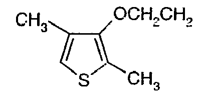 | O |
| A438 | CF₃ | CH=CHCH₂ | 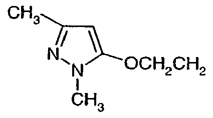 | O |
| A439 | CF₃ | CH=CHCH₂ | 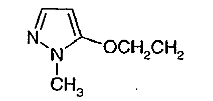 | O |
| A440 | CF₃ | CH=CHCH₂ | 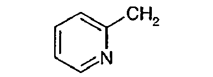 | O |
| A441 | CF₃ | CH=CHCH₂ | 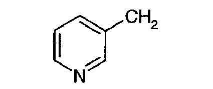 | O |
| A442 | CF₃ | CH=CHCH₂ | 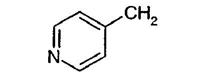 | O |
| A443 | CF₃ | CH=CHCH₂ | 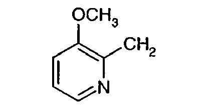 | O |
| A444 | CF₃ | CH=CHCH₂ | 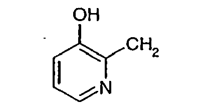 | O |
| A445 | CF₃ | CH=CHCH₂ | 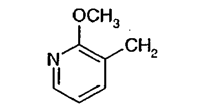 | O |
| A446 | CF₃ | CH=CHCH₂ | 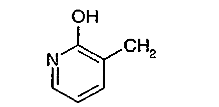 | O |
| A447 | CF₃ | CH=CHCH₂ | 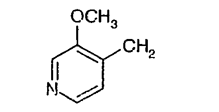 | O |
| A448 | CF₃ | CH=CHCH₂ | 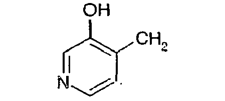 | O |
| A449 | CF₃ | CH=CHCH₂ | 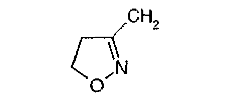 | O |
| A450 | CF₃ | CH=CHCH₂ | 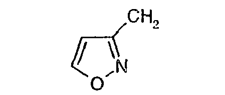 | O |
| A451 | CF₃ | CH=CHCH₂ | 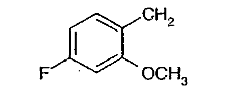 | O |
| A452 | CF₃ | CH=CHCH₂ | 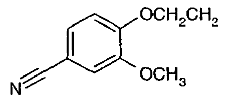 | O |
| A453 | CF₃ | CH=CHCH₂ | 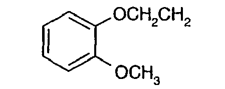 | O |
| A454 | CF₃ | CH=CHCH₂ | 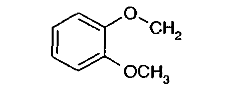 | O |
| A455 | CF₃ | C≡CCH₂ | CH₃ | O |
| A456 | CF₃ | C≡CCH₂ | CH₃CH₂ | O |
| A457 | CF₃ | C≡CCH₂ | (CH₃)₂CH | O |
| A458 | CF₃ | C≡CCH₂ | PhCH₂ | O |
| A459 | CF₃ | C≡CCH₂ | CH₃ | S |
| A460 | CF₃ | C≡CCH₂ | CH₃ | SO |
| A461 | CF₃ | C≡CCH₂ | CH₃ | SO₂ |
| A462 | CF₃ | C≡CCH₂ | CH₃CH₂CH₂ | O |
| A463 | CF₃ | C≡CCH₂ | CH₃OCH₂ | O |
| A464 | CF₃ | C≡CCH₂ | CH₃CH₂OCH₂ | O |
| A465 | CF₃ | C≡CCH₂ | CH₃OCH₂CH₂ | O |
| A466 | CF₃ | C≡CCH₂ | CH₃CH₂OCH₂CH₂ | O |
| A467 | CF₃ | C≡CCH₂ | CH₃OC(CH₃)₂CH₂ | O |
| A468 | CF₃ | C≡CCH₂ | CH₃OCH(CH₃)CH₂ | O |
| A469 | CF₃ | C≡CCH₂ | CH₃OCH₂CH(CH₃) | O |
| A470 | CF₃ | C≡CCH₂ | CH₃OCH₂C(CH₃)₂ | O |
| A471 | CF₃ | C≡CCH₂ | CH₃OCH(CH₃) | O |
| A472 | CF₃ | C≡CCH₂ | CH₃OC(CH₃)₂ | O |
| A473 | CF₃ | C≡CCH₂ | HC≡CCH₂ | O |
| A474 | CF₃ | C≡CCH₂ | H₂C=CHCH₂ | O |
| A475 | CF₃ | C≡CCH₂ | CH₃C≡CCH₂ | O |
| A476 | CF₃ | C≡CCH₂ | 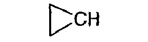 | O |
| A477 | CF₃ | C≡CCH₂ | 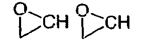 | O |
| A478 | CF₃ | C≡CCH₂ | 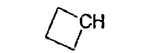 | O |
| A479 | CF₃ | C≡CCH₂ | 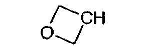 | O |
| A480 | CF₃ | C≡CCH₂ | 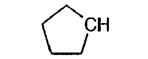 | O |
| A481 | CF₃ | C≡CCH₂ | 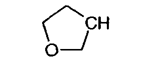 | O |
| A482 | CF₃ | C≡CCH₂ | 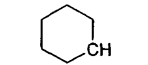 | O |
| A483 | CF₃ | C≡CCH₂ | 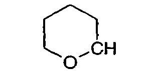 | O |
| A484 | CF₃ | C≡CCH₂ | 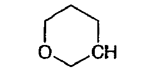 | O |
| A485 | CF₃ | C≡CCH₂ | 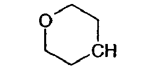 | O |
| A486 | CF₃ | C≡CCH₂ | 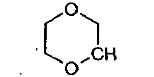 | O |
| A487 | CF₃ | C≡CCH₂ | 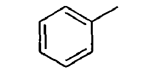 | O |
| A488 | CF₃ | C≡CCH₂ | 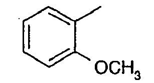 | O |
| A489 | CF₃ | C≡CCH₂ | 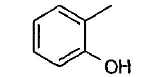 | O |
| A490 | CF₃ | C≡CCH₂ | 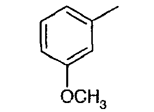 | O |
| A491 | CF₃ | C≡CCH₂ | 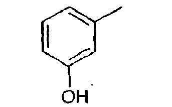 | O |
| A492 | CF₃ | C≡CCH₂ | 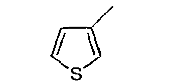 | O |
| A493 | CF₃ | C≡CCH₂ | 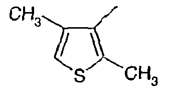 | O |
| A494 | CF₃ | C≡CCH₂ | 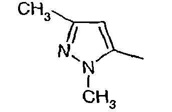 | O |
| A495 | CF₃ | C≡CCH₂ | 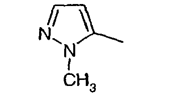 | O |
| A496 | CF₃ | C≡CCH₂ | 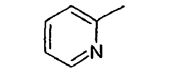 | O |
| A497 | CF₃ | C≡CCH₂ | 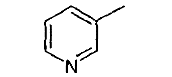 | O |
| A498 | CF₃ | C≡CCH₂ | 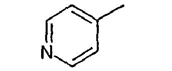 | O |
| A499 | CF₃ | C≡CCH₂ | 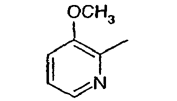 | O |
| A500 | CF₃ | C≡CCH₂ | 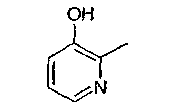 | O |
| A501 | CF₃ | C≡CCH₂ | 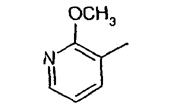 | O |
| A502 | CF₃ | C≡CCH₂ | 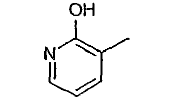 | O |
| A503 | CF₃ | C≡CCH₂ | 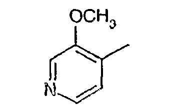 | O |
| A504 | CF₃ | C≡CCH₂ | 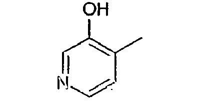 | O |
| A505 | CF₃ | C≡CCH₂ | 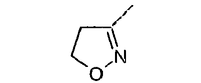 | O |
| A506 | CF₃ | C≡CCH₂ | 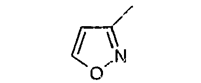 | O |
| A507 | CF₃ | C≡CCH₂ | 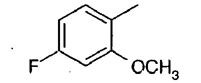 | O |
| A508 | CF₃ | C≡CCH₂ | 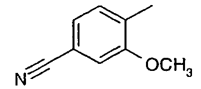 | O |
| A509 | CF₃ | C≡CCH₂ | 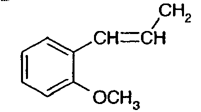 | O |
| A510 | CF₃ | C≡CCH₂ | 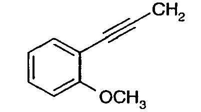 | O |
| A511 | CF₃ | C≡CCH₂ | 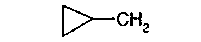 | O |
| A512 | CF₃ | C≡CCH₂ | 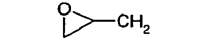 | O |
| A513 | CF₃ | C≡CCH₂ | 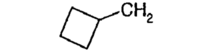 | O |
| A514 | CF₃ | C≡CCH₂ | 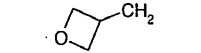 | O |
| A515 | CF₃ | C≡CCH₂ | 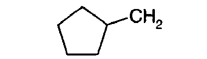 | O |
| A516 | CF₃ | C≡CCH₂ | 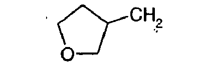 | O |
| A517 | CF₃ | C≡CCH₂ | 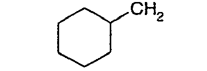 | O |
| A518 | CF₃ | C≡CCH₂ | 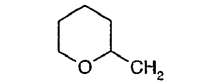 | O |
| A519 | CF₃ | C≡CCH₂ | 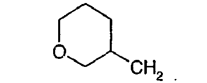 | O |
| A520 | CF₃ | C≡CCH₂ | 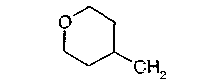 | O |
| A521 | CF₃ | C≡CCH₂ | 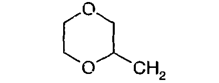 | O |
| A521a | CF₃ | C≡CCH₂ | 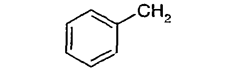 | O |
| A522 | CF₃ | C≡CCH₂ | 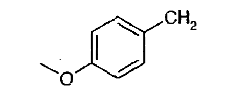 | O |
| A523 | CF₃ | C≡CCH₂ | 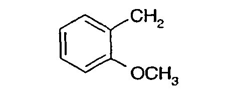 | O |
| A524 | CF₃ | C≡CCH₂ | 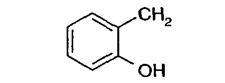 | O |
| A525 | CF₃ | C≡CCH₂ | 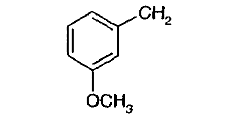 | O |
| A526 | CF₃ | C≡CCH₂ | 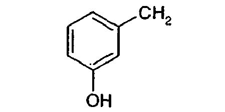 | O |
| A527 | CF₃ | C≡CCH₂ | 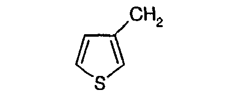 | O |
| A528 | CF₃ | C≡CCH₂ | 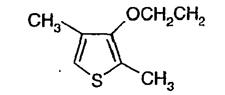 | O |
| A529 | CF₃ | C≡CCH₂ | 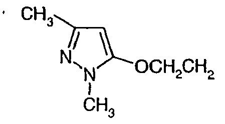 | O |
| A530 | CF₃ | C≡CCH₂ | 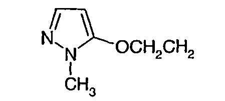 | O |
| A531 | CF₃ | C≡CCH₂ | 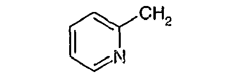 | O |
| A532 | CF₃ | C≡CCH₂ | 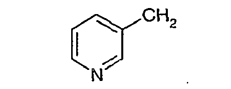 | O |
| A533 | CF₃ | C≡CCH₂ | 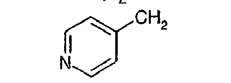 | O |
| A534 | CF₃ | C≡CCH₂ | 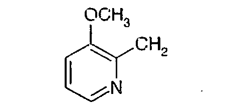 | O |
| R535 | CF₃ | C≡CCH₂ | 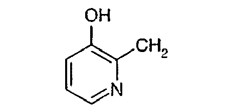 | O |
| A536 | CF₃ | C≡CCH₂ | 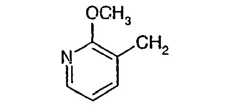 | O |
| A537 | CF₃ | C≡CCH₂ | 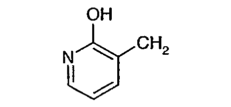 | O |
| A538 | CF₃ | C≡CCH₂ | 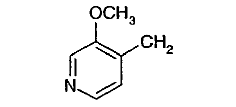 | O |
| A539 | CF₃ | C≡CCH₂ | 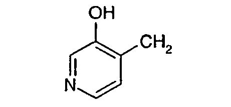 | O |
| A540 | CF₃ | C≡CCH₂ | 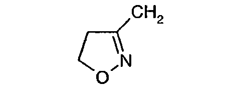 | O |
| A541 | CF₃ | C≡CCH₂ | 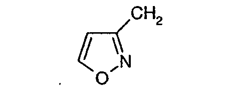 | O |
| A542 | CF₃ | C≡CCH₂ | 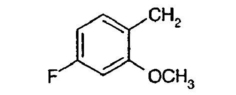 | O |
| A543 | CF₃ | C≡CCH₂ | 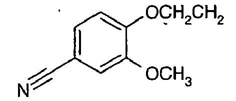 | O |
| A544 | CF₃ | C≡CCH₂ | 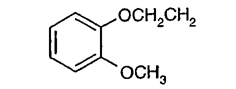 | O |
| A545 | CF₃ | C≡CCH₂ | 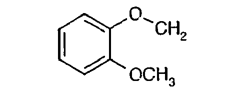 | O |
| A546 | CF₂Cl | CH₂ | CH₃ | O |
| A547 | CF₂Cl | CH₂ | CH₃CH₂ | O |
| A548 | CF₂Cl | CH₂ | (CH₃)₂CH | O |
| A549 | CF₂Cl | CH₂ | PhCH₂ | O |
| A550 | CF₂Cl | CH₂ | CH₃ | S |
| A551 | CF₂Cl | CH₂ | CH₃ | SO |
| A552 | CF₂Cl | CH₂ | CH₃ | SO₂ |
| A553 | CF₂Cl | CH₂ | CH₃CH₂CH₂ | O |
| A554. | CF₂Cl | CH₂ | CH₃OCH₂ | O |
| A555 | CF₂Cl | CH₂ | CH₃CH₂OCH₂ | O |
| A556 | CF₂Cl | CH₂ | CH₃OCH₂CH₂ | O |
| A557 | CF₂Cl | CH₂ | CH₃CH₂OCH₂CH₂ | O |
| A558 | CF₂Cl | CH₂ | CH₃OC(CH₃)₂CH₂ | O |
| A559 | CF₂Cl | CH₂ | CH₃OCH(CH₃)CH₂ | O |
| A560 | CF₂Cl | CH₂ | CH₃OCH₂CH(CH₃) | O |
| A561 | CF₂Cl | CH₂ | CH₃OCH₂C(CH₃)₂ | O |
| A562 | CF₂Cl | CH₂ | CH₃OCH(CH₃) | O |
| A563 | CF₂Cl | CH₂ | CH₃OC(CH₃)₂ | O |
| A564 | CF₂Cl | CH₂ | HC≡CCH₂ | O |
| A565 | CF₂Cl | CH₂ | H₂C=CHCH₂ | O |
| A566 | CF₂Cl | CH₂ | CH₃C≡CCH₂ | O |
| A567 | CF₂Cl | CH₂ | 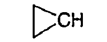 | O |
| A568 | CF₂Cl | CH₂ | 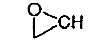 | O |
| A569 | CF₂Cl | CH₂ | 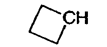 | O |
| A570 | CF₂Cl | CH₂ | 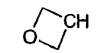 | O |
| A571 | CF₂Cl | CH₂ | 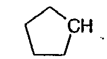 | O |
| A572 | CF₂Cl | CH₂ | 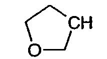 | O |
| A573 | CF₂Cl | CH₂ | 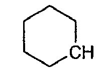 | O |
| A574 | CF₂Cl | CH₂ | 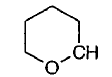 | O |
| A575 | CF₂Cl | CH₂ | 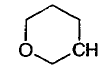 | O |
| A576 | CF₂Cl | CH₂ | 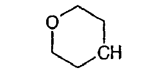 | 0 |
| A577 | CF₂Cl | CH₂ | 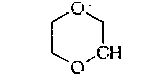 | 0 |
| A578 | CF₂Cl | CH₂ | 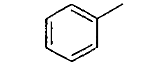 | 0 |
| A579 | CF₂Cl | CH₂ | 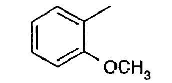 | 0 |
| A580 | CF₂Cl | CH₂ | 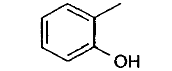 | 0 |
| A581 | CF₂Cl | CH₂ | 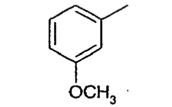 | 0 |
| A582 | CF₂Cl | CH₂ | 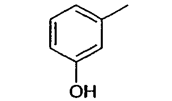 | 0 |
| A583 | CF₂Cl | CH₂ | 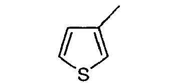 | 0 |
| A584 | CF₂Cl | CH₂ | 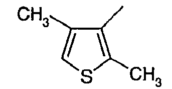 | 0 |
| A585 | CF₂Cl | CH₂ | 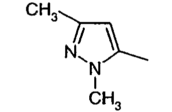 | 0 |
| A586 | CF₂Cl | CH₂ | 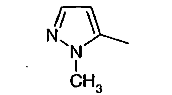 | 0 |
| A587 | CF₂Cl | CH₂ | 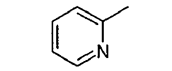 | 0 |
| A588 | CF₂Cl | CH₂ | 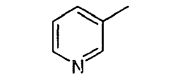 | 0 |
| A589 | CF₂Cl | CH₂ | 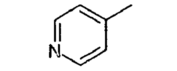 | 0 |
| A590 | CF₂Cl | CH₂ | 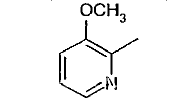 | O |
| A591 | CF₂Cl | CH₂ | 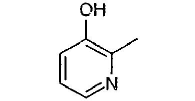 | O |
| A592 | CF₂Cl | CH₂ | 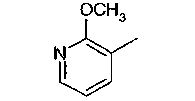 | O |
| A593 | CF₂Cl | CH₂ | 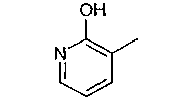 | O |
| A594 | CF₂Cl | CH₂ | 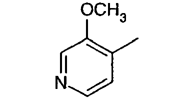 | O |
| A595 | CF₂Cl | CH₂ | 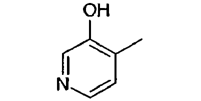 | O |
| A596 | CF₂Cl | CH₂ | 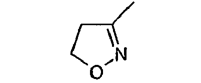 | O |
| A597 | CF₂Cl | CH₂ | 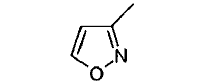 | O |
| A598 | CF₂Cl | CH₂ | 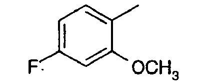 | O |
| A599 | CF₂Cl | CH₂ | 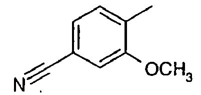 | O |
| A600 | CF₂Cl | CH₂ | 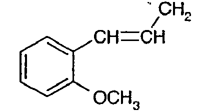 | O |
| A601 | CF₂Cl | CH₂ | 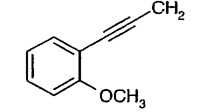 | O |
| A602 | CF₂Cl | CH₂ | 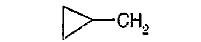 | O |
| A603 | CF₂Cl | CH₂ | 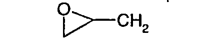 | O |
| A604 | CF₂Cl | CH₂ | 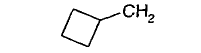 | O |
| A605 | CF₂Cl | CH₂ | 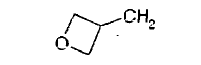 | O |
| A606 | CF₂Cl | CH₂ | 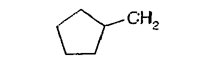 | O |
| A607 | CF₂Cl | CH₂ | 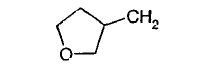 | O |
| A608 | CF₂Cl | CH₂ | 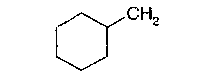 | O |
| A609 | CF₂Cl | CH₂ | 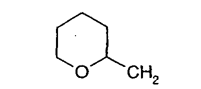 | O |
| A610 | CF₂Cl | CH₂ | 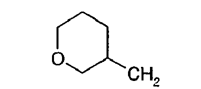 | O |
| A611 | CF₂Cl | CH₂ | 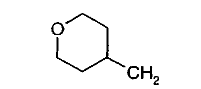 | O |
| A612 | CF₂Cl | CH₂ | 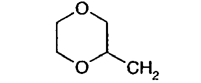 | O |
| A613 | CF₂Cl | CH₂ | 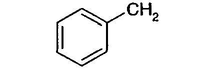 | O |
| A614 | CF₂Cl | CH₂ | 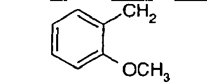 | O |
| A615 | CF₂Cl | CH₂ | 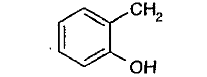 | O |
| A616 | CF₂Cl | CH₂ | 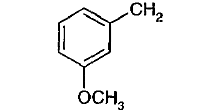 | O |
| A617 | CF₂Cl | CH₂ | 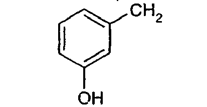 | O |
| A618 | CF₂Cl | CH₂ | 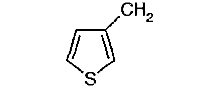 | O |
| A619 | CF₂Cl | CH₂ | 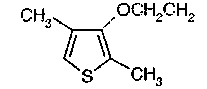 | O |
| A620 | CF₂Cl | CH₂ CH2 | 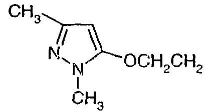 | O |
| A621 | CF₂Cl | CH₂ | 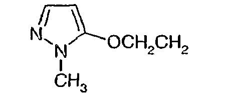 | O |
| A622 | CF₂Cl | CH₂ | 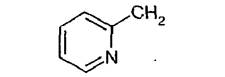 | O |
| A623 | CF₂Cl | CH₂ | 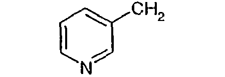 | O |
| A624 | CF₂Cl | CH₂ | 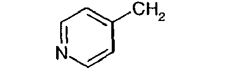 | O |
| A625 | CF₂Cl | CH₂ | 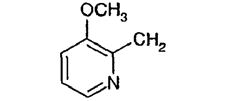 | O |
| A626 | CF₂Cl | CH₂ | 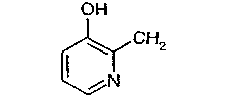 | O |
| A627 | CF₂Cl | CH₂ | 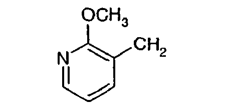 | O |
| A628 | CF₂Cl | CH₂ | 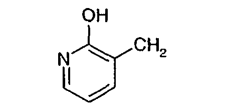 | O |
| A629 | CF₂Cl | CH₂ | 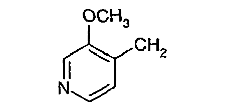 | O |
| A630 | CF₂Cl | CH₂ | 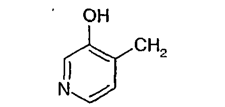 | O |
| A631 | CF₂Cl | CH₂ | 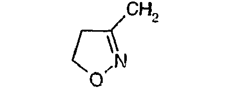 | O |
| A632 | CF₂Cl | CH₂ | 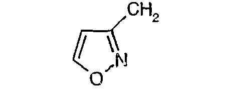 | O |
| A633 | CF₂Cl | CH₂ | 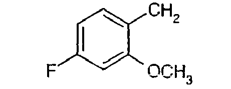 | O |
| A634 | CF₂Cl | CH₂ | 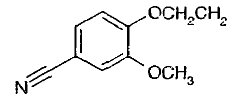 | O |
| A635 | CF₂Cl | CH₂ | 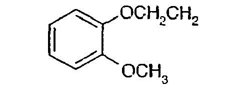 | O |
| A636 | CF₂Cl | CH₂ | 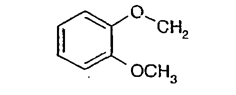 | O |
| A637 | CF₂Cl | CH₂ | CH₃ | O |
| A638 | CF₂Cl | CH₂ | CH₂CH₃ | O |
| A639 | CF₂Cl | CH₂ | (CH₃)₂CH | O |
| A640 | CF₂Cl | CH₂ | PhCH₂ | O |
| A641 | CF₂Cl | CH₂ | CH₃ | S |
| A642 | CF₂Cl | CH₂ | CH₃ | O |
| A643 | CF₂Cl | CH₂ | CH₃ | O |
| A644 | CF₂Cl | CH₂ | CH₃OCH₂ | O |
| A645 | CF₂Cl | CH₂ | CH₃CH₂OCH₂ | O |
| A646 | CF₂Cl | CH₂ | CH₃OCH₂CH₂ | O |
| A647 | CF₂Cl | CH₂ | CH₃CH₂OCH₂CH₂ | O |
| A648 | CF₂Cl | CH₂ | CH₃OC(CH₃)₂CH₂ | O |
| A649 | CF₂Cl | CH₂ | CH₃OCH(CH₃)CH₂ | O |
| A650 | CF₂Cl | CH₂ | CH₃OCH₂CH(CH₃) | O |
| A651 | CF₂Cl | CH₂ | CH₃OCH₂C(CH₃)₂ | O |
| A652 | CF₂Cl | CH₂ | CH₃OCH(CH₃) | O |
| A653 | CF₂Cl | CH₂ | CH₃OC(CH₃)₂ | O |
| A654 | CF₂Cl | CH₂ | HC≡CCH₂ | O |
| A655 | CF₂Cl | CH₂ | H₂C=CHCH₂ | O |
| A656 | CF₂Cl | CH₂ | CH₃C≡CCH₂ | O |
| A657 | CF₂Cl | CH₂ | 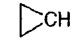 | O |
| A658 | CF₂Cl | CH₂ | 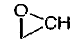 | O |
| A659 | CF₂Cl | CH₂ | 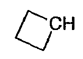 | O |
| A660 | CF₂Cl | CH₂ | 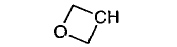 | O |
| A661 | CF₂Cl | CH₂ | 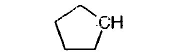 | O |
| A662 | CF₂Cl | CH₂ | 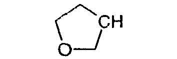 | O |
| A663 | CF₂Cl | CH₂ | 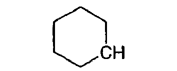 | O |
| A664 | CF₂Cl | CH₂ | 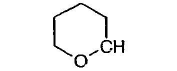 | O |
| A665 | CF₂Cl | CH₂ | 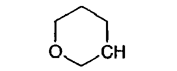 | O |
| A666 | CF₂Cl | CH₂ | 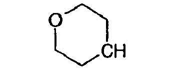 | O |
| A667 | CF₂Cl | CH₂ | 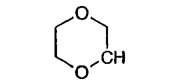 | O |
| A668 | CF₂Cl | CH₂ | 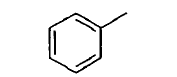 | O |
| A669 | CF₂Cl | CH₂ | 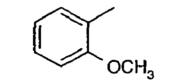 | O |
| A670 | CF₂Cl | CH₂ | 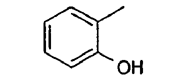 | O |
| A671 | CF₂Cl | CH₂ | 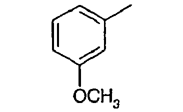 | O |
| A672 | CF₂Cl | CH₂ | 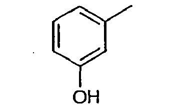 | O |
| A673 | CF₂Cl | CH₂ | 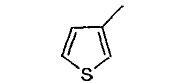 | O |
| A674 | CF₂Cl | CH₂ | 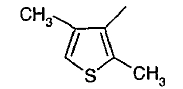 | O |
| A675 | CF₂Cl | CH₂ | 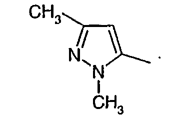 | O |
| A676 | CF₂Cl | CH₂ CH₂ | 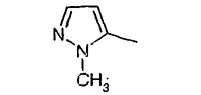 | O |
| A677 | CF₂Cl | CH₂ | 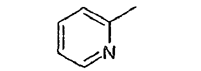 | O |
| A678 | CF₂Cl | CH₂ | 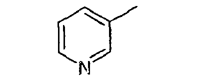 | O |
| A679 | CF₂Cl | CH₂ | 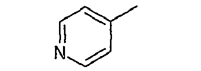 | O |
| A680 | CF₂Cl | CH₂ | 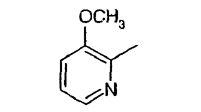 | O |
| A681 | CF₂Cl | CH₂ | 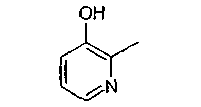 | O |
| A682 | CF₂Cl | CH₂ | 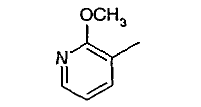 | S |
| A683 | CF₂Cl | CH₂ | 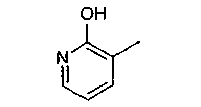 | SO |
| A684 | CF₂Cl | CH₂ | 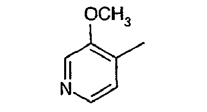 | SO₂ |
| A685 | CF₂Cl | CH₂ | 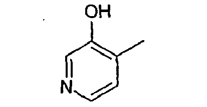 | O |
| A686 | CF₂Cl | CH₂ | 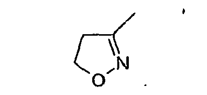 | O |
| A687 | CF₂Cl | CH₂ | 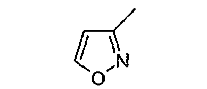 | O |
| A688 | CF₂Cl | GH₂ | 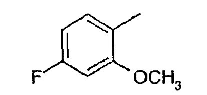 | O |
| A689 | CF₂Cl | CH₂ | 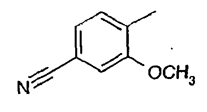 | O |
| A690 | CF₂Cl | CH₂ | 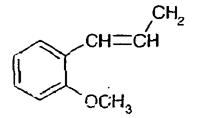 | O |
| A691 | CF₂Cl | CH₂ | 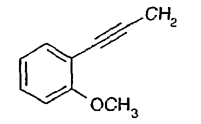 | O |
| A692 | CF₂Cl | CH₂ | 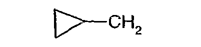 | O |
| A693 | CF₂Cl | CH₂ | 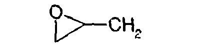 | O |
| A694 | CF₂Cl | CH₂ | 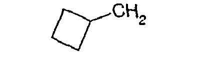 | O |
| A695 | CF₂Cl | CH₂ | 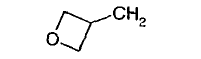 | O |
| A696 | CF₂Cl | CH₂ | 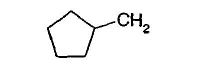 | O |
| A697 | CF₂Cl | CH₂ | 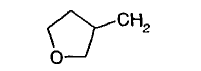 | O |
| A698 | CF₂Cl | CH₂ | 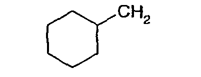 | O |
| A699 | CF₂Cl | CH₂ | 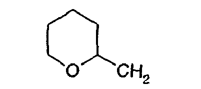 | O |
| A700 | CF₂Cl | CH₂ | 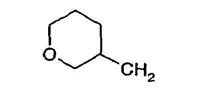 | O |
| A701 | CF₂Cl | CH₂ | 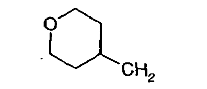 | O |
| A702 | CF₂Cl | CH₂ | 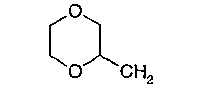 | O |
| A703 | CF₂Cl | CH₂ | 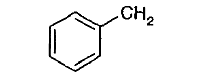 | O |
| A704 | CF₂Cl | CH₂ | 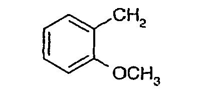 | O |
| A705 | CF₂Cl | CH₂ | 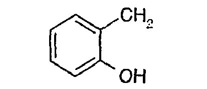 | O |
| A706 | CF₂Cl | CH₂ CH₂ | 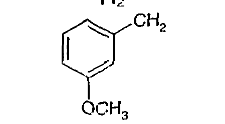 | O |
| A707 | CF₂Cl | CH₂ | 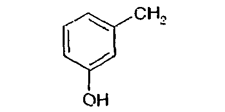 | O |
| A708 | CF₂Cl | CH₂ | 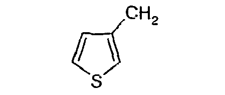 | O |
| A709 | CF₂Cl | CH₂ | 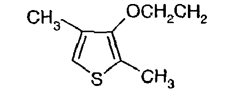 | O |
| A710 | CF₂Cl | CH₂ | 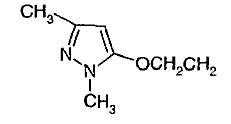 | O |
| A711 | CF₂Cl | CH₂ | 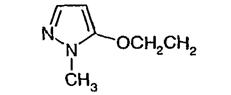 | O |
| A712 | CF₂Cl | CH₂ | 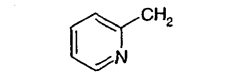 | O |
| A713 | CF₂Cl | CH₂ | 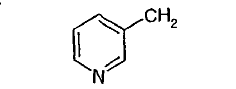 | O |
| A714 | CF₂Cl | CH₂ | 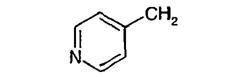 | O |
| A715 | CF₂Cl | CH₂ | 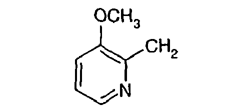 | O |
| A716 | CF₂Cl | CH₂ | 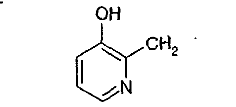 | O |
| A717 | CF₂Cl | CH₂ | 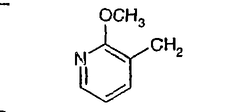 | O |
| A718 | CF₂Cl | CH₂ | 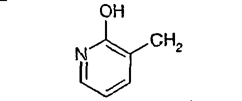 | O |
| A719 | CF₂Cl | CH₂ | 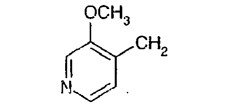 | O |
| A720 | CF₂Cl | CH₂ | 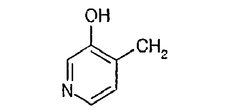 | O |
| A721 | CF₂Cl | CH₂ | 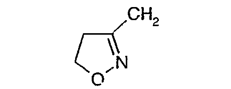 | O |
| A722 | CF₂Cl | CH₂ | 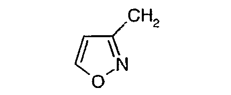 | O |
| A723 | CF₂Cl | CH₂ | 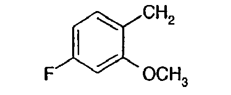 | O |
| A724 | CF₂Cl | CH₂ | 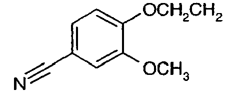 | O |
| A725 | CF₂Cl | CH₂ | 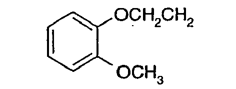 | O |
| A726 | CF₂Cl | CH₂ | 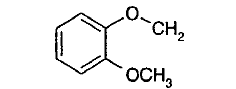 | O |
| A727 | CF₂Cl | CH₂ | CH₃ | O |
| A728 | CF₂Cl | CH₂ | CH₂CH₃ | O |
| A729 | CF₂Cl | CH₂ | (CH₃)₂CH | O |
| A730 | CF₂Cl | CH₂ | PhCH₂ | O |
| A731 | CF₂Cl | CH₂ | CH₃ | S |
| A732 | CF₂Cl | CH₂ | CH₃ | SO |
| A733 | CF₂Cl | CH₂ | CH₃ | SO₂ |
| A734 | CF₂Cl | CH₂ | CH₃OCH₂ | O |
| A735 | CF₂Cl | CH₂ | CH₃CH₂OCH₂ | O |
| A736 | CF₂Cl | CH₂ | CH₃OCH₂CH₂ | O |
| A737 | CF₂Cl | CH₂ | CH₃CH₂OCH₂CH₂ | O |
| A738 | CF₂Cl | CH₂ | CH₃OC(CH₃)₂CH₂ | O |
| A739 | CF₂Cl | CH₂ | CH₃OCH(CH₃)CH₂ | O |
| A740 | CF₂Cl | CH₂ | CH₃OCH₂CH(CH₃) | O |
| A741 | CF₂Cl | CH₂ | CH₃OCH₂C(CH₃)₂ | O |
| A742 | CF₂Cl | CH₂ | CH₃OCH(CH₃) | O |
| A743 | CF₂Cl | CH₂ | CH₃OC(CH₃)₂ | O |
| A744 | CF₂Cl | CH₂ | HC≡CCH₂ | O |
| A745 | CF₂Cl | CH₂ | H₂C=CHCH₂ | O |
| A746 | CF₂Cl | CH₂ | CH₃C≡CCH₂ | O |
| A747 | CF₂Cl | CH₂ | 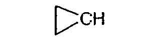 | O |
| A748 | CF₂Cl | CH₂ | 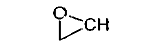 | O |
| 49 | CF₂Cl | CH₂ | 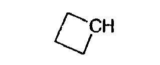 | O |
| A750 | CF₂Cl | CH₂ | 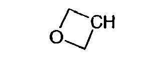 | O |
| A751 | CF₂Cl | CH₂ | 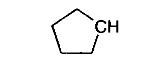 | O |
| A752 | CF₂Cl | CH₂ | 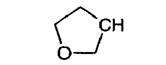 | O |
| A753 | CF₂Cl | CH₂ | 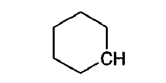 | O |
| A754 | CF₂Cl | CH₂ | 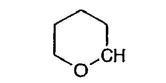 | O |
| A755 | CF₂Cl | CH₂ | 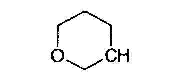 | O |
| A756 | CF₂Cl | CH₂ | 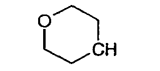 | O |
| A757 | CF₂Cl | CH₂ | 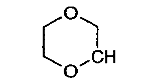 | O |
| A758 | CF₂Cl | CH₂ | 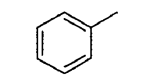 | O |
| A759 | CF₂Cl | CH₂ | 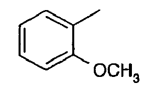 | O |
| A760 | CF₂Cl | CH₂ | 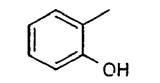 | O |
| A761 | CF₂Cl | CH₂ | 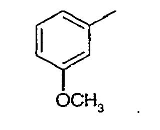 | O |
| A762 | CF₂Cl | CH₂ | 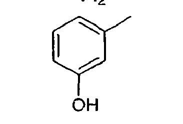 | O |
| A763 | CF₂Cl | CH₂ | 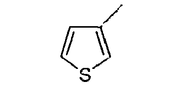 | O |
| A764 | CF₂Cl | CH₂ | 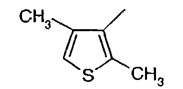 | O |
| A765 | CF₂Cl | CH₂ | 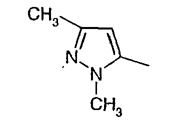 | O |
| A766 | CF₂Cl | CH₂ | 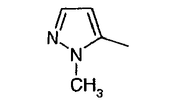 | O |
| A767 | CF₂Cl | CH₂ | 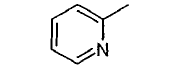 | O |
| A768 | CF₂Cl | CH₂ | 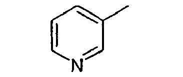 | O |
| A769 | CF₂Cl | CH₂ | 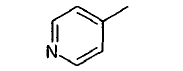 | O |
| A770 | CF₂Cl | CH₂ | 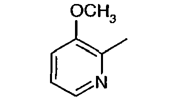 | O |
| A771 | CF₂Cl | CH₂ | 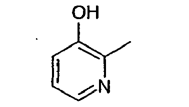 | O |
| A772 | CF₂Cl | CH₂ | 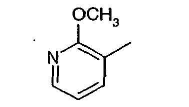 | O |
| A773 | CF₂Cl | CH₂ | 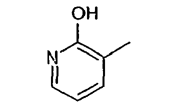 | O |
| A774 | CF₂Cl | CH₂ | 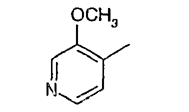 | O |
| A775 | CF₂Cl | CH₂ | 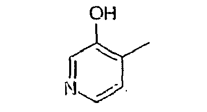 | O |
| A776 | CF₂Cl | CH₂ | 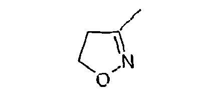 | O |
| A777 | CF₂Cl | CH₂ | 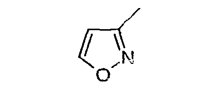 | O |
| A778 | CF₂Cl | CH₂ | 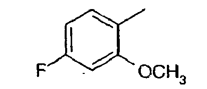 | O |
| A779 | CF₂Cl | CH₂ | 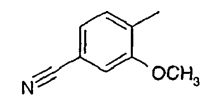 | O |
| A780 | CF₂Cl | CH₂ | 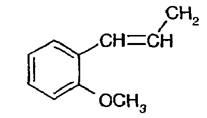 | O |
| A781 | CF₂Cl | CH₂ | 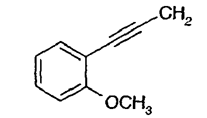 | O |
| A782 | CF₂Cl | CH₂ | 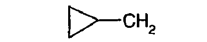 | O |
| A783 | CF₂Cl | CH₂ | 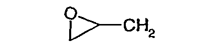 | O |
| A784 | CF₂Cl | CH₂ | 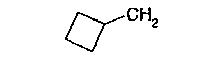 | O |
| A785 | CF₂Cl | CH₂ | 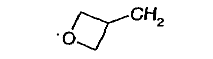 | O |
| A786 | CF₂Cl | CH₂ | 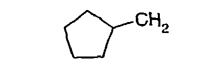 | O |
| A787 | CF₂Cl | CH₂ | 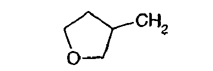 | O |
| A788 | CF₂Cl | CH₂ | 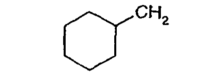 | O |
| A789 | CF₂Cl | CH₂ | 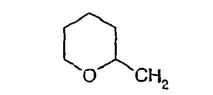 | O |
| A790 | CF₂Cl | CH₂ | 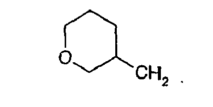 | O |
| A791 | CF₂Cl | CH₂ | 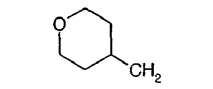 | O |
| A792 | CF₂Cl | CH₂ | 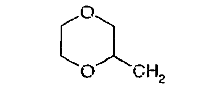 | O |
| A793 | CF₂Cl | CH₂ | 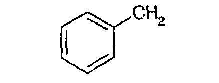 | O |
| A794 | CF₂Cl | CH₂ | 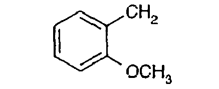 | O |
| A795 | CF₂Cl | CH₂ | 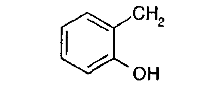 | O |
| A796 | CF₂Cl | CH₂ | 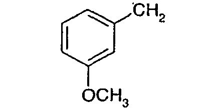 | O |
| A797 | CF₂Cl | CH₂ | 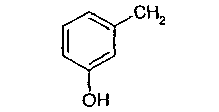 | O |
| A798 | CF₂Cl | CH₂ | 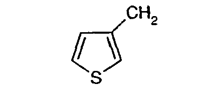 | O |
| A799 | CF₂Cl | CH₂ | 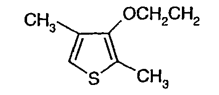 | O |
| A800 | CF₂Cl | CH₂ | 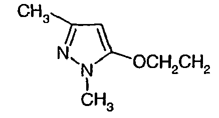 | O |
| A801 | CF₂Cl | CH₂ | 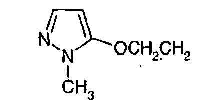 | O |
| A802 | CF₂Cl | CH₂ | 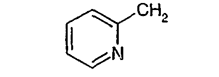 | O |
| A803 | CF₂Cl | CH₂ | 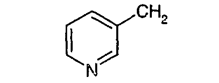 | O |
| A804 | CF₂Cl | CH₂ | 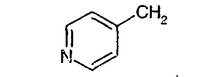 | O |
| A805 | CF₂Cl | CH₂ CH₂ | 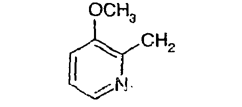 | O |
| A806 | CF₂Cl | CH₂ | 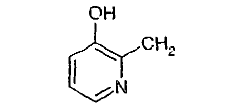 | O |
| A807 | CF₂Cl | CH₂ | 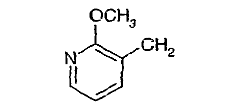 | O |
| A808 | CF₂Cl | CH₂ | 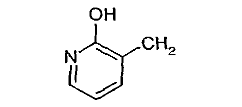 | O |
| A809 | CF₂Cl | CH₂ | 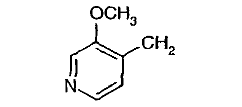 | O |
| A810 | CF₂Cl | CH₂ | 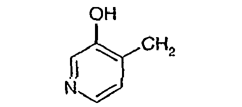 | O |
| A811 | CF₂Cl | CH₂ | 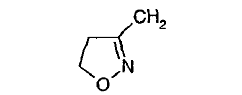 | O |
| A812 | CF₂Cl | CH₂ | 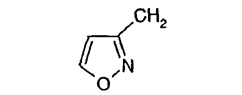 | O |
| A813 | CF₂Cl | CH₂ | 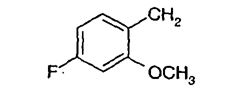 | O |
| A814 | CF₂Cl | CH₂ | 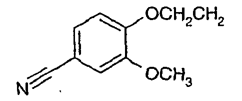 | O |
| A815 | CF₂Cl | CH₂ | 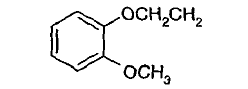 | O |
| A816 | CF₂Cl | CH₂ | 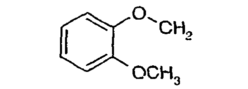 | O |
| A817 | CF₃ | CH₂ | CH₃SCH₂CH₂ | O |
| A818 | CF₃ | CH₂ | CH₃SOCH₂CH₂ | O |
| A819 | CF₃ | CH₂ | CH₃SO₂CH₂CH₂ | O |
| A820 | CF₃ | CH₂ | CH₃OCH₂CH₂ | O |
| A821 | CF₃ | CH₂ | CH₃OCH₂CH₂ | O |
| A822 | CF₃ | CH₂ | CH₃OCH₂CH₂ | O |
| A823 | CF₃ | CH₂ | CH₃OCH₂CH₂ | O |
| A824 | CF₃ | CH₂ | CH₃OCH₂CH₂ | O |
| A825 | CF₃ | CH₂ | CH₃OCH₂CH₂ | S |
| A826 | CF₃ | CH₂ | CH₃OCH₂CH₂ | SO |
| A827 | CF₃ | CH₂ | CH₃OCH₂CH₂ | SO₂ |
| A828 | CF₃ | CH₂ | CH₃SO₂CH₂CH₂ | O |
| A829 | CF₃ | CH₂ | 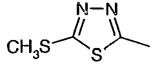 | S |
| A830 | CF₃ | CH₂ | 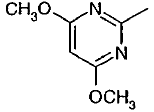 | S |
| A831 | CF₃ | CH₂ | 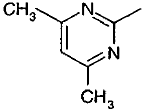 | S |
| A832 | CF₃ | CH₂ | 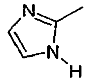 | S |
| A833 | CF₃ | CH₂ | CH₃C(O) | O |
| A834 | CF₃ | CH₂ | CF₃CH₂ | O |
| A835 | CF₃ | CH₂ | CH₃OCH₂CH₂OCH₂CH₂ | O |
| A836 | CF₃ | CH₂ | HC≡CCH₂CH₂ | O |
| A837 | CF₃ | CH₂ | 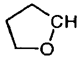 | O |
| A838 | CF₃ | CH₂ | CH₃CH₂C(OCH₃)HOCH₂CH₂ | O |
| A839 | CF₃ | CH₂ | (CH₃)₃CC(O) | O |
| A840 | CF₃ | CH₂ | CH₂=CHCH₂OCH₂CH₂ | O |
| A841 | CF₃ | CH₂ | CH₃CH₂CH₂OCH₂CH₂ | O |
| A842 | CF₃ | CH₂ | 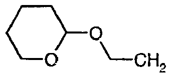 | O |
| A843 | CF₃ | CH₂ | n-heptyl-C(O) | O |
| A844 | CF₃ | CH₂ | phenyl-C(O) | O |
| A845 | CF₃ | CH₂ | CF₃CH₂OCH₂CH₂ | O |
| A846 | CF₃ | CH₂ | CH₃OCH₂CH₂CH₂ | O |
| A847 | CF₃ | CH₂ | HOCH₂CH₂CH₂ | O |
| A848 | CF₃ | CH₂ | 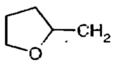 | O |
| A849 | CF₃ | CH₂ | N=CCH₂CH₂ | O |
| A850 | CF₃ | CH₂ | ClCH₂CH₂ | O |
| A851 | CF₃ | CH₂ | 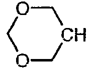 | O |
| A852 | CF₃ | CH₂ | 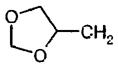 | O |
| A853 | CF₃ | CH₂ | CH₃OCH₂C(Br)HCH₂ | O |
| A854 | CF₃ | CH₂ | 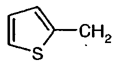 | O |
| A855 | CF₃ | CH₂ | 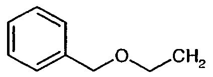 | O |
| A856 | CF₃ | CH₂ | HOCH₂CH₂ | O |
| A857 | CF₃ | CH₂ | 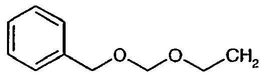 | O |
| A858 | CF₃ | CH₂ | CH₃(OCH₂CH₂)₃ | O |
| A859 | CF₃ | CH₂ | CH₃CH₂OC(CH₃)HOCH₂CH₂ | O |
| A860 | CF₃ | CH₂ | n-heptyl-C(O)OCH₂CH₂ | O |
| A861 | CF₃ | CH₂ | CH₃C(O)OCH₂CH₂ | O |
| A862 | CF₃ | CH₂ | CH₃SO₂OCH₂CH₂ | O |
| A863 | CF₃ | CH₂ | 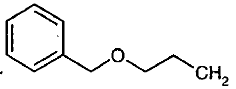 | O |
| A864 | CF₃ | CH₂ | CH₃ | -N(CH₃)SO₂- |
| A865 | CF₃ | CH₂ | HOCH₂C(OH)HCH₂ | O |
| A866 | CF₃ | CH₂ | phenyl-C(O)OCH₂CH₂ | O |
| A867 | CF₃ | CH₂ | tert-butyl-C(O)OCH₂CH₂ | O |
| A868 | CF₃ | CH₂ | CH₃OC(O)CH₂ | O |
| A869 | CF₃ | CH₂CH₂CH₂ | CH₃ | O |
| A870 | CF₃ | CH₂CH₂CH₂ | CH₂CH₃ | O |
| A871 | CF₂Cl | CH₂CH₂CH₂ | CH₃ | O |
| A872 | CF₂Cl | CH₂CH₂CH₂ | CH₂CH₃ | O |

The process according to the invention can be used especially advantageously for the preparation of the following compounds of Table 2:

In Table 2 which follows, the attachment position of the individual structures of the heterocycles of the group R₂ to the substituent R₁-X₁-, or to the C₁-C₄alkylene, C₂-C₄alkenyl-C₁-C₄alkylene, C₂-C₄alkynyl-C₁-C₄alkylene, -N(R₁₂)-C₁-C₄alkylene, -SO-C₁-C₄alkylene or -SO₂-C₁-C₄alkylene groups which connect the heterocycle of R₂ to the basic structure of formula I, is that nitrogen atom which is located at the same geometric position as is indicated in the Example below.

For example, the attachment position of the group 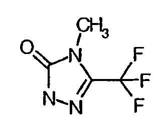 in the case of compound A 1.001 is the position indicated by an arrow: 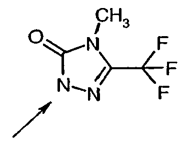 The free valencies in those structures represent terminal CH₃ groups, as in the case of, for example, the structure 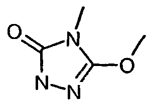 which may also be depicted as follows:

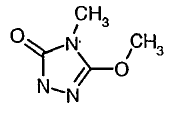

**Table 2: Compounds of formula Ia wherein R is either methyl or ethyl:**

| | | | |
|---|---|---|---|
| 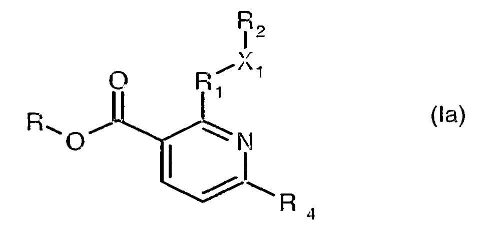 | | | |

| Comp. no. | R₄ | -R₁- | -X₁-R₂ |
|---|---|---|---|
| A1.001 | CF₂Cl | CH₂ | 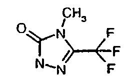 |
| A1.002 | CF₂H | CH₂ | 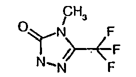 |
| A1.003 | CF₃ | CH₂ | 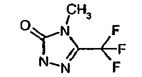 |
| A1.004 | CF₃ | CH₂OCH₂CH₂ | 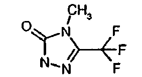 |
| A1.005 | CF₂Cl | CH₂OCH₂CH₂ | 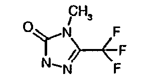 |
| A1.006 | CHF₂ | CH₂OCH₂CH₂ | 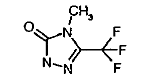 |
| A1.007 | CF₃ | CH₂ | 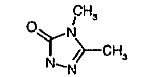 |
| A1.008 | CF₂Cl | CH₂ | 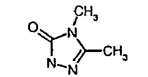 |
| A1.009 | CHF₂ | CH₂ | 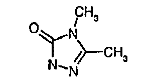 |
| A1.010 | CF₃ | CH₂OCH₂CH₂ | 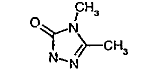 |
| A1.011 | CF₂Cl | CH₂OCH₂CH₂ | 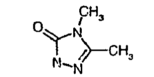 |
| A1.012 | CHF₂ | CH₂OCH₂CH₂ | 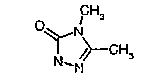 |
| A1.013 | CF₃ | CH₂ | 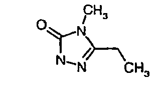 |
| A1.014 | CF₂Cl | CH₂ | 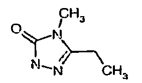 |
| A1.015 | CHF₂ | CH₂ | 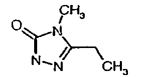 |
| A1.016 | CF₃ | CH₂OCH₂CH₂ | 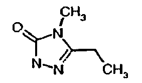 |
| A1.017 | CF₂Cl | CH₂OCH₂CH₂ | 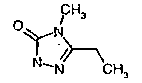 |
| A1.018 | CHF₂ | CH₂OCH₂CH₂ | 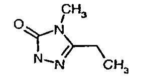 |
| A1.019 | CF₃ | CH₂ | 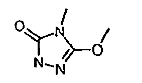 |
| A1.020 | CF₂Cl | CH₂ | 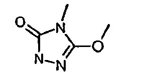 |
| A1.021 | CHF₂ | CH₂ | 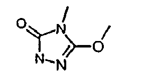 |
| A1.022 | CF₃ | CH₂OCH₂CH₂ | 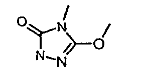 |
| A1.023 | CF₂Cl | CH₂OCH₂CH₂ | 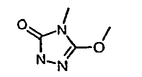 |
| A1.024 | CHF₂ | CH₂OCH₂CH₂ | 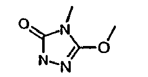 |
| A1.025 | CF₃ | CH₂ | 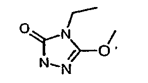 |
| A1.026 | CF₂Cl | CH₂ | 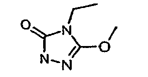 |
| A1.027 | CHF₂ | CH₂ | 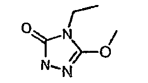 |
| A1.028 | CF₃ | CH₂OCH₂CH₂ | 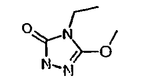 |
| A1.029 | CF₂Cl | CH₂OCH₂CH₂ | 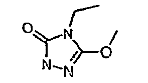 |
| A1.030 | CHF₂ | CH₂OCH₂CH₂ | 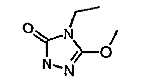 |
| A1.031 | CF₃ | CH₂ | 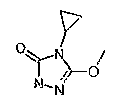 |
| A1.032 | CF₂Cl | CH₂ | 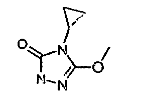 |
| A1.033 | CHF₂ | CH₂ | 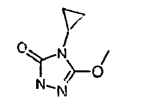 |
| A1.034 | CF₃ | CH₂OCH₂CH₂ | 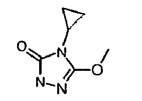 |
| A1.035 | CF₂Cl | CH₂OCH₂CH₂ | 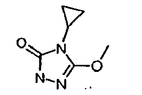 |
| A1.036 | CHF₂ | CH₂OCH₂CH₂ | 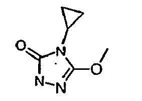 |
| A1.037 | CF₃ | CH₂ | 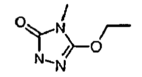 |
| A1.038 | CF₂Cl | CH₂ | 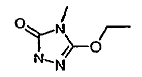 |
| A1.039 | CHF₂ | CH₂ | 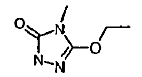 |
| A1.040 | CF₃ | CH₂OCH₂CH₂ | 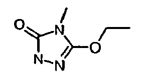 |
| A1.041 | CF₂Cl | CH₂OCH₂CH₂ | 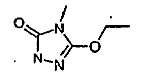 |
| A1.042 | CHF₂ | CH₂OCH₂CH₂ | 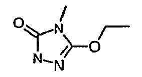 |
| A1.043 | CF₃ | CH₂ | 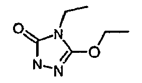 |
| A1.044 | CF₂Cl | CH₂ | 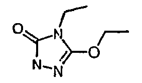 |
| A1.045 | CHF₂ | CH₂ | 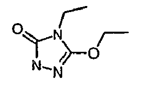 |
| A1.046 | CF₃ | CH₂OCH₂CH₂ | 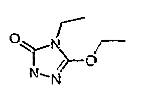 |
| A1.047 | CF₂Cl | CH₂OCH₂CH₂ | 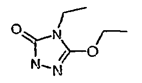 |
| A1.048 | CHF₂ | CH₂OCH₂CH₂ | 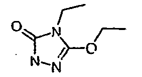 |
| A1.049 | CF₃ | CH₂ | 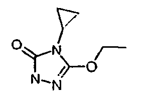 |
| A1.050 | CF₂Cl | CH₂ | 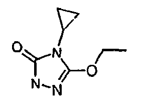 |
| A1.051 | CHF₂ | CH₂ | 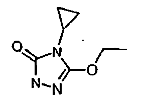 |
| A1.052 | CF₃ | CH₂OCH₂CH₂ | 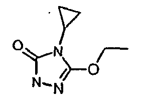 |
| A1.053 | CF₂Cl | CH₂OCH₂CH₂ | 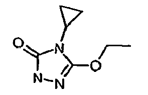 |
| A1.054 | CHF₂ | CH₂OCH₂CH₂ | 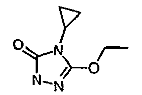 |
| A1.055 | CF₃ | CH₂ | 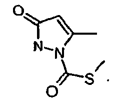 |
| A1.056 | CF₂Cl | CH₂ | 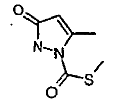 |
| A1.057 | CHF₂ | CH₂ | 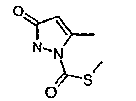 |
| A1.058 | CF₃ | CH₂OCH₂CH₂ | 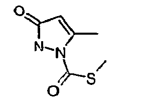 |
| A1.050 | CF₂Cl | CH₂OCH₂CH₂ | 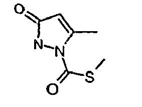 |
| A1.060 | CHF₂ | CH₂OCH₂CH₂ | 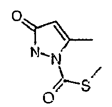 |
| A1.061 | CF₃ | CH₂ | 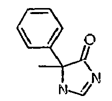 |
| A1.062 | CF₂Cl | CH₂ | 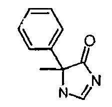 |
| A1.063 | CHF₂ | CH₂ | 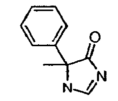 |
| A1.064 | CF₃ | CH₂OCH₂CH₂ | 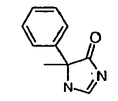 |
| A1.065 | CF₂Cl | CH₂OCH₂CH₂ | 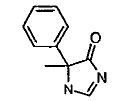 |
| A1.066 | CHF₂ | CH₂OCH₂CH₂ | 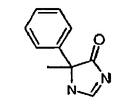 |
| A1.067 | CF₃ | CH₂ | 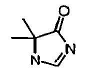 |
| A1.068 | CF₂Cl | CH₂ | 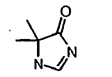 |
| A1.069 | CHF₂ | CH₂ | 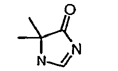 |
| A1.070 | CF₃ | CH₂OCH₂CH₂ | 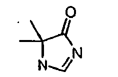 |
| A1.071 | CF₂Cl | CH₂OCH₂CH₂ | 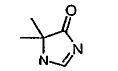 |
| A1.072 | CHF₂ | CH₂OCH₂CH₂ | 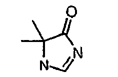 |
| A1.073 | CF₃ | CH₂ | 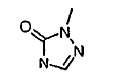 |
| A1.074 | CF₂Cl | CH₂ | 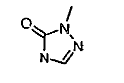 |
| A1.075 | CHF₂ | CH₂ | 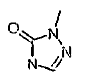 |
| A1.076 | CF₃ | CH₂OCH₂CH₂ | 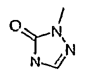 |
| A1.077 | CF₂Cl | CH₂OCH₂CH₂ | 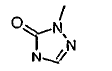 |
| A1.078 | CHF₂ | CH₂OCH₂CH₂ | 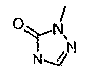 |
| A1.079 | CF₃ | CH₂ | 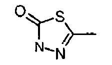 |
| A1.080 | CF₂Cl | CH₂ | 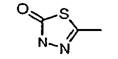 |
| A1.081 | CHF₂ | CH₂ | 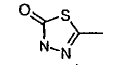 |
| A1.082 | CF₃ | CH₂OCH₂CH₂ | 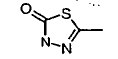 |
| A1.083 | CF₂Cl | CH₂OCH₂CH₂ | 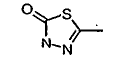 |
| A1.084 | CHF₂ | CH₂OCH₂CH₂ | 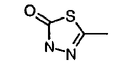 |
| A1.085 | CF₃ | CH₂ | 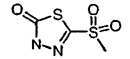 |
| A1.086 | CF₂Cl | CH₂ | 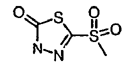 |
| A1.087 | CHF₂ | CH₂ | 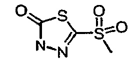 |
| A1.088 | CF₃ | CH₂OCH₂CH₂ | 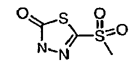 |
| A1.089 | CF₂Cl | CH₂OCH₂CH₂ | 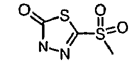 |
| A1.090 | CHF₂ | CH₂OCH₂CH₂ | 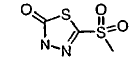 |
| A1.091 | CF₃ | CH₂ | 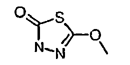 |
| A1.092 | CF₂Cl | CH₂ | 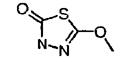 |
| A1.093 | CHF₂ | CH₂ | 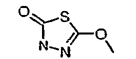 |
| A1.094 | CF₃ | CH₂OCH₂CH₂ | 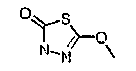 |
| A1.095 | CF₂Cl | CH₂OCH₂CH₂ | 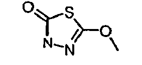 |
| A1.096 | CHF₂ | CH₂OCH₂CH₂ | 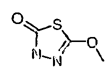 |
| A1.097 | CF₃ | CH₂ | 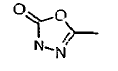 |
| A1.098 | CF₂Cl | CH₂ | 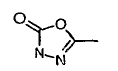 |
| A1.099 | CHF₂ | CH₂ | 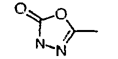 |
| A1.100 | CF₃ | CH₂OCH₂CH₂ | 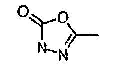 |
| A1.101 | CF₂Cl | CH₂OCH₂CH₂ | 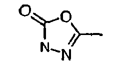 |
| A1.102 | CHF₂ | CH₂OCH₂CH₂ | 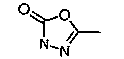 |
| A1.103 | CF₃ | CH₂ | 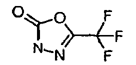 |
| A1.104 | CF₂Cl | CH₂ | 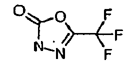 |
| A1.105 | CHF₂ | CH₂ | 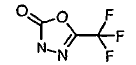 |
| A1.106 | CF₃ | CH₂OCH₂CH₂ | 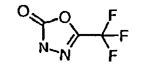 |
| A1.107 | CF₂Cl | CH₂OCH₂CH₂ | 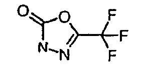 |
| A1.108 | CHF₂ | CH₂OCH₂CH₂ | 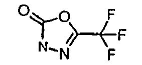 |
| A1.109 | CF₃ | CH₂ | 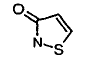 |
| A1.110 | CF₂Cl | CH₂ | 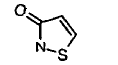 |
| A1.111 | CHF₂ | CH₂ | 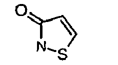 |
| A1.112 | CF₃ | CH₂OCH₂CH₂ | 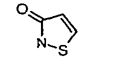 |
| A1.113 | CF₂Cl | CH₂OCH₂CH₂ | 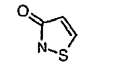 |
| A1.114 | CHF₂ | CH₂OCH₂CH₂ . | 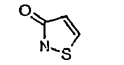 |
| A1.115 | CF₃ | CH₂ | 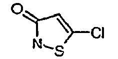 |
| A1.116 | CF₂Cl | CH₂ | 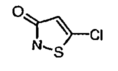 |
| A1.117 | CHF₂ | CH₂ | 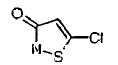 |
| A1.118 | CF₃ | CH₂OCH₂CH₂ | 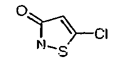 |
| A1.119 | CF₂Cl | CH₂OCH₂CH₂ | 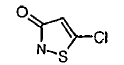 |
| A1.120 | CHF₂ | CH₂OCH₂CH₂ | 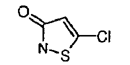 |
| A1.121 | CF₃ | CH₂ | 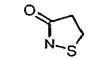 |
| A1.122 | CF₂Cl | CH₂ | 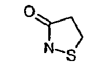 |
| A1.123 | CHF₂ | CH₂ | 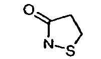 |
| A1.124 | CF₃ | CH₂OCH₂CH₂ | 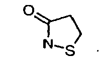 |
| A1.125 | CF₂Cl | CH₂OCH₂CH₂ | 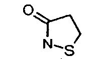 |
| A1.126 | CHF₂ | CH₂OCH₂CH₂ | 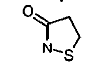 |
| A1.127 | CF₃ | CH₂ | 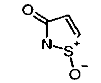 |
| A1.128 | CF₂Cl | CH₂ | 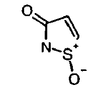 |
| A1.129 | CHF₂ | CH₂ | 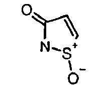 |
| A1.130 | CF₃ | CH₂OCH₂CH₂ | 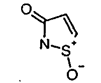 |
| A1.131 | CF₂Cl | CH₂OCH₂CH₂ | 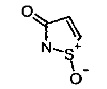 |
| A1.132 | CHF₂ | CH₂OCH₂CH₂ | 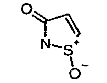 |
| A1.133 | CF₃ | CH₂ | 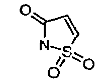 |
| A1.134 | CF₂Cl | CH₂ | 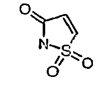 |
| A1.135 | CHF₂ | CH₂ | 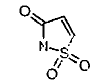 |
| A1.136 | CF₃ | CH₂OCH₂CH₂ | 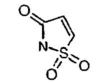 |
| A1.137 | CF₂Cl | CH₂OCH₂CH₂ | 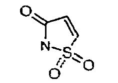 |
| A1.138 | CHF₂ | CH₂OCH₂CH₂ | 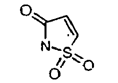 |
| A1.139 | CF₃ | CH₂ | 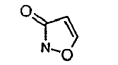 |
| A1.140 | CF₂Cl | CH₂ | 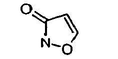 |
| A1.141 | CHF₂ | CH₂ | 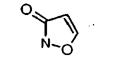 |
| A1.142 | CF₃ | CH₂OCH₂CH₂ | 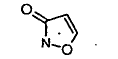 |
| A1.143 | CF₂Cl | CH₂OCH₂CH₂ | 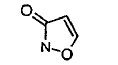 |
| A1.144 | CHF₂ | CH₂OCH₂CH₂ | 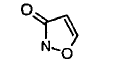 |
| A1.145 | CF₃ | CH₂ | 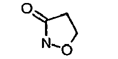 |
| A1.146 | CF₂Cl | CH₂ | 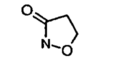 |
| A1.147 | CHF₂ | CH₂ | 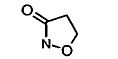 |
| A1.148 | CF₃ | CH₂OCH₂CH₂ | 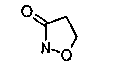 |
| A1.149 | CF₂Cl | CH₂OCH₂CH₂ | 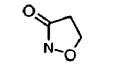 |
| A1.150 | CHF₂ | CH₂OCH₂CH₂ | 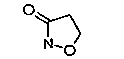 |
| A1.151 | CF₃ | CH₂ | 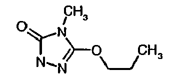 |
| A1.152 | CF₂Cl | CH₂ | 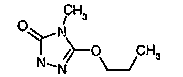 |
| A1.153 | CHF₂ | CH₂ | 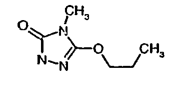 |
| A1.154 | CF₃ | CH₂OCH₂CH₂ | 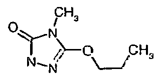 |
| A1.155 | CF₂Cl | CH₂OCH₂CH₂ | 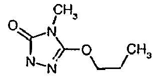 |
| A1.156 | CHF₂ | CH₂OCH₂CH₂ | 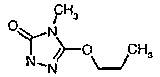 |
| A1.157 | CF₃ | CH₂ | 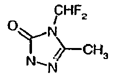 |
| A1.158 | CF₂Cl | CH₂ | 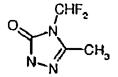 |
| A1.159 | CHF₂ | CH₂ | 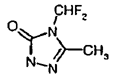 |
| A1.160 | CF₃ | CH₂OCH₂CH₂ | 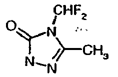 |
| A1.161 | CF₂Cl | CH₂OCH₂CH₂ | 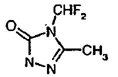 |
| A1.162 | CHF₂ | CH₂OCH₂CH₂ | 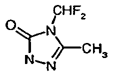 |
| A1.163 | CF₃ | CH₂ | 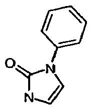 |
| A1.164 | CF₂Cl | CH₂ | 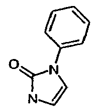 |
| A1.165 | CHF₂ | CH₂ | 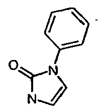 |
| A1.166 | CF₃ | CH₂OCH₂CH₂ | 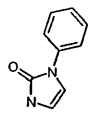 |
| A1.167 | CF₂Cl | CH₂OCH₂CH₂ | 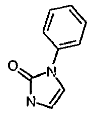 |
| A1.168 | CHF₂ | CH₂OCH₂CH₂ | 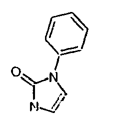 |
| A1.169 | CF₃ | CH₂ | 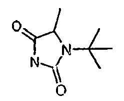 |
| A1.170 | CF₂Cl | CH₂ | 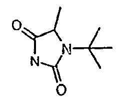 |
| A1.171 | CHF₂ | CH₂ | 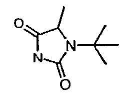 |
| A1.172 | CF₃ | CH₂OCH₂CH₂ | 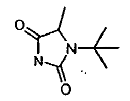 |
| A1.173 | CF₂Cl | CH₂OCH₂CH₂ | 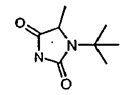 |
| A1.174 | CHF₂ | CH₂OCH₂CH₂ | 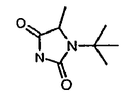 |
| A1.175 | CF₃ | CH₂ | 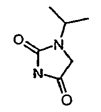 |
| A1.176 | CF₂Cl | CH₂ | 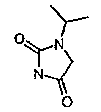 |
| A1.177 | CHF₂ | CH₂ | 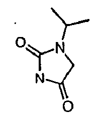 |
| A1.178 | CF₃ | CH₂OCH₂CH₂ | 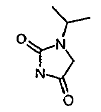 |
| A1.179 | CF₂Cl | CH₂OCH₂CH₂ | 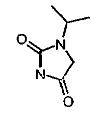 |
| A1.180 | CHF₂ | CH₂OCH₂CH₂ | 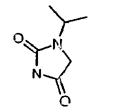 |
| A1.181 | CF₃ | CH₂ | 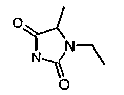 |
| A1.182 | CF₂Cl | CH₂ | 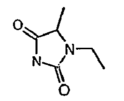 |
| A1.183 | CHF₂ | CH₂ | 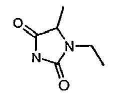 |
| A1.184 | CF₃ | CH₂OCH₂CH₂ | 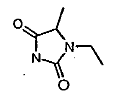 |
| A1.185 | CF₂Cl | CH₂OCH₂CH₂ | 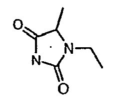 |
| A1.186 | CHF₂ | CH₂OCH₂CH₂ | 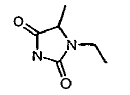 |
| A1.187 | CF₃ | CH₂ | 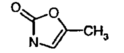 |
| A1.188 | CF₂Cl | CH₂ | 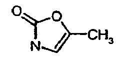 |
| A1.189 | CHF₂ | CH₂ | 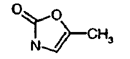 |
| A1.190 | CF₃ | CH₂OCH₂CH₂ | 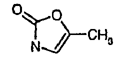 |
| A1.191 | CF₂Cl | CH₂OCH₂CH₂ | 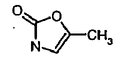 |
| A1.192 | CHF₂ | CH₂OCH₂CH₂ | 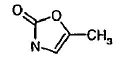 |
| A1.193 | CF₃ | CH₂ | 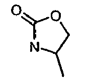 |
| A1.194 | CF₂Cl | CH₂ | 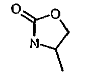 |
| A1.195 | CHF₂ | CH₂ | 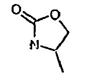 |
| A1.196 | CF₃ | CH₂OCH₂CH₂ | 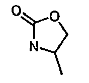 |
| A1.197 | CF₂Cl | CH₂OCH₂CH₂ | 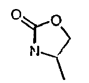 |
| A1.198 | CHF₂ | CH₂OCH₂CH₂ | 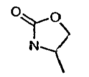 |
| A1.199 | CF₃ | CH₂ | 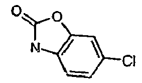 |
| A1.200 | CF₂Cl | CH₂ | 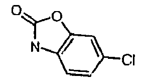 |
| A1.201 | CHF₂ | CH₂ | 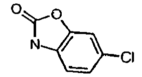 |
| A1.202 | CF₃ | CH₂ | 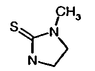 |
| A1.203 | CF₂Cl | CH₂ | 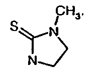 |
| A1.204 | CHF₂ | CH₂ | 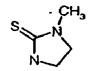 |
| A1.205 | CF₃ | CH₂OCH₂CH₂ | 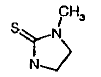 |
| A1.206 | CF₂Cl | CH₂OCH₂CH₂ | 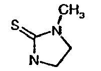 |
| A1.207 | CHF₂ | CH₂OCH₂CH₂ | 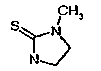 |
| A1.208 | CF₃ | CH₂ | 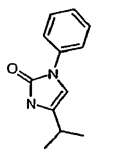 |
| A1.209 | CF₃ | CH₂ | 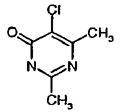 |
| A1.210 | CHF₂ | CH₂ | 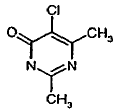 |
| A1.211 | CF₃ | CH₂ | 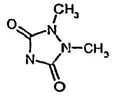 |
| A1.212 | CHF₂ | CH₂ | 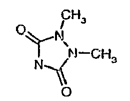 |
| A1.213 | CF₃ | CH₂ | 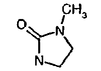 |
| A1.214 | CF₂Cl | CH₂ | 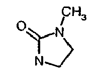 |
| A1.215 | CHF₂ | CH₂ | 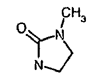 |
| A1.216 | CF₃ | CH₂OCH₂CH₂ | 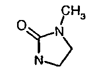 |
| A1.217 | CF₂Cl | CH₂OCH₂CH₂ | 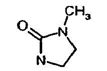 |
| A1.218 | CHF₂ | CH₂OCH₂CH₂ | 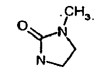 |
| A1.219 | CF₃ | CH₂ | 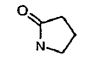 |
| A1.220 | CF₃ | CH₂OCH₂CH₂ | 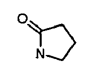 |
| A1.221 | CF₃ | CH₂ | 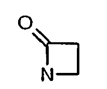 |
| A1.222 | CF₃ | CH₂ | 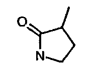 |
| A1.223 | CF₃ | CH₂ | 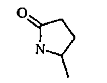 |
| A1.224 | CF₃ | CH₂ | 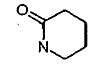 |
| A1.225 | CClF₂ | CH₂ | 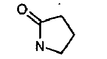 |
| A1.226 | CClF₂ | CH₂ | 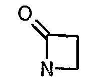 |
| A1.227 | CClF₂ | CH₂ | 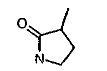 |
| A1.228 | CClF₂ | CH₂ | 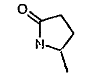 |
| A1.229 | CClF₂ | CH₂ | 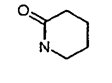 |
| A1.230 | CHF₂ | CH₂ | 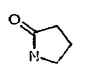 |
| A1.231 | CHF₂ | CH₂ | 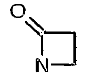 |
| A1.232 | CHF₂ | CH₂ | 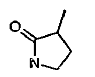 |
| A1.233 | CHF₂ | CH₂ | 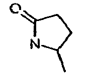 |
| A1.234 | CHF₂ | CH₂ | 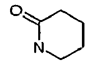 |
| A1.235 | CF₃ | CH₂ | 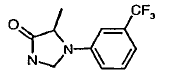 |
| A1.236 | CHF₂. | CH₂ | 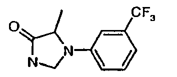 |
| A1.237 | CF₃ | CH₂ | 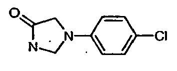 |
| A1.238 | CHF₂ | CH₂ | 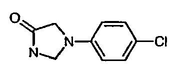 |
| A1.240 | CF₃ | CH₂ | 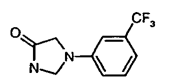 |
| A1.241 | CHF₂ | CH₂ | 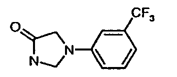 |
| A1.242 | CF₃ | CH₂ | 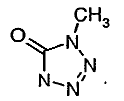 |
| A1.243 | CF₃ | CH₂ | 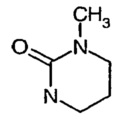 |
| A1.244 | CF₃ | CH₂ | 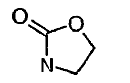 |
| A1.245 | CF₃ | CH₂ | 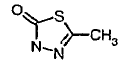 |

## Claims

1. A process for the preparation of a compound of formula I 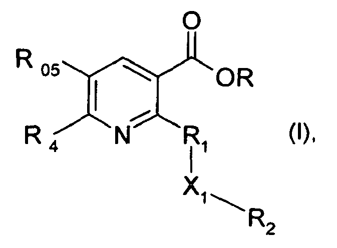 wherein
R is C₁-C₆alkyl;
R₀₅ is hydrogen;
R₁ is a C₁-C₆alkylene, C₃-C₆alkenylene or C₃-C₆alkynylene chain which may be substituted one or more times by halogen and/or by R₅, the unsaturated bonds of the chain not being attached directly to the substituent X₁;
R₄ is halomethyl or haloethyl;
X₁ is oxygen, -O(CO)-, -(CO)O-, -O(CO)O-, -N(R₆)-O-, -O-NR₁₇-, thio, sulfinyl, sulfonyl, -SO₂NR₇-, -NR₁₈SO₂-, or -NR₈-;
R₂ is hydrogen or C₁-C₈alkyl, or is a C₁-C₈alkyl, C₃-C₆alkenyl or C₃-C₆alkynyl group which may be substituted one or more times by halogen, hydroxy, amino, formyl, nitro, cyano, mercapto, carbamoyl, C₁-C₆alkoxy, C₁-C₆alkoxycarbonyl, C₂-C₆alkenyl, C₂-C₆haloalkenyl, C₂-C₆alkynyl, C₂-C₆haloalkynyl, C₃-C₆cycloalkyl, halo-substituted C₃-C₆cycloalkyl, C₃-C₆alkenyloxy, C₃-C₆alkynyloxy, C₁-C₆haloalkoxy, C₃-C₆haloalkenyloxy, cyano-C₁-C₆alkoxy, C₁-C₆alkoxy-C₁-C₆alkoxy, C₁-C₆alkoxy-C₁-C₆alkoxy-C₁-C₆alkoxy, C₁-C₆alkyl-thi0-C₁-C₆alkoxy, C₁-C₆alkylsulfinyl-C₁-C₆alkoxy, C₁-C₆alkylsulfonyl-C₁-C₆alkoxy, C₁-C₆-alkoxycarbonyl-C₁-C₆alkoxy, C₁-C₆alkylcarbonyl, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, oxiranyl (which may in turn be substituted by C₁-C₆alkyl), (3-oxetanyl)oxy (which may in turn be substituted by C₁-C₆alkyl), or by benzylthio, benzylsulfinyl, benzylsulfonyl, C₁-C₆alkylamino, di(C₁-C₆alkyl)amino, R₉S(O)₂O-, R₁₀N(R₁₁)SO₂-, rhodano, phenyl, phenoxy, phenylthio, phenylsulfinyl or by phenylsulfonyl;
it being possible for the phenyl- or benzyl-containing groups to be in turn substituted by one or more C₁-C₆alkyl, C₁-C₆haloalkyl, C₁-C₆alkoxy, C₁-C₆haloalkoxy, halogen, cyano, hydroxy or nitro groups, or
R₂ is phenyl which may be substituted one or more times by C₁-C₆alkyl, C₁-C₆haloalkyl, C₁-C₆alkoxy, C₁-C₆haloalkoxy, halogen, cyano, hydroxy or by nitro; or
R₂ is C₃-C₆cycloalkyl, C₁-C₆alkoxy- or C₁-C₆alkyl-substituted C₃-C₆cycloalkyl, 3-oxetanyl or C₁-C₆alkyl-substituted 3-oxetanyl; or
R₂ is a five- to ten-membered, monocyclic or fused bicyclic, ring system which may be aromatic, partially saturated or fully saturated and may contain from 1 to 4 hetero atoms selected from nitrogen, oxygen, sulfur, and/or may contain the group -C(=O)-, -C(=S)-, -C(=NR₁₉)-, -(N=O)-, -S(=O)- or -SO₂-, the ring system being attached to the substituent X₁ either directly or by way of a C₁-C₄alkylene, -N(R₁₂)-C₁-C₄alkylene, -SO-C₁-C₄alkylene or -SO₂-C₁-C₄alkylene group and each ring system containing no more than 2 oxygen atoms and no more than two sulfur atoms, and it being possible for each ring system itself to be substituted one or more times by C₁-C₆alkyl, C₁-C₆haloalkyl, C₂-C₆alkenyl, C₂-C₆haloalkenyl, C₂-C₆alkynyl, C₂-C₆haloalkynyl, C₁-C₆alkoxy, C₁-C₆haloalkoxy, C₃-C₆alkenyloxy, C₃-C₆alkynyloxy, mercapto, amino, hydroxy, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₁-C₄alkylcarbonyl-C₁-C₃alkylthio, C₁-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsuffonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano, nitro or by phenyl, it being possible for the phenyl group to be in turn substituted by hydroxy, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₁-C₄alkylcarbonyl-C₁-C₃alkylthio, C₁-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, N,N-di(C₁-C₂alkyl)aminosulfonyl, di(C₁-C₄alkyl)amino, halogen, cyano or by nitro, and the substituents on nitrogen in a heterocyclic ring being other than halogen;
R₅ is hydroxy, C₁-C₅alkoxy, C₃-C₆cycloalkyloxy, C₁-C₆alkoxy-C₁-C₆alkoxy, C₁-C₆alkoxy-C₁-C₆alkoxy-C₁-C₆alkoxy or C₁-C₂alkylsulfonyloxy;
R₆, R₇, R₈, R₉, R₁₀ R₁₁, R₁₂, R₁₇ and R₁₈ are each independently of the others hydrogen, C₁-C₆alkyl, C₁-C₆haloalkyl, C₁-C₆alkoxycarbonyl, C₁-C₆alkylcarbonyl, C₁-C₆alkoxy-C₁-C₆alkyl, C₁-C₆alkoxy-C₁-C₆alkyl substituted by C₁-C₆alkoxy, benzyl, or phenyl, it being possible for phenyl and benzyl to be in turn substituted one or more times by C₁-C₆alkyl, C₁-C₆haloalkyl, C₁-C₆alkoxy, C₁-C₆haloalkoxy, halogen, cyano, hydroxy or by nitro; R₆ not being hydrogen when R₉ is hydrogen, C₁-C₆alkoxycarbonyl or C₁-C₆alkylcarbonyl;
or the group -R₁-X₁-R₂ together is C₁-C₆alkyl, C₂-C₆alkenyl, C₂-C₆haloalkenyl, C₂-C₆-alkynyl, C₂-C₆haloalkynyl, C₃-C₆cycloalkyl, C₁-C₆alkoxy, C₁-C₆haloalkoxy, C₁-C₆alkylthio, C₁-C₆alkylsulfinyl, C₁-C₆alkylsulfonyl, C₁-C₆haloalkyl, C₁-C₆haloalkylthio, C₁-C₆haloalkyl-sulfinyl, C₁-C₆haloalkylsulfonyl, C₁-C₆alkoxycarbonyl, C₁-C₆alky[carbonyl, C₁-C₆alkylamino, di(C₁-C₆alkyl)amino, C₁-C₆alkylaminosulfonyl, di(C₁-C₆alkyl)aminosulfonyl, -NH-S-R₁₃, -N-(C₁-C₄alkylthio)-R₁₃, -NH-SO-R₁₄, -N-(C₁-C₄alkylsulfonyl)-R₁₄, -NH-SO₂-R₁₅, -N-(C₁-C₄alkylsulfonyl)-R₁₅, nitro, cyano, halogen, hydroxy, amino, formyl, rhodano-C₁-C₆alkyl, cyano-C₁-C₆alkyl, oxiranyl, C₃-C₆alkenyloxy, C₃-C₆alkynyloxy, C₁-C₆alkoxy-C₁-C₆alkoxy, cyano-C₁-C₆alkenyloxy, C₁-C₆alkoxycarbonyloxy-C₁-C₆alkoxy, C₃-C₆alkynyloxy, cyano-C₁-C₆alkoxy, C₁-C₆alkoxycarbonyl-C₁-C₆alkoxy, C₁-C₆alkylthio-C₁-C₆alkoxy, C₁-C₆alkoxycarbonyl-C₁-C₆alkylthio, C₁-C₆alkoxycarbonyl-C₁-C₆alkylsulfinyl, C₁-C₆alkoxycarbonyl-C₁-C₆alkylsulfonyl, C₁-C₆alkylsulfonyloxy, C₁-C₆haloalkylsulfonyloxy, phenyl, benzyl, phenoxy, phenylthio, phenylsulfinyl, phenylsulfonyl, benzylthio, benzylsulfinyl or benzylsulfonyl, it being possible for the phenyl groups to be substituted one or more times by halogen, methyl, ethyl, trifluoromethyl, methoxy or by nitro;
or the group -R₁-X₁-R₂ together is a five- to ten-membered, monocyclic or fused bicyclic, ring system, which may be aromatic or partially saturated and which may contain from 1 to 4 hetero atoms selected from nitrogen, oxygen and sulfur, the ring system either being directly attached to the pyridine ring or being attached to the pyridine ring by way of a C₁-C₄alkylene group, and it being possible for each ring system to contain no more than 2 oxygen atoms and no more than two sulfur atoms, and/or to contain the group -C(=O)-, -C(=S)-, -C(=NR₂₀)-, -(N=O)-, -S(=O)- or -SO₂-;
and the ring system itself may be substituted one, two or three times by C₁-C₆alkyl, C₁-C₆haloalkyl, C₃-C₆alkenyl, C₃-C₆haloalkenyl, C₃-C₆alkynyl, C₃-C₆haloalkynyl, C₁-C₆alkoxy, C₁-C₆haloalkoxy, C₃-C₆alkenyloxy, C₃-C₆alkynyloxy, mercapto, C₁-C₆alkylthio, C₁-C₆haloalkylthio, C₃-C₆alkenylthio, C₃-C₆haloalkenylthio, C₃-C₆alkynylthio, C₁-C₃alkoxy-C₁-C₃alkylthio, C₃-C₅acetylalkylthio, C₁-C₄alkoxycarbonyl-C₁-C₃alkylthio, cyano-C₁-C₃alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, di(C₁-C₆alkyl)aminosulfonyl, C₁-C₃alkylene-R₁₆, N(H)-C₁-C₆alkyl, N(H)-C₁-C₆alkoxy, N-(C₁-C₆alkyl)-C₁-C₆alkyl, N-(C₁-C₆alkyl)-C₁-C₆alkoxy, halogen, cyano, nitro, phenyl, and by benzylthio, it being possible for phenyl and benzylthio to be in turn substituted on the phenyl ring by C₁-C₃alkyl, C₁-C₃haloalkyl, C₁-C₃alkoxy, C₁-C₃haloalkoxy, halogen, cyano or by nitro, and substituents on a nitrogen atom in a heterocyclic ring being other than halogen;
R₁₃ is N(H)-C₁-C₆alkyl, N(H)-C₁-C₆alkoxy, N-(C₁-C₆alkyl)-C₁-C₆alkyl, N-(C₁-C₆alkyl)-C₁-C₆alkoxy, C₁-C₆alkoxy, C₁-C₆haloalkoxy, C₁-C₆alkyl, C₁-C₆haloalkyl, C₃-C₆alkenyl, C₃-C₆haloalkenyl, C₃-C₆alkynyl, C₃-C₆haloalkynyl, C₃-C₆cycloalkyl or phenyl, it being possible for phenyl to be in turn substituted by C₁-C₃alkyl, C₁-C₃haloalkyl, C₁-C₃alkoxy, C₁-C₃haloalkoxy, halogen, cyano or by nitro;
R₁₄ is N(H)-C₁-C₆alkyl, N(H)-C₁-C₆alkoxy, N-(C₁-C₆alkyl)-C₁-C₆alkyl, N-(C₁-C₆alkyl)-C₁-C₆alkoxy, C₁-C₆alkoxy, C₁-C₆haloalkoxy, C₁-C₆alkyl, C₁-C₆haloalkyl, C₃-C₆alkenyl, C₃-C₆haloalkenyl, C₃-C₆alkynyl, C₃-C₆haloalkynyl, C₃-C₆cycloalkyl or phenyl, it being possible for phenyl to be in turn substituted by C₁-C₃alkyl, C₁-C₃haloalkyl, C₁-C₃alkoxy, C₁-C₃haloalkoxy, halogen, cyano or by nitro;
R₁₅ is N(H)-C₁-C₆alkyl, N(H)-C₁-C₆alkoxy, N-(C₁-C₆alkyl)-C₁-C₆alkyl, N-(C₁-C₆alkyl)-C₁-C₆alkoxy, C₁-C₆alkoxy, C₁-C₆haloalkoxy, C₁-C₆alkyl, C₁-C₆haloalkyl, C₃-C₆alkenyl, C₃-C₆haloalkenyl, C₃-C₆alkynyl, C₃-C₆haloalkynyl, C₃-C₆cycloalkyl or phenyl, it being possible for phenyl to be in turn substituted by C₁-C₃alkyl, C₁-C₃haloalkyl, C₁-C₃alkoxy, C₁-C₃haloalkoxy, halogen, cyano or by nitro;
R₁₆ is C₁-C₃alkoxy, C₂-C₄alkoxycarbonyl, C₁-C₃alkylthio, C₁-C₃alkylsulfinyl, C₁-C₃alkylsulfonyl or phenyl, it being possible for phenyl to be in turn substituted by C₁-C₃alkyl, C₁-C₃haloalkyl, C₁-C₃alkoxy, C₁-C₃haloalkoxy, halogen, cyano or by nitro; and
R₁₉ and R₂₀ are each independently of the other hydrogen, hydroxy, C₁-C₆alkyl, C₁-C₆haloalkyl, C₁-C₆alkoxy, C₁-C₆alkylcarbonyl, C₁-C₆alkoxycarbonyl or C₁-C₆alkylsulfonyl; which process comprises reacting
a compound of formula II 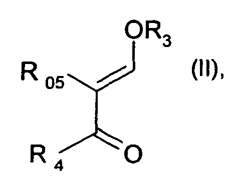
wherein R₃ is C₁-C₈alkyl or C₃-C₆cycloalkyl and R₄ and R₀₅ are as defined for formula I, with a compound of formula III 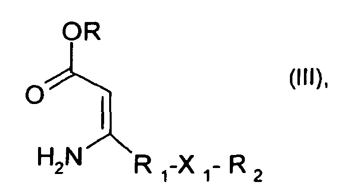 wherein R, R₁, R₂ and X₁ are as defined for formula I, in an inert solvent in the presence of a proton source.

2. A compound of formula IIIa 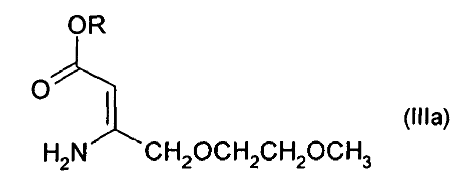 wherein R is as defined for formula I in claim 1.

## Patentansprüche

1. Verfahren zur Herstellung einer Verbindung der Formel I 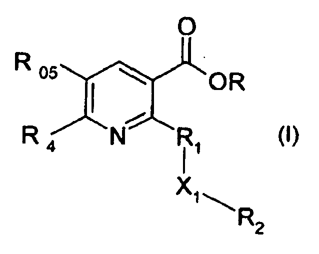 worin
R C₁-C₆-Alkyl darstellt;
R₀₅ Wasserstoff darstellt;
R₁ eine C₁-C₆-Alkylen-, C₃-C₆ Alkenylen- oder C₃-C₆-Alki-nylen-Kette darstellt, die einmal oder mehrere Male mit Halogen und/oder mit R₅ substituiert sein kann, wobei die ungesättigten Bindungen der Kette nicht direkt an den Substituenten X₁ gebunden sind;
R₄ Halogenmethyl oder Halogenethyl darstellt;
X₁ Sauerstoff, -O (CO)-, -(CO) O-, -O(CO)O-, -N (R₆)-O-, -O-NR₁₇-, Thio, Sulfinyl, Sulfonyl, -SO₂NR₇-, -NR₁₈SO₂- oder -NR₈-darstellt;
R₂ Wasserstoff oder C₁-C₈-Alkyl darstellt, oder eine C₁-C₈-Alkyl-, C₃-C₆-Alkenyl- oder C₃-C₆ Alkinyl-Gruppe, die einmal oder mehrere Male mit Halogen, Hydroxy, Amino, Formyl, Nitro, Cyano, Mercapto, Carbamoyl, C₁-C₆-Alkoxy, C₁-C₆-Alkoxycarbonyl, C₂-C₆-Alkenyl, C₂-C₆-Halogenalkenyl , C₂-C₆-Alkinyl, C₂-C₆-Halogenalkinyl, C₃-C₆-Cycloalkyl, Halogen-substituiertem C₃-C₆-Cycloalkyl, C₃-C₆-Alkenyloxy, C₃-C₆-Alkinyloxy, C₁-C₆-Halogenalkoxy, C₃-C₆-Halogenalkenyloxy, Cyano-C₁-C₆-alkoxy, C₁-C₆-Alkoxy-C₁-C₆-alkoxy, C₁-C₆-Alkoxy-C₁-C₆-alkoxy-C₁-C₆-alkoxy, C₁-C₆-Alkylthio-C₁-C₆-alkoxy, C₁-C₆-Alkylsulfinyl-C₁-C₆-alkoxy, C₁-C₆-Alkylsulfonyl-C₁-C₆-alkoxy, C₁-C₆-Alkoxycarbonyl-C₁-C₆-alkoxy, C₁-C₆-Alkylcarbonyl, C₁-C₆-Alkylthio, C₁-C₆-Alkylsulfinyl, C₁-C₆-Alkylsulfonyl, C₁-C₆-Halogen-alkylthio, C₁-C₆-Halogenalkylsulfinyl, C₁-C₆-Halogenalkylsulfonyl, Oxiranyl (das wiederum mit C₁-C₆-Alkyl substituiert sein kann), (3-Oxetanyl)-oxy (das wiederum mit C₁-C₆-Alkyl substituiert sein kann), oder mit Benzylthio, Benzylsulfinyl, Benzylsulfonyl, C₁-C₆-Alkylamino, Di-(C₁-C₆-alkyl)-amino, R₉S(O)₂O-, R₁₀N(R₁₁)SO₂-, Rhodano, Phenyl, Phenoxy, Phenylthio, Phenylsulfinyl oder mit Phenylsulfonyl substituiert sein kann, darstellt;
wobei es möglich ist, dass die Phenyl- oder Benzylenthaltenden Gruppen wiederum mit einer oder mehreren C₁-C₆-Alkyl-, C₁-C₆-Halogenalkyl-, C₁-C₆-Alkoxy-, C₁-C₆-Halogenalkoxy-, Halogen-, Cyano-, Hydroxy- oder Nitro-Gruppen substituiert sind, oder
R₂ Phenyl, das einmal oder mehrere Male mit C₁-C₆-Alkyl, C₁-C₆-Halogenalkyl, C₁-C₆-Alkoxy, C₁-C₆-Halogenalkoxy, Halogen, Cyano, Hydroxy oder mit Nitro substituiert sein kann, darstellt; oder
R₂ C₃-C₆-Cycloalkyl, C₁-C₆-Alkoxy- oder C₁-C₆-Alkyl-substituiertes C₃-C₆-Cycloalkyl, 3-Oxetanyl oder C₁-C₆-Alkyl-substituiertes 3-Oxetanyl darstellt; oder
R₂ ein fünf- bis zehn-gliedriges, monocyclisches oder kondensiertes bicyclisches Ringsystem darstellt, das aromatisch, teilweise gesättigt oder vollständig gesättigt sein kann, und 1 bis 4 Hetero-Atome, ausgewählt aus Stickstoff, Sauerstoff, Schwefel, enthalten kann, und/oder die Gruppe - C (=O)-, -C(=S)-, -C(=NR₁₉)-, -(N=O)-, -S (=O)- oder -SO₂- enthalten kann, wobei das Ringsystem an den Substituenten X₁ entweder direkt oder über eine C₁-C₄-Alkylen-, -N(R₁₂)-C₁-C₄-Alkylen-, -SO-C₁-C₄-Alkylen- oder -SO₂-C₁-C₄-Alkylen-Gruppe gebunden ist und jedes Ringsystem nicht mehr als 2 Sauerstoff-Atome und nicht mehr als zwei Schwefel-Atome enthält, und es möglich ist, dass jedes Ringsystem selbst einmal oder mehrere Male mit C₁-C₆-Alkyl, C₁-C₆-Halogenalkyl, C₂-C₆-Alkenyl, C₂-C₆-Halogenalkenyl, C₂-C₆-Alki-nyl, C₂-C₆-Halogenalkinyl, C₁-C₆-Alkoxy, C₁-C₆-Halogenalkoxy, C₃-C₆-Alkenyloxy, C₃-C₆-Alkinyloxy, Mercapto, Amino, Hydroxy, C₁-C₆-Al-kylthio, C₁-C₆-Halogenalkylthio, C₃-C₆-Alkenylthio, C₃-C₆-Halogen-alkenylthio, C₃-C₆-Alkinylthio, C₁-C₃-Alkoxy-C₁-C₃-alkylthio, C₁-C₄-Alkylcarbonyl-C₁-C₃-alkylthio, C₁-C₄-Alkoxycarbonyl-C₁-C₃-alkyl-thio, Cyano-C₁-C₃-alkylthio, C₁-C₆-Alkylsulfinyl, C₁-C₆-Halogenal-kylsulfinyl, C₁-C₆-Alkylsulfonyl, C₁-C₆-Halogenalkylsulfonyl, Aminosulfonyl, C₁-C₂-Alkylaminosulfonyl, N,N-Di-(C₁-C₂-alkyl)-aminosulfonyl, Di-(C₁-C₄-alkyl)-amino, Halogen, Cyano, Nitro oder mit Phenyl substituiert sind, wobei es möglich ist, dass die Phenyl-Gruppe wiederum mit Hydroxy, C₁-C₆-Alkylthio, C₁-C₆-Halogenalkylthio, C₃-C₆-Alkenylthio, C₃-C₆-Halogenalkenylthio, C₃-C₆-Alkinylthio, C₁-C₃-Alkoxy-C₁-C₃-alkylthio, C₁-C₄-Alkylcarbonyl-C₁-C₃-alkylthio, C₁-C₄-Alkoxycarbonyl-C₁-C₃-alkylthio, Cyano-C₁-C₃-alkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, C₁-C₂-Alkylaminosulfonyl, N,N-Di-(C₁-C₂-alkyl)-aminosulfonyl, Di-(C₁-C₄-alkyl)-amino, Halogen, Cyano oder mit Nitro substituiert sind, und wobei die Substituenten am Stickstoff in einem heterocyclischen Ring von Halogen verschieden sind;
R₅ Hydroxy, C₁-C₆-Alkoxy, C₃-C₆-Cycloalkyloxy, C₁-C₆-Alkoxy-C₁-C₆-alkoxy, C₁-C₆-Alkoxy-C₁-C₆-alkoxy-C₁-C₆-alkoxy oder C₁-C₂-Alkylsulfonyloxy darstellt;
R₆, R₇, R₈, R₉, R₁₀ R₁₁, R₁₂, R₁₇ und R₁₈, jeweils unabhängig von den anderen, Wasserstoff, C₁-C₆-Alkyl, C₁-C₆-Halogen-alkyl, C₁-C₆-Alkoxycarbonyl, C₁-C₆-Alkylcarbonyl, C₁-C₆-Alkoxy-C₁-C₆-alkyl, C₁-C₆-Alkoxy-C₁-C₆-alkyl, substituiert mit C₁-C₆-Alkoxy, Benzyl oder Phenyl, darstellen, wobei es möglich ist, dass Phenyl und Benzyl wiederum einmal oder mehrere Male mit C₁-C₆-Alkyl, C₁-C₆-Halogenalkyl, C₁-C₆-Alkoxy, C₁-C₆-Halogenalkoxy, Halogen, Cyano, Hydroxy oder mit Nitro substituiert sind; wobei R₆ nicht Wasserstoff darstellt, wenn R₉ Wasserstoff, C₁-C₆-Alk-oxycarbonyl oder C₁-C₆-Alkylcarbonyl darstellt; oder die Gruppe -R₁-X₁-R₂ zusammen C₁-C₆-Alkyl, C₂-C₆-Alkenyl, C₂-C₆-Halogenalke-nyl, C₂-C₆-Alkinyl, C₂-C₆-Halogenalkinyl, C₃-C₆-Cycloalkyl, C₁-C₆-Alkoxy, C₁-C₆-Halogenalkoxy, C₁-C₆-Alkylthio, C₁-C₆-Alkylsulfi-nyl, C₁-C₆-Alkylsulfonyl, C₁-C₆-Halogenalkyl, C₁-C₆-Halogenalkyl-thio, C₁-C₆-Halogenalkylsulfinyl, C₁-C₆-Halogenalkylsulfonyl, C₁-C₆-Alkoxycarbonyl, C₁-C₆-Alkylcarbonyl, C₁-C₆-Alkylamino, Di-(C₁-C₆-alkyl)-amino, C₁-C₆-Alkylaminosulfonyl, Di-(C₁-C₆-alkyl)-aminosulfonyl, -NH-S-R₁₃, -N-(C₁-C₄-Alkylthio)-R₁₃, -NH-SO-R₁₄ , -N-(C₁-C₄-Alkylsulfonyl)-R₁₄, -NH-SO₂-R₁₅, -N-(C₁-C₄-Alkylsulfonyl)-R₁₅, Nitro, Cyano, Halogen, Hydroxy, Amino, Formyl, Rhodano-C₁-C₆-alkyl, Cyano-C₁-C₆-alkyl, Oxiranyl, C₃-C₆-Alkenyloxy, C₃-C₆-Alkinyloxy, C₁-C₆-Alkoxy-C₁-C₆-alkoxy, Cyano-C₁-C₆-alkenyloxy, C₁-C₆-Alkoxycarbonyloxy-C₁-C₆-alkoxy, C₃-C₆-Alkinyloxy, Cyano-C₁-C₆-alkoxy, C₁-C₆-Alkoxycarbonyl-C₁-C₆-alkoxy, C₁-C₆-Alkylthio-C₁-C₆-alkoxy, C₁-C₆-Alkoxycarbonyl-C₁-C₆-alkylthio, C₁-C₆-Alkoxycarbonyl-C₁-C₆-alkylsulfinyl, C₁-C₆-Alkoxycarbonyl-C₁-C₆-alkylsulfonyl, C₁-C₆-Alkylsulfonyloxy, C₁-C₆-Halogenalkylsulfonyloxy, Phenyl, Benzyl, Phenoxy, Phenylthio, Phenylsulfinyl, Phenylsulfonyl, Benzylthio, Benzylsulfinyl oder Benzylsulfonyl darstellt, wobei es möglich ist, dass die Phenyl-Gruppen einmal oder mehrere Male mit Halogen, Methyl, Ethyl, Trifluormethyl, Methoxy oder mit Nitro substituiert sind;
oder die Gruppe -R₁-X₁-R₂ zusammen ein fünf- bis zehngliedriges, monocyclisches oder kondensiertes bicyclisches Ringsystem darstellt, das aromatisch oder teilweise gesättigt sein kann, und das 1 bis 4 Hetero-Atome, ausgewählt aus Stickstoff, Sauerstoff und Schwefel, enthalten kann, wobei das Ringsystem entweder direkt an den Pyridin-Ring gebunden ist, oder an den Pyridin-Ring mit Hilfe einer C₁-C₄-Alkylen-Gruppe gebunden ist, und es möglich ist, dass jedes Ringsystem nicht mehr als 2 Sauerstoff-Atome und nicht mehr als zwei Schwefel-Atome enthält, und/oder die Gruppe -C (=O)-, -C(=S)-, -C(=NR₂₀)-, -(N=O)-, -S (=O)- oder -SO₂- enthält,
und das Ringsystem selbst ein-, zwei- oder dreimal mit C₁-C₆-Alkyl, C₁-C₆-Halogenalkyl, C₃-C₆-Alkenyl, C₃-C₆-Halogenalkenyl, C₃-C₆-Alkinyl, C₃-C₆-Halogenalkinyl, C₁-C₆-Alkoxy, C₁-C₆-Halogenalkoxy, C₃-C₆-Alkenyloxy, C₃-C₆-Alkinyloxy, Mercapto, C₁-C₆-Alkylthio, C₁-C₆-Halogenalkylthio, C₃-C₆-Alkenylthio, C₃-C₆-Halogenalkenylthio, C₃-C₆-Alkinylthio, C₁-C₃-Alkoxy-C₁-C₃-alkylthio, C₃-C₅-Acetylalkylthio, C₁-C₄-Alkoxycarbonyl-C₁-C₃-alkylthio, Cyano-C₁-C₃-alkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, C₁-C₂-Alkylaminosulfonyl, Di-(C₁-C₆-alkyl)-aminosulfonyl, C₁-C₃-Alkylen-R₁₆, N(H)-C₁-C₆-Alkyl, N(H)-C₁-C₆-Alkoxy, N-(C₁-C₆-Alkyl)-C₁-C₆-alkyl, N-(C₁-C₆-Alkyl)-C₁-C₆-alkoxy, Halogen, Cyano, Nitro, Phenyl, und mit Benzylthio substituiert sein kann, wobei es möglich ist, dass Phenyl und Benzylthio wiederum an dem PhenylRing mit C₁-C₃-Alkyl, C₁-C₃-Halogenalkyl, C₁-C₃-Alkoxy, C₁-C₃-Halogenalkoxy, Halogen, Cyano oder mit Nitro substituiert sind, und wobei die Substituenten an einem Stickstoff-Atom in einem heterocyclischen Ring von Halogen verschieden sind;
R₁₃ N(H)-C₁-C₆-Alkyl, N(H)-C₁-C₆-Alkoxy, N-(C₁-C₆-Alkyl)-C₁-C₆-alkyl, N-(C₁-C₆-Alkyl)-C₁-C₆-alkoxy, C₁-C₆-Alkoxy, C₁-C₆-Halogenalkoxy, C₁-C₆-Alkyl, C₁-C₆-Halogenalkyl, C₃-C₆-Alkenyl, C₃-C₆-Halogenalkenyl, C₃-C₆-Alkinyl, C₃-C₆-Halogenalkinyl, C₃-C₆-Cycloalkyl oder Phenyl darstellt, wobei es möglich ist, dass Phenyl wiederum mit C₁-C₃-Alkyl, C₁-C₃-Halogenalkyl, C₁-C₃-Alkoxy, C₁-C₃-Ha-logenalkoxy, Halogen, Cyano oder mit Nitro substituiert ist;
R₁₄ N(H)-C₁-C₆-Alkyl, N(H)-C₁-C₆-Alkoxy, N-(C₁-C₆-Alkyl)-C₁-C₆-alkyl, N-(C₁-C₆-Alkyl)-C₁-C₆-alkoxy, C₁-C₆-Alkoxy, C₁-C₆-Halogenalkoxy, C₁-C₆-Alkyl, C₁-C₆-Halogenalkyl, C₃-C₆-Alkenyl, C₃-C₆-Halogenalkenyl, C₃-C₆-Alkinyl, C₃-C₆-Halogenalkinyl, C₃-C₆-Cycloalkyl oder Phenyl darstellt, wobei es möglich ist, dass Phenyl wiederum mit C₁-C₃-Alkyl, C₁-C₃-Halogenalkyl, C₁-C₃-Alkoxy, C₁-C₃-Halogenalkoxy, Halogen, Cyano oder mit Nitro substituiert ist;
R₁₅ N(H)-C₁-C₆-Alkyl, N(H)-C₁-C₆-Alkoxy, N-(C₁-C₆-Alkyl)-C₁-C₆-alkyl, N-(C₁-C₆-Alkyl)-C₁-C₆-alkoxy, C₁-C₆-Alkoxy, C₁-C₆-Halogenalkoxy, C₁-C₆-Alkyl, C₁-C₆-Halogenalkyl, C₃-C₆-Alkenyl, C₃-C₆-Halogenalkenyl, C₃-C₆-Alkinyl, C₃-C₆-Halogenalkinyl, C₃-C₆-Cycloalkyl oder Phenyl darstellt, wobei es möglich ist, dass Phenyl wiederum mit C₁-C₃-Alkyl, C₁-C₃-Halogenalkyl, C₁-C₃-Alkoxy, C₁-C₃-Halo-genalkoxy, Halogen, Cyano oder mit Nitro substituiert ist;
R₁₆ C₁-C₃-Alkoxy, C₂-C₄-Alkoxycarbonyl, C₁-C₃-Alkylthio, C₁-C₃-Alkylsulfinyl, C₁-C₃-Alkylsulfonyl oder Phenyl darstellt, wobei es möglich ist, dass Phenyl wiederum mit C₁-C₃-Alkyl, C₁-C₃-Halogenalkyl, C₁-C₃-Alkoxy, C₁-C₃-Halogenalkoxy, Halogen, Cyano oder mit Nitro substituiert ist; und
R₁₉ und R₂₀, jeweils unabhängig von dem anderen, Wasserstoff, Hydroxy, C₁-C₆-Alkyl, C₁-C₆-Halogenalkyl , C₁-C₆-Alkoxy, C₁-C₆-Alkylcarbonyl, C₁-C₆-Alkoxycarbonyl oder C₁-C₆-Alkylsulfonyl darstellen; wobei das Verfahren umfasst: Umsetzen einer Verbindung der Formel II 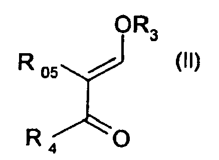
worin R₃ C₁-C₈-Alkyl oder C₃-C₆-Cycloalkyl darstellt, und R₄ und R₀₅ wie für Formel I definiert sind, mit einer Verbindung der Formel III 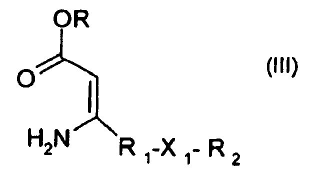 worin R, R₁, R₂ und X₁ wie für Formel I definiert sind, in einem inerten Lösungsmittel, in Gegenwart einer Protonenquelle.

2. Verbindung der Formel IIIa 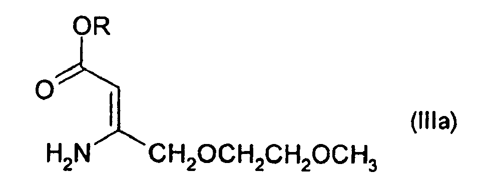 worin R wie für Formel I in Anspruch 1 definiert ist.

## Revendications

1. Procédé pour la préparation d'un composé de la formule (I) 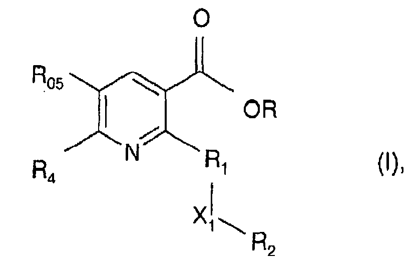 dans laquelle
R est un groupe alkyle en C₁-C₆ ;
R₀₅ est un atome d'hydrogène ;
R₁ est une chaine alkylène en C₁-C₆, alcénylène en C₃-C₆ ou alcynylène en C₃-C₆ qui peut être substituée une ou plusieurs fois par un atome d'halogène et/ou par R₅, les liaisons insaturées de la chaîne n'étant pas directement fixées au substituant X₁ ;
R₄ est un groupe halométhyle ou haloéthyle ;
X₁ est un atome d'oxygène, -O(CO)-, -(CO)O-, -O(CO)O-, -N(R₆)-O-, -O-NR₁₇-, thio, sulfinyle, sulfonyle, -SO₂NR₇-, -NR₁₈SO₂-, ou -NR₈- ;
R₂ est un atome d'hydrogène ou un groupe alkyle en C₁-C₈ ou est un groupe alkyle en C₁-C₈, alcényle en C₃-C₆ ou alcynyle en C₃-C₆ qui peut être substitué une ou plusieurs fois par un atome d'halogène, un groupe hydroxy, amino, formyle, nitro, cyano, mercapto, carbamoyle, alcoxy en C₁-C₆, alcoxycarbonyle en C₁-C₆, alcényle en C₂-C₆, haloalcényle en C₂-C₆, alcynyle en C₂-C₆, haloalcynyle en C₂-C₆, cycloalkyle en C₃-C₆, cycloalkyle en C₃-C₆ halo-substitué, alcényloxy en C₃-C₆, alcynyloxy en C₃-C₆, haloalcoxy en C₁-C₆, haloalcényloxy en C₃-C₆, cyano-(alcoxy en C₁-C₆), (alcoxy en C₁-C₆)-(alcoxy en C₁-C₆), (alcoxy en C₁-C₆)-(alcoxy en C₁-C₆)-(alcoxy en C₁-C₆), (alkyle en C₁-C₆)-thio-(alcoxy en C₁-C₆), (alkyle en C₁-C₆)-sulfinyl-(alcoxy en C₁-C₆), (alkyle en C₁-C₅)-sulfonyl-(alcoxy en C₁-C₆), (alcoxy en C₁-C₆)-carbonyl-(alcoxy en C₁-C₆), (alkyle en C₁-C₆)carbonyle, (alkyle en C₁-C₆)thio, (alkyle en C₁-C₆)sulfinyle, (alkyle en C₁-C₆)sulfonyle, (haloalkyle en C₁-C₆)thio, (haloalkyle en C₁-C₆)sulfinyle, (haloalkyle en C₁-C₆)sulfonyle, oxyranyle (qui peut à son tour être substitué par un groupe alkyle en C₁-C₆), (3-oxétanyl)oxy (qui peut à son tour être substitué par un groupe alkyle en C₁-C₆) ou par un groupe benzylthio, benzylsulfinyle, benzylsulfonyle, (alkyle en C₁-C₆)amino, di(alkyle en C₁-C₆)amino, R₉S(O)₂O-, R₁₀N(R₁₁)SO₂-, rhodano, phényle, phénoxy, phénylthio, phénylsulfinyle ou par un groupe phénylsulfonyle ;
il est possible pour les groupes contenant un groupe phényle ou benzyle d'être à leur tour substitué par un ou plusieurs groupes parmi un groupe alkyle en C₁-C₆, haloalkyle enC₁-C₆, alcoxy en C₁-C₆, haloalcoxy en C₁-C₆, un atome d'halogène, un groupe cyano, hydroxy ou nitro, ou
R₂ est un groupe phényle qui peut être substitué une ou plusieurs fois par un groupe alkyle en C₁-C₆, haloalkyle en C₁-C₆, alcoxy en C₁-C₆, haloalcoxy en C₁-C₆, un atome d'halogène, un groupe cyano, hydroxy ou par un groupe nitro ; ou
R₂ est un groupe cycloalkyle en C₃-C₆, alcoxy en C₁-C₆ ou cycloalkyle en C₃-C₆ (alkyle en C₁-C₆)-substitué, 3-oxétanyle ou 3-oxétanyle (alkyle en C₁-C₆)-substitué ; ou
R₂ est un système de noyau monocyclique ou bicyclique condensé à de 5 à 10 éléments qui peut être aromatique, partiellement saturé ou totalement saturé et qui peut contenir de 1 à 4 hétéroatomes choisis parmi les atomes d'azote, d'oxygène, de soufre, et/ou peut contenir le groupe -C(=O)-, -C(=S)-, -C(=NR₁₉)-, -(N=O)-, -S(=O)- ou -SO₂-, le système de noyau étant fixé au substituant X₁ soit directement, soit au moyen d'un groupe alkylène en C₁-C₄, -N(R₁₂)-(alkylène en C₁-C₄), -SO-(alkylène en C₁-C₄) ou -SO₂-(alkylène en C₁-C₄) et chaque système de noyau contenant au plus deux atomes d'oxygène et au plus deux atomes de soufre, et il est possible pour chaque système de noyau lui-même d'être substitué une ou plusieurs fois par un groupe alkyle en C₁-C₄, haloalkyle en C₁-C₆, alcényle en C₁-C₆, haloalcényle en C₂-C₆, alcynyle en C₂-C₆, haloalcynyle en C₂-C₆, alcoxy en C₁-C₆, haloalcoxy en C₁-C₆, alcényloxy en C₃-C₆, alcynyloxy en C₃-C₆, mercapto, amino, hydroxy, alkylthio en C₁-C₆, haloalkylthio en C₁-C₆, alcénylthio en C₃-C₆, haloalcénylthio en C₃-C₆, alcynylthio en C₃-C₆, (alcoxy en C₁-C₃)-(alkylthio en C₁-C₃), (alkyle en C₁-C₄)-carbonyl-(alkylthio en C₁-C₃), (alcoxy en C₁-C₄)-carbonyl-(alkylthio en C₁-C₃), cyano-(alkylthio en C₁-C₃), alkylsulfinyle en C₁-C₆, haloalkylsulfinyle en C₁-C₆, alkylsulfonyle en C₁-C₆, haloalkylsulfonyle en C₁-C₆, aminosulfonyle, alkylaminosulfonyle en C₁-C₂, N,N-di(alkyle en C₁-C₂)-aminosulfonyle, di(alkyle en C₁-C₄)amino, un atome d'halogène, un groupe cyano, nitro ou par un groupe phényle, il est possible que le groupe phényle soit à son tour substitué par un groupe hydroxy, alkylthio en C₁-C₆, haloalkylthio en C₁-C₆, alcénylthio en C₃-C₆, haloalcénylthio en C₃-C₆, alcylnyithio en C₃-C₆, (alcoxy en C₁-C₃)-(alkylthio en C₁-C₃), (alkylcarbonyle en C₁-C₄)-(alkylthio en C₁-C₃), (alcoxycarbonyle en C₁-C₄)-(alkylthio en C₁-C₃), cyano-(alkylthio en C₁-C₃), (alkyle en C₁-C₆)sulfinyle, haloalkylsulfinyle en C₁-C₆, alkylsulfonyle en C₁-C₆, haloalkylsulfonyle en C₁-C₆, aminosulfonyle, alkylaminosulfonyle en C₁-C₂, N,N-di(alkyle en C₁-C₂)-aminosulfonyle, di(alkyle en C₁-C₄)amino, un atome d'halogène, un groupe cyano ou par un groupe nitro, et les substituants sur l'atome d'azote dans un noyau hétérocyclique étant différents d'un atome d'halogène ;
R₅ est un groupe hydroxy, alcoxy en C₁-C₆, cycloalkyloxy en C₃-C₆, (alcoxy en C₁-C₆)-(alcoxy en C₁-C₆), (alcoxy en C₁-C₆)-(alcoxy en C₁-C₆)-(alcoxy en C₁-C₆) ou alkylsulfonyloxy en C₁-C₂ ;
R₆, R₇, R₈, R₉, R₁₀, R₁₁, R₁₂, R₁₇ et R₁₈ sont chacun indépendamment des autres un atome d'hydrogène, un groupe alkyle en C₁-C₆, haloalkyle en C₁-C₆, alcoxycarbonyle en C₁-C₆, alkylcarbonyle en C₁-C₆, (alcoxy en C₁-C₆)-(alkyle en C₁-C₆), (alcoxy en C₁-C₆)-(alkyle en C₁-C₆) substitué par un groupe alcoxy en C₁-C₆, benzyle ou phényle, il est possible pour le groupe phényle et benzyle qu'il soit à son tour substitué une ou plusieurs fois par un groupe alkyle en C₁-C₆, haloalkyle en C₁-C₆, alcoxy en C₁-C₆, haloalcoxy en C₁-C₆, un atome d'halogène, un groupe cyano, hydroxy ou par un groupe nitro ; R₆ n'étant pas un atome d'hydrogène lorsque R₉ est un atome d'hydrogène, un groupe alcoxycarbonyle en C₁-C₆ ou alkylcarbonyle en C₁-C₆ ; ou le groupe -R₁-X₁-R₂ est dans son ensemble un groupe alkyle en C₁-C₆, alcényle en C₂-C₆, haloalcényle en C₂-C₆, alcynyle en C₂-C₆, haloalcynyle en C₂-C₆, cycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkyle en C₁-C₆, haloalkylthio en C₁-C₆, haloalkylsulfinyle en C₁-C₆, haloalkylsulfonyle en C₁-C₆, alcoxycarbonyle en C₁-C₆, alkylcarbonyle en C₁-C₆, alkylamino en C₁-C₆, di(alkyle en C₁-C₆)amino, alkylaminosulfonyle en C₁-C₆, di(alkyle en C₁-C₆)aminosulfonyle, -NH-S-R₁₃, -N-(alkylthio en C₁-C₄)-R₁₃, -NH-SO-R₁₄, -N-(alkylsulfonyle en C₁-C₄)-R₁₄, -NH-SO₂-R₁₅, -N-(alkylsulfonyle en C₁-C₁₄)-R₁₅, nitro, cyano, un atome d'halogène, un groupe hydroxy, amino, formyle, rhodano-(alkyle en C₁-C₆), cyano-(alkyle en C₁-C₆), oxyranyle, alcényloxy en C₃-C₆, alcynyloxy en C₃-C₆, (alcoxy en C₁-C₆)-(alcoxy en C₁-C₆), cyano-(alcényloxy en C₁-C₆), (alcoxy en C₁-C₆)-carbonyloxy-(alcoxy en C₁-C₆), alcynyloxy en C₃-C₆, cyano-(alcoxy en C₁-C₆), (alcoxycarbonyle en C₁-C₆)-(alcoxy en C₁-C₆), (alkyle en C₁-C₆)-thio-(alcoxy en C₁-C₆), (alcoxy en C₁-C₆)-carbony-(alkylthio en C₁-C₆), (alcoxycarbonyle en C₁-C₆,)-(alkylsulfinyle en C₁-C₆), (alcoxy en C₁-C₆)-carbonyl-(alkylsulfonyle en C₁-C₆), alkylsulfonyloxy en C₁-C₆, haloalkylsulfonyloxy en C₁-C₆, phényle, benzyle, phénoxy, phénylthio, phénylsulfinyle, phénylsulfonyle, benzylthio, benzylsulfinyle ou benzylsulfonyle, il est possible pour les groupes phényles qu'ils soient substitués une ou plusieurs fois par un atome d'halogène, un groupe méthyle, éthyle, trifluorométhyle, méthoxy ou par un groupe nitro ; ou le groupe -R₁-X₁-R₂ est dans son ensemble un système de noyau monocyclique ou bicyclique condensé à de cinq à dix éléments qui peut être aromatique ou partiellement, saturé et qui peut contenir de 1 à 4 hétéroatomes choisis parmi un atome d'azote, d'oxygène et de soufre, le système de noyau étant soit directement fixé au noyau pyridine, soit étant fixé au noyau pyridine au moyen d'un groupe alkylène en C₁-C₄, et il est possible pour chaque système de noyau qu'il contienne au plus deux atomes d'oxygène et au plus deux atomes de soufre et/ou qu'il contienne le groupe -C(=O)-, -C(=S)-, -C(=NR₂₀)-, -(N=O)-, -S(-O)- ou -SO₂- ;
et le système de noyau lui-même peut être substitué une, deux ou trois fois par un groupe alkyle en C₁-C₆, haloalkyle en C₁-C₆, alcényle en C₃-C₆, haloalcényle en C₃-C₆, alcynyle en C₃-C₆, haloalcynyle en C₃-C₆, alcoxy en C₁-C₆, haloalcoxy en C₁-C₆, alcényloxy en C₃-C₆, alcynyloxy en C₃-C₆, mercapto, alkylthio en C₁-C₆, haloalkylthio en C₁-C₆" alcénylthio en C₃-C₆, haloalcénylthio en C₃-C₆, alcynylthio en C₃-C₆, (alcoxy en C₁-C₃)-(alkylthio en C₁-C₃), acétylalkylthio en C₃-C₅, (alcoxycarbonyle en C₁-C₄)-(alkylthio en C₁-C₃), cyano-(alkylthio en C₁-C₃), alkylsulfinyle en C₁-C₆, haloalkylsulfinyle en C₁-C₆, alkylsulfinyle en C₁-C₆, haloalkylsulfonyle en C₁-C₆, aminosulfonyle, alkylaminosulfonyle en C₁-C₂, di(alkyle en C₁-C₆)-aminosulfonyle, (alkylène en C₁-C₃)-R₁₆, N(H)-(alkyle en C₁-C₆), N(H)-(alcoxy en C₁-C₆), N-(alkyle en C₁-C₆)-(alkyle en C₁-C₆), N-(alkyle en C₁-C₆)-(alcoxy en C₁-C₆), un atome d'halogène, un groupe cyano, nitro, phényle et par un groupe benzylthio, il est possible que le groupe phényle et benzylthio soient à son tour substitués sur le noyau phényle par un groupe alkyle en C₁-C₃, haloalkyle en C₁-C₃, alcoxy en C₁-C₃, haloalcoxy en C₁-C₃, un atome d'halogène, un groupe cyano ou par un groupe nitro et les substituants sur un atome d'azote dans un noyau hétérocyclique étant différents d'un atome d'halogène ;
R₁₃ est un groupe N(H)-(alkyle en C₁-C₆), N(H)-(alcoxy en C₁-C₆), N-(alkyle en C₁-C₆)-(alkyle en C₁-C₆), N-(alkyle en C₁-C₆)-(alcoxy en C₁-C₆), alcoxy en C₁-C₆, haloalcoxy en C₁-C₆, alkyle en C₁-C₆, haloalkyle en C₁-C₆, alcényle en C₃-C₆, haloalcényle en C₃-C₆, alcynyle en C₃-C₆, haloalcynyle en C₃-C₆, cycloalkyle en C₃-C₆ ou phényle, il est possible que le groupe phényle soit à son tour substitué par un groupe alkyle en C₁-C₃, haloalkyle en C₁-C₃, alcoxy en C₁-C₃, haloalcoxy en C₁-C₃, un atome d'halogène, un groupe cyano ou par un groupe nitro ;
R₁₄est N(H)-(alkyle en C₁-C₆), N(H)-(alcoxy en C₁-C₆), N-(alkyle en C₁-C₆)-(alkyle en C₁-C₆), N(alkyle en C₁-C₆)-(alcoxy en C₁-C₆), alcoxy en C₁-C₆, haloalcoxy en C₁-C₆, alkyle en C₁-C₆, haloalkyle en C₁-C₆, alcényle en C₃-C₆, haloalcényle en C₃-C₆, alcynyle en C₃-C₆, haloalcynyle en C₃-C₆, cycloalkyle en C₃-C₆ ou phényle, il est possible que le groupe phényle soit à son tour substitué par un groupe alkyle en C₁-C₃, haloalkyle en C₁-C₃, alcoxy en C₁-C₃, haloalcoxy en C₁-C₃, un atome d'halogène, un groupe cyano ou un groupe nitro ;
R₁₅ est un groupe N(H)-alkyle en C₁-C₆, N(H)-(alcoxy en C₁-C₆), N-(alkyle en C₁-C₆)-(alkyle en C₁-C₆), N-(alkyle en C₁-C₆)-(alcoxy en C₁-C₆), alcoxy en C₁-C₆, haloalcoxy en C₁-C₆, alkyle en C₁-C₆, haloalkyle en C₁-C₆, alcényle en C₃-C₆, haloalcényle en C₃-C₆, alcynyle en C₃-C₆, haloalcynyle en C₃-C₆, cycloalkyle en C₃-C₆ ou phényle, il est possible que le groupe phényle soit à son tour substitué par un groupe alkyle en C₁-C₃, haloalkyle en C₁-C₃, alcoxy en C₁-C₃, haloalcoxy en C₁-C₃, un atome d'halogène, un groupe cyano ou par un groupe nitro ;
R₁₆ est un groupe alcoxy en C₁-C₃, alcoxycarbonyle en C₂-C₄, alkylthio en C₁-C₃, alkylsulfinyle en C₁-C₃, alkylsulfonyle en C₁-C₃ ou phényle, il est possible que le groupe phényle soit à son tour substitué par un groupe alkyle en C₁-C₃, haloalkyle en C₁-C₃, alcoxy en C₁-C₃, haloalcoxy en C₁-C₃, un atome d'halogène, un groupe cyano ou par un groupe nitro ; et
R₁₉ et R₂₀ sont chacun indépendamment l'un de l'autre un atome d'hydrogène, un groupe hydroxy, un groupe alkyle en C₁-C₆, haloalkyle en C₁-C₆, alcoxy en C₁-C₆, alkylcarbonyle en C₁-C₆, alcoxycarbonyle en C₁-C₆ ou alkylsulfonyle en C₁-C₆ ; lequel procédé comprend la réaction d'un composé de la formule II 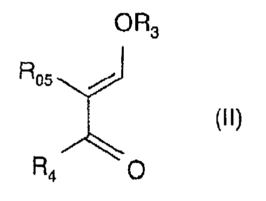
dans laquelle R₃ est un groupe alkyle en C₁-C₈ ou cycloalkyle en C₃-C₆ et R₄ et R₀₅ sont comme définis pour la formule I
avec un composé de la formule III 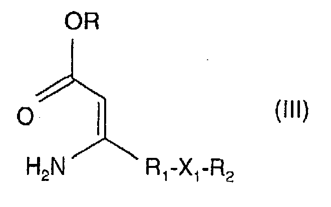 dans laquelle R, R₁, R₂ et X₁ sont comme définis pour la formule I, dans un solvant inerte en présence d'une source de protons.

2. Composé de la formule IIIa 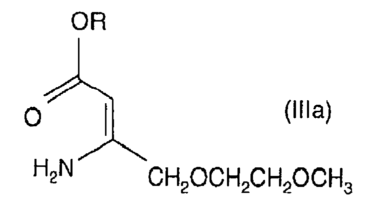 dans laquelle R est comme défini pour la formule I dans la revendication 1.
